# EUROPEAN PATENT SPECIFICATION

(11) **EP 2 490 688 B1**
(45) Date of publication and mention of the grant of the patent: **08.10.2014**
(21) Application number: 10768357.5
(22) Date of filing: 19.10.2010
(51) Int. Cl.: A61K 31/4196, A61K 31/337, A61K 45/06, A61P 35/00

(54) **COMBINATION CANCER THERAPY WITH HSP90 INHIBITORY COMPOUNDS**
KOMBINATIONSKREBSTHERAPIE MIT HSP90-HEMMERVERBINDUNGEN
COMBINAISON DE THÉRAPIE CONTRE LE CANCER AVEC DES COMPOSÉS INHIBITEURS DE HSP90

(30) Priority: 19.10.2009 US 279330 P; 11.01.2010 US 335778 P
(43) Date of publication of application: 29.08.2012
(73) Proprietor: Synta Pharmaceuticals Corp., Lexington, MA 02421 (US)
(72) Inventor: BLACKMAN, Ronald, K., Brookline Massachusetts 02446 (US); FOLEY, Kevin, Paul, Landsdale Pennsylvania 19446 (US); PROIA, David, Newton Massachusetts 02458 (US)
(74) Representative: Potter Clarkson LLP
(86) International application number: PCT/US2010/053199
(87) International publication number: WO 2011/049946

(56) References cited:
- WO-A1-2008/086857
- WO-A1-2008/108386
- WO-A1-2009/023211
- US-A1- 2008 004 266
- NAGOURNEY ROBERT A ET AL: "Geldenamycin and 17-allylamino-17-demethoxygeldenamycin alone and in combination with cytotoxic drugs in human tumor primary cultures.", PROCEEDINGS OF THE AMERICAN ASSOCIATION FOR CANCER RESEARCH ANNUAL MEETING, vol. 46, April 2005 (2005-04), page 404, XP002612414, & 96TH ANNUAL MEETING OF THE AMERICAN-ASSOCIATION-FOR-CANCER-RESEARCH; ANAHEIM, CA, USA; APRIL 16 -20, 2005 ISSN: 0197-016X
- SAWAI AYANA ET AL: "Inhibition of Hsp90 down-regulates mutant epidermal growth factor receptor (EGFR) expression and sensitizes EGFR mutant tumors to paclitaxel", CANCER RESEARCH, vol. 68, no. 2, January 2008 (2008-01), pages 589-596, XP002612415, ISSN: 0008-5472

## Description

### BACKGROUND OF THE INVENTION

Cancer continues to be a leading cause of death in the United States and around the world. As such, there is a continuing need for therapies against cancer.

### SUMMARY OF THE INVENTION

It is now found that certain triazolone HSP90 inhibitors and taxane combinations are surprisingly effective at treating subjects with non-small cell lung cancer, colon carcinoma and erythroleukemia without further increasing side effects. The particular combination therapies disclosed herein demonstrate surprising biological activity by demonstrating significant anticancer effects while showing minimal side effects.

The present invention utilizes triazolone compounds which inhibit the activity of Hsp90 and are useful in the treatment of lung cancer disorders (*e.g.,* non-small cell lung cancer), colon carcinoma and erythroleukemia in combination with a taxane compound. The present invention comprises compounds for uses as described in the attached claims. This includes compounds for use in a method of treating a subject with lung cancer (*e.g.,* non-small cell lung cancer), colon carcinoma or erythroleukemia includes the step of administering to the subject an HSP90 inhibitor described herein and a taxane. In one embodiment, the administration of the HSP90 inhibitor and the taxane are done concurrently. In another embodiment, the administration of the HSP90 inhibitor and the taxane are done sequentially. In any one of these embodiments, the taxane is docetaxel, paclitaxel or Abraxane^{™}. In any one of these embodiments, the HSP90 inhibitor is a compound represented in Table 1A or 2A. Also disclosed herein, the HSP90 inhibitor may be a compound represented in Table 1B or 2B.

In one embodiment, the invention includes the use of an HSP90 inhibitor described herein for the manufacture of a medicament for treating lung cancer (*e.g.,* non-small cell lung cancer), colon carcinoma or erythroleukemia in combination with a taxane.

In certain embodiments, the combination treatment utilizing an HSP90 compound described herein with other chemotherapeutic agents may help to prevent or reduce the development of multidrug resistant lung cancer (*e.g.,* non-small cell lung cancer), colon carcinoma or erythroleukemia cells in a mammal. In this embodiment, the compounds of the invention may allow a reduced efficacious amount of a second chemotherapeutic agent given to a mammal, because the HSP90 inhibitor should inhibit the development of multidrug resistant cancerous lung cancer (*e.g.,* non-small cell lung cancer), colon carcinoma or erythroleukemia. In one embodiment, the second chemotherapeutic agent is a taxane. In another embodiment, the taxane is paclitaxel, docetaxel or Abraxane^{®}.

The taxanes paclitaxel and docetaxel are widely used in the treatment of advanced-stage non-small cell lung cancer (NSCLC). To examine the combination of HSP90 inhibitors of the invention, such as Compound 1, used in combination with taxanes, *in vitro* studies were conducted with NCI-H1975 cells. Using the median-effect method of Chau and Talalay, Compound 1 in combination with either paclitaxel or docetaxel displayed combination index values within the synergistic range (0.23-0.65 CI) (see Example 2). *In vivo,* in the NCI-H1975 xenograft model, the combination of Compound 1 and paclitaxel displayed greater efficacy than either single agent alone (%T/C values of 7, 38 and 55, for the combination, paclitaxel and Compound 1, respectively) (see Example 1). Synergy between Compound 1 and paclitaxel was not due to alterations in the pharmacokinetics of either agent, and the combination treatment did not result in additional toxicity. Synergy was also demonstrated with HT29 colon carcinoma cells and HEL92.1.7 erythroleukemia cells (Example 3).

These results demonstrate that Compound 1 is a highly potent Hsp90 inhibitor that displays broad *in vitro* and *in vivo* anti-cancer activity in preclinical models of NSCLC, colon carcinoma and erythroleukemia. HSP90 inhibitors of the invention, such as Compound 1, also synergize with paclitaxel and docetaxel.

### BRIEF DESCRIPTION OF THE FIGURES

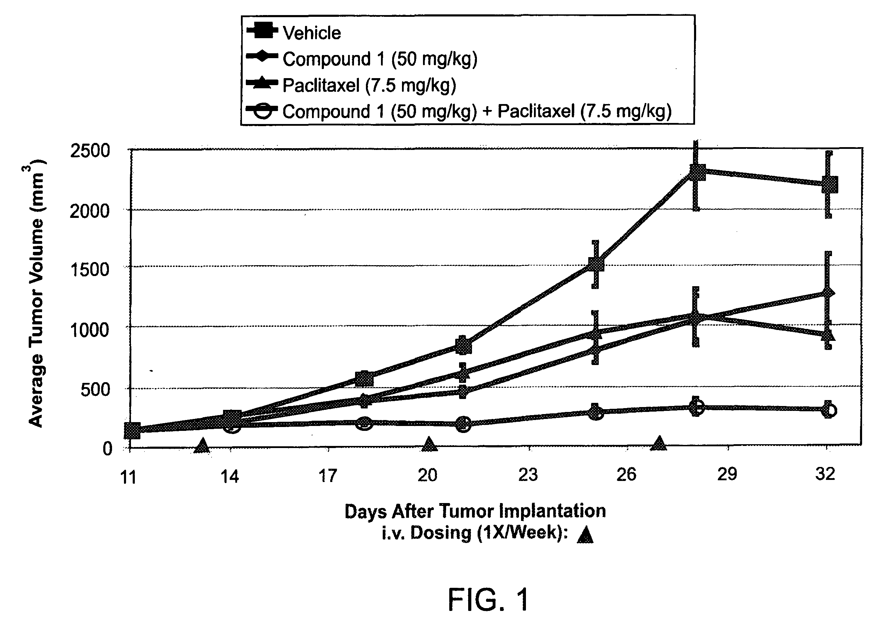

Figure 1 shows a SCID mouse xenograft study conducted to determine the effects of the combination of Compound 1 plus paclitaxel on the *in vivo* growth rate of the human NSCLC cell line NCI-H1975. Tumor-bearing animals (8 mice/group) were i.v. injected 1 time per week for a total of 3 doses (arrowheads) with vehicle alone, Compound 1 alone, paclitaxel alone or a combination of Compound 1 and paclitaxel dosed concurrently. The average tumor volumes for each group (error bars represent SEM) were determined every 3-4 days. Treatment with a dose of 50 mg/kg body weight of Compound 1 moderately inhibited tumor growth, with a %T/C value of 55 observed on day 32. Treatment with a dose of 7.5 mg/kg body weight of paclitaxel moderately inhibited tumor growth, with a %T/C value of 38 observed on day 32. Concurrent treatment with a combination of 50 mg/kg body weight of Compound 1 plus 7.5 mg/kg body weight paclitaxel dramatically inhibited tumor growth, with a %T/C value of 7 observed on day 32. The efficacy observed for the combination treatment group was significantly greater than for either single agent alone (*P* < 0.05; one-way ANOVA). Overt toxicity was not observed, with the Compound I plus paclitaxel combination treatment group having an average bodyweight change on day 29 (last day measured) relative to the start of the study of +3.1% (+/- 1.2 SEM), as compared to +5.1% (+/- 1.4 SEM) for the vehicle-treated group.
Figure 2 shows that the combination treatment detailed in Figure 1 did not result in additional toxicity relative to the single agents, with only minimal effects on cumulative average body weight changes over the course of the study.
Figure 3 shows that Compound 1 did not affect the plasma exposure of paclitaxel in CD-1 Nude mice. Error bars represent +/-
Figure 4 shows that paclitaxel did not affect the plasma exposure of Compound 1 in CD-1 Nude mice.
Figure 5 shows the half maximal inhibitory concentration (IC₅₀) for paclitaxel and Compound 1 determined using three-fold serial dilutions of compound starting with a top concentration of 1 µM. The single agent IC₅₀ value for Compound 1 in H 1975 was calculated at 15 nM; for paclitaxel the IC₅₀ was 7 nM.
Figure 6 demonstrates the result of concurrent treatment with the combination of paclitaxel and Compound 1 in H1975 cells.
Figure 7 demonstrates the result of a sequential treatment regimen: initial treatment with paclitaxel followed after 24 hours by treatment with Compound 1 in H1975 cells.
Figure 8 shows the percent of H1975 cells killed by Compound 1 (Cmpd 1), paclitaxel or the combination of the two drugs at indicated concentrations.
Figures 9a-b depict the median-effect method of Chau and Talalay (4) and CalcuSyn 2.0 software (Biosoft, Cambridge, UK) were used to examine the interaction between Compound 1 and paclitaxel or docetaxel. Different concentrations of each drug were added concurrently to NCI-H1975 cells for 72 hr and viability was measured by alamarBlue^{®} assay (Invitrogen). (A)

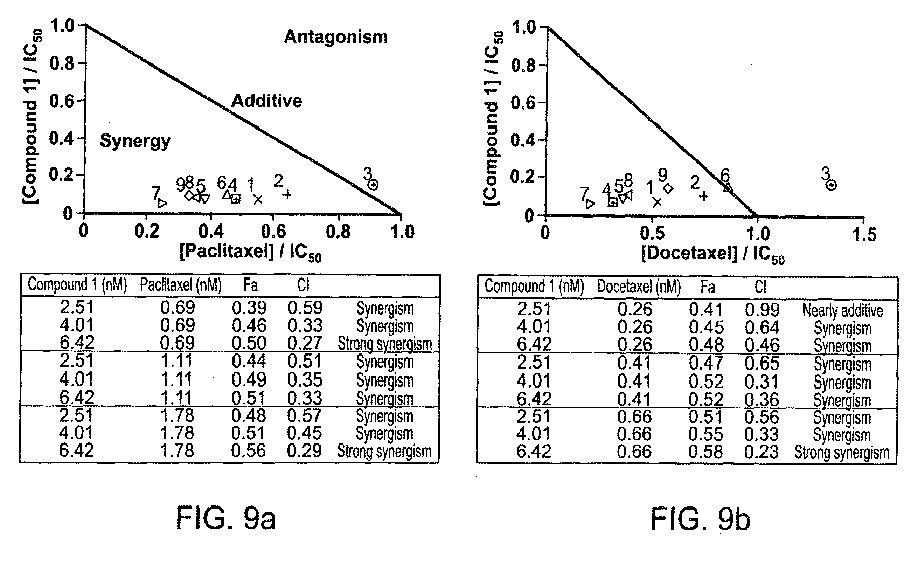

Figure 9a shows the isobologram and Combination Index analysis of Compound 1 in combination with paclitaxel using NCI-H1975 cells. Data points below the red line in the isobologram indicate synergy, whereas data points above the red line indicate antagonism between the two drugs. Combination Index (CI) values < 1 indicate synergy, whereas CI > 1 indicates antagonism between two drugs. Similar results were also observed using HCC827 cells (data not shown). Compound 1 was found to potently synergize with paclitaxel. (B)
Figure 9b shows a isobologram and Combination Index analysis of Compound 1 in combination with docetaxel using NCI-H1975 cells as above. Compound 1 was found to potently synergize with docetaxel.

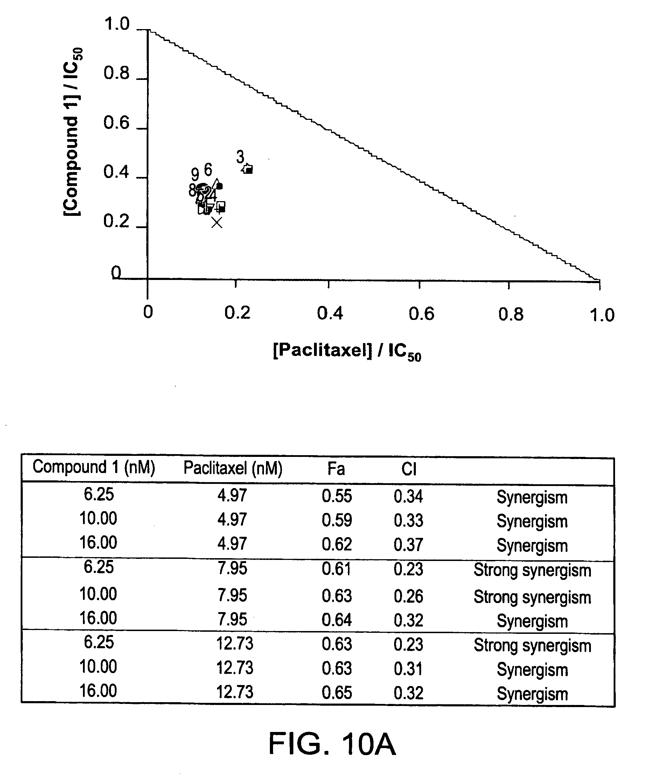

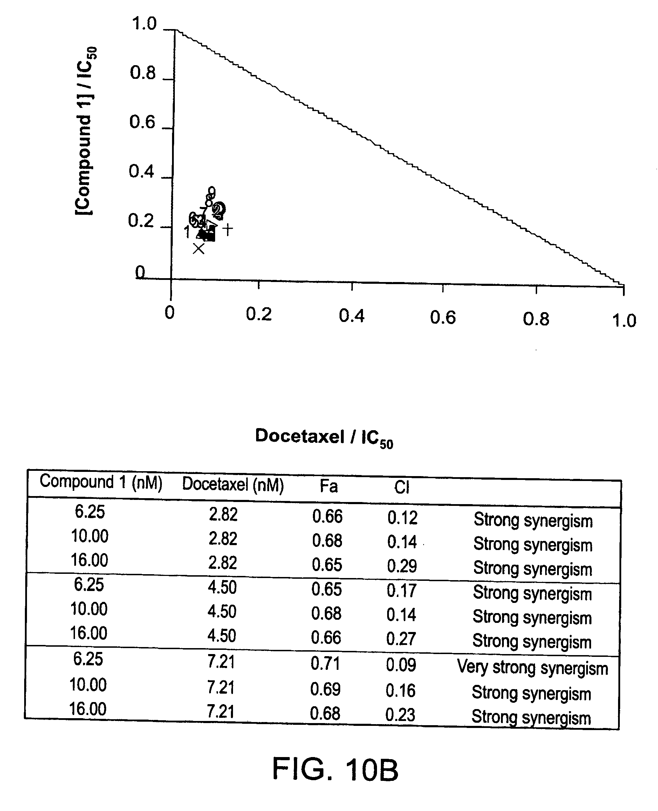

Figure 10 shows normalized isobolograms (top panels) and CI values (bottom panels) for the concurrent treatment of paclitaxel (A) or docetaxel (B) with Compound 1 in HEL92.1.7 cells.

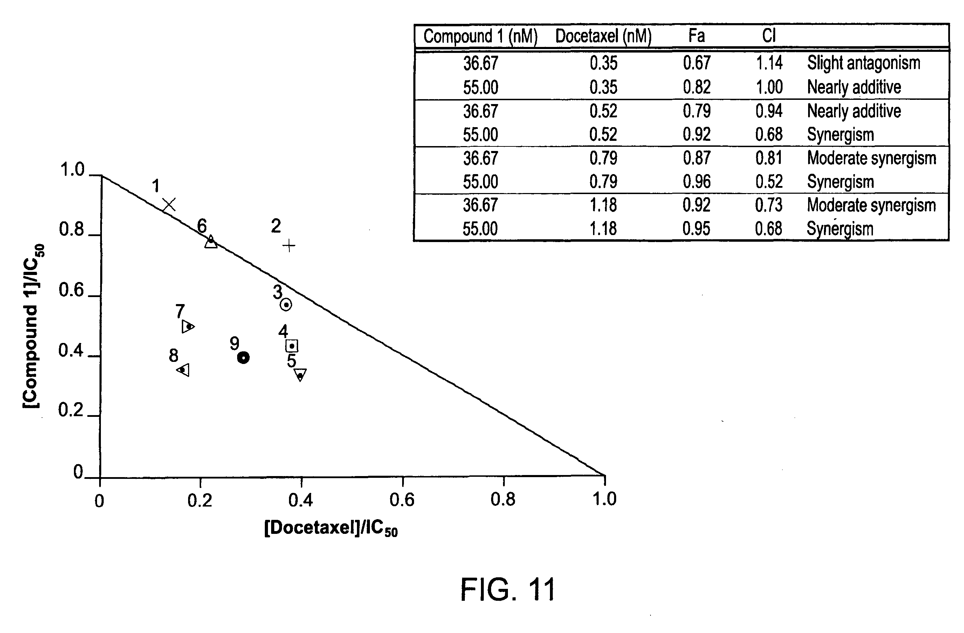

Figure 11 shows normalized isobolograms (left panel) and CI values (right panel) for the concurrent treatment of docetaxel with Compound 1 in HT29 cells.

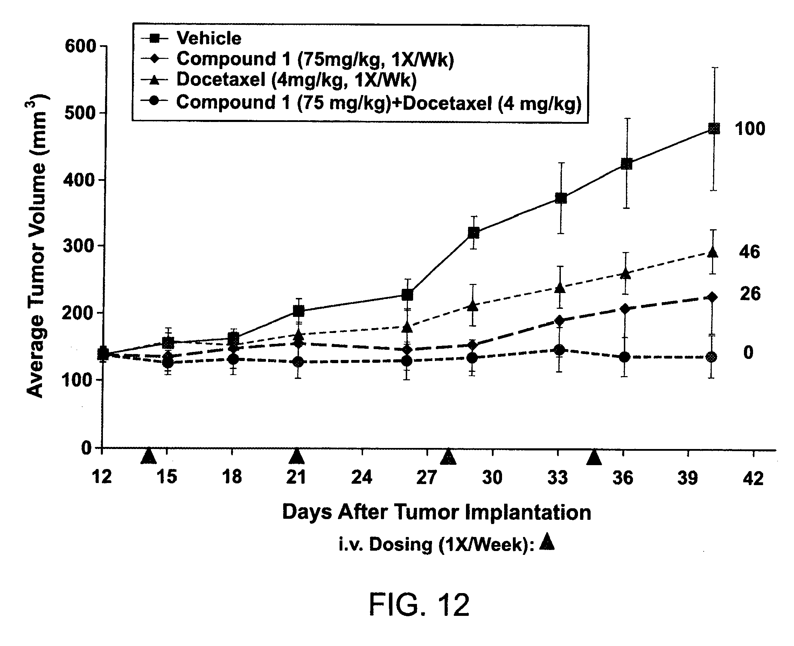

Figure 12 is a graph showing the effect of the combination of 75 mg/kg Compound 1 and 4 mg/kg docetaxel dosed 1X/week in the NCI-HCC827 NSCLC model. %T/C values for day 40 are indicated on the right. Error bars represent + or - 0.5 SEM.

### DETAILED DESCRIPTION OF THE INVENTION

Unless otherwise specified, the below terms used herein are defined as follows: As used herein, the term "alkyl" means a saturated, straight chain or branched, non-cyclic hydrocarbon having from I to 10 carbon atoms. Representative straight chain alkyls include methyl, ethyl, n-propyl, n-butyl, n-pentyl, n-hexyl, n-heptyl, n-octyl, n-nonyl and n-decyl; while representative branched alkyls include isopropyl, sec-butyl, isobutyl, tert-butyl, isopentyl, 2-methylbutyl, 3-methylbutyl, 2-methylpentyl, 3-methylpentyl, 4-methylpentyl, 2-methylhexyl, 3-methylhexyl, 4-methylhexyl, 5-methylhexyl, 2,3-dimethylbutyl, 2,3-dimethylpentyl, 2,4-dimethylpentyl, 2,3-dimethylhexyl, 2,4-dimethylhexyl, 2,5-dimethylhexyl, 2,2-dimethylpentyl, 2,2-dimethylhexyl, 3,3-dimtheylpentyl, 3,3-dimethylhexyl, 4,4-dimethylhexyl, 2-ethylpentyl, 3-ethylpentyl, 2-ethylhexyl, 3-ethylhexyl, 4-ethylhexyl, 2-methyl-2-ethylpentyl, 2-methyl-3-ethylpentyl, 2-methyl-4-ethylpentyl, 2-methyl-2-ethylhexyl, 2-methyl-3-ethylhexyl, 2-methyl-4-ethylhexyl, 2,2-diethylpentyl, 3,3-diethylhexyl, 2,2-diethylhexyl, 3,3-diethylhexyl, and the like. The term "(C₁-C₆)alkyl" means a saturated, straight chain or branched, non-cyclic hydrocarbon having from 1 to 6 carbon atoms. Alkyl groups included in compounds of this invention may be optionally substituted with one or more substituents.

As used herein, the term "alkenyl" means a straight chain or branched, non-cyclic hydrocarbon having from 2 to 10 carbon atoms and having at least one carbon-carbon double bond. Representative straight chain and branched (C₂-C₁₀)alkenyls include vinyl, allyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, 1-hexenyl, 2-hexenyl, 3-hexenyl, 1-heptenyl, 2-heptenyl, 3-heptenyl, 1-octenyl, 2-octenyl, 3-octenyl, 1-nonenyl, 2-nonenyl, 3-nonenyl, 1-decenyl, 2-decenyl, 3-decenyl, and the like. Alkenyl groups included in compounds of the invention may be optionally substituted with one or more substituents.

As used herein, the term "alkynyl" means a straight chain or branched, non-cyclic hydrocarbon having from 2 to 10 carbon atoms and having at least one carbon-carbon triple bond. Representative straight chain and branched alkynyls include acetylenyl, propynyl, 1-butynyl, 2-butynyl, 1-pentynyl, 2-pentynyl, 3-methyl-1-butynyl, 4-pentynyl, 1-hexynyl, 2-hexynyl, 5-hexynyl, 1-heptynyl, 2-heptynyl, 6-heptynyl, 1-octynyl, 2-octynyl, 7-octynyl, 1-nonynyl, 2-nonynyl, 8-nonynyl, 1-decynyl, 2-decynyl, 9-decynyl, and the like. Alkynyl groups included in compounds of the invention may be optionally substituted with one or more substituents.

As used herein, the term "cycloalkyl" means a saturated, mono- or polycyclic, non-aromatic hydrocarbon having from 3 to 20 carbon atoms. Representative cycloalkyls include cyclopropyl, 1-methylcyclopropyl, cyclobutyl, cyclopentyl, cyclohexyl, cycloheptyl, cyclooctyl, cyclononyl, cyclodecyl, octahydropentalenyl, and the like. Cycloalkyl groups included in compounds of the invention may be optionally substituted with one or more substituents.

As used herein, the term "cycloalkenyl" means a mono- or polycyclic, non-aromatic hydrocarbon having at least one carbon-carbon double bond in the cyclic system and having from 3 to 20 carbon atoms. Representative cycloalkenyls include cyclopentenyl, cyclopentadienyl, cyclohexenyl, cyclohexadienyl, cycloheptenyl, cycloheptadienyl, cycloheptatrienyl, cyclooctenyl, cyclooctadienyl, cyclooctatrienyl, cyclooctatetraenyl, cyclononenyl, cyclononadienyl, cyclodecenyl, cyclodecadienyl, 1,2,3,4,5,8-hexahydronaphthalenyl, and the like. Cycloalkenyl groups included in compounds of the invention may be optionally substituted with one or more substituents.

As used herein, the term "alkylene" refers to an alkyl group that has two points of attachment. The term "(C₁-C₆)alkylene" refers to an alkylene group that has from one to six carbon atoms. Straight chain (C₁-C₆)alkylene groups are preferred. Non-limiting examples of alkylene groups include methylene (-CH₂-), ethylene (-CH₂CH₂-), n-propylene (-CH₂CH₂CH₂-), isopropylene (-CH₂CH(CH₃)-), and the like. Alkylene groups included in compounds of this invention may be optionally substituted with one or more substituents.

As used herein, the term "lower" refers to a group having up to four atoms. For example, a "lower alkyl" refers to an alkyl radical having from 1 to 4 carbon atoms, "lower alkoxy" refers to "-O-(C₁-C₄)alkyl and a "lower alkenyl" or "lower alkynyl" refers to an alkenyl or alkynyl radical having from 2 to 4 carbon atoms.

As used herein, the term "haloalkyl" means an alkyl group, in which one or more, including all, the hydrogen radicals are replaced by a halo group(s), wherein each halo group is independently selected from -F, -Cl, -Br, and -I. For example, the term "halomethyl" means a methyl in which one to three hydrogen radical(s) have been replaced by a halo group. Representative haloalkyl groups include trifluoromethyl, bromomethyl, 1,2-dichloroethyl, 4-iodobutyl, 2-fluoropentyl, and the like.

As used herein, an "alkoxy" is an alkyl group which is attached to another moiety via an oxygen linker. Alkoxy groups included in compounds of this invention may be optionally substituted with one or more substituents.

As used herein, a "haloalkoxy" is a haloalkyl group which is attached to another moiety via an oxygen linker.

As used herein, the term an "aromatic ring" or "aryl" means a mono- or polycyclic hydrocarbon, containing from 6 to 15 carbon atoms, in which at least one ring is aromatic. Examples of suitable aryl groups include, but are not limited to, phenyl, tolyl, anthracenyl, fluorenyl, indenyl, azulenyl, and naphthyl, as well as benzo-fused carbocyclic moieties such as 5,6,7,8-tetrahydronaphthyl. Aryl groups included in compounds of this invention may be optionally substituted with one or more substituents. In one embodiment, the aryl group is a monocyclic ring, wherein the ring comprises 6 carbon atoms, referred to herein as "(C₆)aryl."

As used herein, the term "aralkyl" means an aryl group that is attached to another group by a (C₁-C₆)alkylene group. Representative aralkyl groups include benzyl, 2-phenyl-ethyl, naphth-3-yl-methyl and the like. Aralkyl groups included in compounds of this invention may be optionally substituted with one or more substituents.

As used herein, the term "heterocyclyl" means a monocyclic or a polycyclic, saturated or unsaturated, non-aromatic ring or ring system which typically contains 5- to 20-members and at least one heteroatom. A heterocyclic ring system can contain saturated ring(s) or unsaturated non-aromatic ring(s), or a mixture thereof. A 3- to 10-membered heterocycle can contain up to 5 heteroatoms, and a 7- to 20-membered heterocycle can contain up to 7 heteroatoms. Typically, a heterocycle has at least one carbon atom ring member. Each heteroatom is independently selected from nitrogen, which can be oxidized (*e.g.,* N(O)) or quaternized, oxygen and sulfur, including sulfoxide and sulfate. The heterocycle may be attached via any heteroatom or carbon atom. Representative heterocycles include morpholinyl, thiomorpholinyl, pyrrolidinonyl, pyrrolidinyl, piperidinyl, piperazinyl, hydantoinyl, valerolactamyl, oxiranyl, oxetanyl, tetrahydrofuranyl, tetrahydropyranyl, tetrahydropyrindinyl, tetrahydropyrimidinyl, tetrahydrothiophenyl, tetrahydrothiopyranyl, and the like. A heteroatom may be substituted with a protecting group known to those of ordinary skill in the art, for example, a nitrogen atom may be substituted with a tert-butoxycarbonyl group. Furthermore, the heterocyclyl included in compounds of this invention may be optionally substituted with one or more substituents. Only stable isomers of such substituted heterocyclic groups are contemplated in this definition.

As used herein, the term "heteroaromatic", "heteroaryl", or like terms, means a monocyclic or a polycyclic, unsaturated radical containing at least one heteroatom, in which at least one ring is aromatic. Polycyclic heteroaryl rings must contain at least one heteroatom, but not all rings of a polycyclic heteroaryl moiety must contain heteroatoms. Each heteroatom is independently selected from nitrogen, which can be oxidized (e.g., N(O)) or quaternized, oxygen and sulfur, including sulfoxide and sulfone. Representative heteroaryl groups include pyridyl, 1-oxo-pyridyl, furanyl, benzo[1,3]dioxolyl, benzo[1,4]dioxinyl, thienyl, pyrrolyl, oxazolyl, imidazolyl, thiazolyl, a isoxazolyl, quinolinyl, pyrazolyl, isothiazolyl, pyridazinyl, pyrimidinyl, pyrazinyl, a triazinyl, triazolyl, thiadiazolyl, isoquinolinyl, indazolyl, benzoxazolyl, benzofuryl, indolizinyl, imidazopyridyl, tetrazolyl, benzimidazolyl, benzothiazolyl, benzothiadiazolyl, benzoxadiazolyl, indolyl, tetrahydroindolyl, azaindolyl, imidazopyridyl, quinazolinyl, purinyl, pyrrolo[2,3]pyrimidinyl, pyrazolo[3,4]pyrimidinyl, imidazo[1,2-a]pyridyl, and benzothienyl. In one embodiment, the heteroaromatic ring is selected from 5-8 membered monocyclic heteroaryl rings. The point of attachment of a heteroaromatic or heteroaryl ring may be at either a carbon atom or a heteroatom. Heteroaryl groups included in compounds of this invention may be optionally substituted with one or more substituents. As used herein, the term "(C₅)heteroaryl" means an heteroaromatic ring of 5 members, wherein at least one carbon atom of the ring is replaced with a heteroatom, such as, for example, oxygen, sulfur or nitrogen. Representative (C₅)heteroaryls include furanyl, thienyl, pyrrolyl, oxazolyl, imidazolyl, thiazolyl, isoxazolyl, pyrazolyl, isothiazolyl, pyrazinyl, triazolyl, thiadiazolyl, and the like. As used herein, the term "(C₆)heteroaryl" means an aromatic heterocyclic ring of 6 members, wherein at least one carbon atom of the ring is replaced with a heteroatom such as, for example, oxygen, nitrogen or sulfur. Representative (C₆)heteroaryls include pyridyl, pyridazinyl, pyrazinyl, triazinyl, tetrazinyl, and the like.

As used herein, the term "heteroaralkyl" means a heteroaryl group that is attached to another group by a (C₁-C₆)alkylene. Representative heteroaralkyls include 2-(pyridin-4-yl)-propyl, 2-(thien-3-yl)-ethyl, imidazol-4-yl-methyl, and the like. Heteroaralkyl groups included in compounds of this invention may be optionally substituted with one or more substituents.

As used herein, the term "halogen" or "halo" means -F, -Cl, -Br or -I.

As used herein the term "heteroalkyl" means a straight or branched alkyl group wherein one or more of the internal carbon atoms in the chain is replaced by a heteroatom. For example, a heteroalkyl is represented by the formula -[CH₂]ₓ-Z-[CH₂]_{y}[CH₃], wherein x is a positive integer and y is zero or a positive integer, Z is O, NR, S, S(O), or S(O)₂, and wherein replacement of the carbon atom does not result in a unstable compound. Heteroalkyl groups included in compounds of this invention may be optionally substituted with one or more substituents.

Suitable substituents for an alkyl, alkylene, alkenyl, alkynyl, cycloalkyl, cycloalkenyl, heterocyclyl, aryl, aralkyl, heteroaryl, and heteroaralkyl groups include are those substituents which form a stable compound of the invention without significantly adversely affecting the reactivity or biological activity of the compound of the invention. Examples of substituents for an alkyl, alkylene, alkenyl, alkynyl, cycloalkyl, cycloalkenyl, heterocyclyl, aryl, aralkyl, heteroaryl, and heteroaralkyl include an alkyl, alkenyl, alkynyl, cycloalkyl, cycloalkenyl, heterocyclyl, aryl, heteroaryl, aralkyl, heteraralkyl, heteroalkyl, alkoxy, (each of which can be optionally and independently substituted), -C(O)NR²⁸R²⁹, -C(S)NR²⁸R²⁹, -C(NR³²)NR²⁸R²⁹, -NR³³C(O)R³¹, -NR³³C(S)R³¹, NR³³C(NR³²)R³¹, halo, -OR³³, cyano, nitro, -C(O)R³³, -C(S)R³³, -C(NR³²)R³³, -NR²⁸R²⁹, -C(O)OR³³, -C(S)OR³³, -C(NR³²)OR³³, -OC(O)R³³, -OC(S)R³³, -OC(NR³²)R³³, -NR³⁰C(O)NR²⁸R²⁹, -NR³³C(S)NR²⁸R²⁹, -NR³³C(NR³²)NR²⁸R²⁹, -OC(O)NR²⁸R²⁹, -OC(S)NR²⁸R²⁹, -OC(NR³²)NR²⁸R²⁹, -NR³³C(O)OR³¹, -NR³³C(S)OR³¹, -NR³³C(NR³²)OR³¹, -S(O)ₚR³³, -OS(O)ₚR³³, -NR³³S(O)ₚR³³, -S(O)ₚNR²⁸R²⁹, -OS(O)ₚNR²⁸R²⁹, -NR³³S(O)ₚNR²⁸R²⁹, guanidino, -C(O)SR³¹, -C(S)SR³¹, -C(NR³²)SR³¹, -OC(O)OR³¹, -OC(S)OR³¹, -OC(NR³²)OR³¹, -SC(O)R³³, -SC(O)OR³¹, -SC(NR³²)OR³¹, -SC(S)R³³, -SC(S)OR³¹, -SC(O)NR²⁸R²⁹, -SC(NR³²)NR²⁸R²⁹, -SC(S)NR²⁸R²⁹, -SC(NR³²)R³³, -OS(O)ₚOR³¹, -S(O)ₚOR³¹, -NR³⁰S(O)ₚOR³¹, -SS(O)ₚR³³, -SS(O)ₚOR³¹, -SS(O)ₚNR²⁸R²⁹, -OP(O)(OR³¹)₂, or -SP(O)(OR³¹)₂. In addition, any saturated portion of an alkyl, cycloalkyl, alkylene, heterocyclyl, alkenyl, cycloalkenyl, alkynyl, aralkyl and heteroaralkyl groups, may also be substituted with =O, =S, or =N-R³².

Each R²⁸ and R²⁹ is independently H, alkyl, alkenyl, alkynyl, cycloalkyl, cycloalkenyl, heterocyclyl, aryl, heteroaryl, aralkyl, or heteraralkyl, wherein each alkyl, alkenyl, alkynyl, cycloalkyl, cycloalkenyl, heterocyclyl, aryl, heteroaryl, aralkyl, or heteroalkyl represented by R²⁸ or R²⁹ is optionally and independently substituted.

Each R³¹ and R³³ is independently H, alkyl, alkenyl, alkynyl, cycloalkyl, cycloalkenyl, heterocyclyl, aryl, heteroaryl, aralkyl, or heteraralkyl, wherein each alkyl, alkenyl, alkynyl, cycloalkyl, cycloalkenyl, heterocyclyl, aryl, heteroaryl, aralkyl, and heteraralkyl represented by R³¹ or R³³ is optionally and independently unsubstituted.

Each R³² is independently H, alkyl, alkenyl, alkynyl, cycloalkyl, cycloalkenyl, heterocyclyl, aryl, heteroaryl, aralkyl, heteraralkyl, -C(O)R³³, -C(O)NR²⁸R²⁹, -S(O)ₚR³³, or -S(O)ₚNR²⁸R²⁹, wherein each alkyl, alkenyl, alkynyl, cycloalkyl, cycloalkenyl, heterocyclyl, aryl, heteroaryl, aralkyl and heteraralkyl represented by R³² is optionally and independently substituted.

The variable p is 0, 1 or 2.

When a heterocyclyl, heteroaryl or heteroaralkyl group contains a nitrogen atom, it may be substituted or unsubstituted. When a nitrogen atom in the aromatic ring of a heteroaryl group has a substituent, the nitrogen may be oxidized or a quaternary nitrogen.

As used herein, the terms "subject", "patient" and "mammal" are used interchangeably. The terms "subject" and "patient" refer to an animal (e.g., a bird such as a chicken, quail or turkey, or a mammal), preferably a mammal including a non-primate (*e.g.,* a cow, pig, horse, sheep, rabbit, guinea pig, rat, cat, dog, and mouse) and a primate *(e.g.,* a monkey, chimpanzee and a human), and more preferably a human. In one embodiment, the subject is a non-human animal such as a farm animal (*e.g.,* a horse, cow, pig or sheep), or a pet *(e.g.,* a dog, cat, guinea pig or rabbit). In a preferred embodiment, the subject is a human.

As used herein, the term "compound(s) of this invention" and similar terms refers to a compound selected from 3-(2,4-dihydroxy-5-isopropyl-phenyl)-4-(1-methyl-indol-5-yl)-5-hydroxy-[1,2,4]triazole and 5-hydroxy-4-(5-hydroxy-4-(1-methyl-1H-indol-5-yl)-4H-1,2,4-triazol-3-yl)-2-isopropylphenyl dihydrogen phosphate or a tautomer, or a pharmaceutically acceptable salt thereof. Also disclosed herein are compounds of formulae (I)-(III) or (Ia)-(IIIa) or a compound in Table 1B or 2B or a pharmaceutically acceptable salt thereof.

Some of the disclosed uses can be particularly effective at treating subjects whose cancer has become "drug resistant" or "multi-drug resistant". A cancer which initially responded to an anti-cancer drug becomes resistant to the anti-cancer drug when the anti-cancer drug is no longer effective in treating the subject with the cancer. For example, many tumors will initially respond to treatment with an anti-cancer drug by decreasing in size or even going into remission, only to develop resistance to the drug. "Drug resistant" tumors are characterized by a resumption of their growth and/or reappearance after having seemingly gone into remission, despite the administration of increased dosages of the anti-cancer drug. Cancers that have developed resistance to two or more anti-cancer drugs are said to be "multi-drug resistant". For example, it is common for cancers to become resistant to three or more anti-cancer agents, often five or more anti-cancer agents and at times ten or more anti-cancer agents.

As used herein, the term "pharmaceutically acceptable salt" refers to a salt prepared from a compound of any one of formulae (I)-(III) or (Ia)-(IIIa) or a compound in Table 1A, 1B, 2A or 2B having an acidic functional group, such as a carboxylic acid functional group, and a pharmaceutically acceptable inorganic or organic base. Suitable bases include, but are not limited to, hydroxides of alkali metals such as sodium, potassium, and lithium; hydroxides of alkaline earth metal such as calcium and magnesium; hydroxides of other metals, such as aluminum and zinc; ammonia, and organic amines, such as unsubstituted or hydroxy-substituted mono-, di-, or trialkylamines; dicyclohexylamine; tributyl amine; pyridine; N-methyl, N-ethylamine; diethylamine; triethylamine; mono-, bis-, or tris-(2-hydroxy-lower alkyl amines), such as mono-, bis-, or tris-(2-hydroxyethyl)amine, 2-hydroxy-tert-butylamine, or tris-(hydroxymethyl)methylamine, N, N,-di-lower alkyl-N-(hydroxy lower alkyl)-amines, such as N,N-dimethyl-N-(2-hydroxyethyl)amine, or tri-(2-hydroxyethyl)amine; N-methyl-D-glucamine; and amino acids such as arginine, lysine, and the like. The term "pharmaceutically acceptable salt" also refers to a salt prepared from a compound of any one of formulae (I)-(III) or (Ia)-(IIIa) or a compound in Table 1A, 1B, 2A or 2B having a basic functional group, such as an amine functional group, and a pharmaceutically acceptable inorganic or organic acid. Suitable acids include, but are not limited to, hydrogen sulfate, citric acid, acetic acid, oxalic acid, hydrochloric acid (HCl), hydrogen bromide (HBr), hydrogen iodide (HI), nitric acid, hydrogen bisulfide, phosphoric acid, isonicotinic acid, oleic acid, tannic acid, pantothenic acid, saccharic acid, lactic acid, salicylic acid, tartaric acid, bitartratic acid, ascorbic acid, succinic acid, maleic acid, besylic acid, fumaric acid, gluconic acid, glucaronic acid, formic acid, benzoic acid, glutamic acid, methanesulfonic acid, ethanesulfonic acid, benzenesulfonic acid, pamoic acid and *p-*toluenesulfonic acid.

A pharmaceutically acceptable carrier may contain inert ingredients which do not unduly inhibit the biological activity of the compound(s). The pharmaceutically acceptable carriers should be biocompatible, *i.e.,* non-toxic, non-inflammatory, non-immunogenic and devoid of other undesired reactions upon the administration to a subject. Standard pharmaceutical formulation techniques can be employed, such as those described in REMINGTON, J. P., REMINGTON'S PHARMACEUTICAL SCIENCES (Mack Pub. Co., 17th ed., 1985). Suitable pharmaceutical carriers for parenteral administration include, for example, sterile water, physiological saline, bacteriostatic saline (saline containing about 0.9% mg/ml benzyl alcohol), phosphate-buffered saline, Hank's solution, Ringer's-lactate, and the like. Methods for encapsulating compositions, such as in a coating of hard gelatin or cyclodextran, are known in the art. *See* BAKER, ET AL., CONTROLLED RELEASE OF BIOLOGICAL ACTIVE AGENTS, (John Wiley and Sons, 1986).

As used herein, the term "effective amount" refers to an amount of a compound of this invention which is sufficient to reduce or ameliorate the severity, duration, progression, or onset of a disease or disorder, delay onset of a disease or disorder, retard or halt the advancement of a disease or disorder, cause the regression of a disease or disorder, prevent or delay the recurrence, development, onset or progression of a symptom associated with a disease or disorder, or enhance or improve the therapeutic effect(s) of another therapy. The precise amount of compound administered to a subject will depend on the mode of administration, the type and severity of the disease or condition and on the characteristics of the subject, such as general health, age, sex, body weight and tolerance to drugs. For example, for a proliferative disease or disorder, determination of an effective amount will also depend on the degree, severity and type of cell proliferation. The skilled artisan will be able to determine appropriate dosages depending on these and other factors. When co-administered with other therapeutic agents, *e.g.,* when co-administered with an anti-cancer agent, an "effective amount" of any additional therapeutic agent(s) will depend on the type of drug used. Suitable dosages are known for approved therapeutic agents and can be adjusted by the skilled artisan according to the condition of the subject, the type of condition(s) being treated and the amount of a compound of the invention being used. In cases where no amount is expressly noted, an effective amount should be assumed. Non-limiting examples of an effective amount of a compound of the invention are provided herein below. In a specific embodiment, the invention provides a compound for use in a method of treating, managing, or ameliorating NSCLC, colon carcinoma or erythroleukemia, or one or more symptoms thereof, said method comprising administering to a subject in need thereof a dose of at least 150 µg/kg, at least 250 µg/kg, at least 500 µg/kg, at least 1 mg/kg, at least 5 mg/kg, at least 10 mg/kg, at least 25 mg/kg, at least 50 mg/kg, at least 75 mg/kg, at least 100 mg/kg, at least 125 mg/kg, at least 150 mg/kg, or at least 200 mg/kg or more of one or more compounds of the invention once every day, once every 2 days, once every 3 days, once every 4 days, once every 5 days, once every 6 days, once every 7 days, once every 8 days, once every 10 days, once every two weeks, once every three weeks, or once a month. The daily dose can be administered in a single portion. Alternatively, the daily dose can be divided into portions (typically equal portions) administered two times, three times, four times or more per day.

The dosage of a therapeutic agent other than a compound of the invention, which has been or is currently being used to treat, manage, or ameliorate lung cancer (*e.g.,* NSCLC), colon carcinoma or erythroleukemia, or one or more symptoms thereof, can be used in the combination therapies of the invention. Preferably, the dosage of each individual therapeutic agent used in said combination therapy is lower than the dose of an individual therapeutic agent when given independently to treat, manage, or ameliorate a disease or disorder, or one or more symptoms thereof. The recommended dosages of therapeutic agents currently used for the treatment, management, or amelioration of a disease or disorder, or one or more symptoms thereof, can obtained from any reference in the art. *See, e.g.,* GOODMAN & GILMAN'S THE PHARMACOLOGICAL BASIS OF BASIS OF THERAPEUTICS 9TH ED, (Hardman, et al., Eds., NY:Mc-Graw-Hill (1996)); PHYSICIAN'S DESK REFERENCE 57TH ED. (Medical Economics Co., Inc., Montvale, NJ (2003)).

As used herein, the terms "treat", "treatment" and "treating" refer to the reduction or amelioration of the progression, severity and/or duration of a disease or disorder, delay of the onset of a disease or disorder, or the amelioration of one or more symptoms (preferably, one or more discernible symptoms) of a disease or disorder, resulting from the administration of one or more therapies (*e.g.,* one or more therapeutic agents such as a compound of the invention). The terms "treat", "treatment" and "treating" also encompass the reduction of the risk of developing a disease or disorder, and the delay or inhibition of the recurrence of a disease or disorder. In specific embodiments, the terms "treat", "treatment" and "treating" refer to the amelioration of at least one measurable physical parameter of a disease or disorder, such as growth of a tumor, not necessarily discernible by the patient. In other embodiments the terms "treat", "treatment" and "treating" refer to the inhibition of the progression of a disease or disorder, *e.g.,* lung cancer (*e.g.,* NSCLC), colon carcinoma or erythroleukemia, either physically by the stabilization of a discernible symptom, physiologically by the stabilization of a physical parameter, or both. In another embodiment, the terms "treat", "treatment" and "treating" of a proliferative disease or disorder refers to the reduction or stabilization of tumor size or cancerous cell count, and/or delay of tumor formation.

As used herein, the terms "therapeutic agent" and "therapeutic agents" refer to any agent(s) that can be used in the treatment of a disease or disorder, *e.g.* lung cancer (*e.g.,* NSCLC), colon carcinoma or erythroleukemia or one or more symptoms thereof. In certain embodiments, the term "therapeutic agent" refers to a compound of the invention. In certain other embodiments, the term "therapeutic agent" does not refer to a compound of the invention. Preferably, a therapeutic agent is an agent that is known to be useful for, or has been or is currently being used for the treatment of a disease or disorder, *e.g.,* lung cancer (*e.g.,* NSCLC), colon carcinoma or erythroleukemia, or one or more symptoms thereof.

As used herein, the term "synergistic" refers to a combination of a compound of the invention and another therapeutic agent, which, when taken together, is more effective than the additive effects of the individual therapies. A synergistic effect of a combination of therapies (*e.g.,* a combination of therapeutic agents) permits the use of lower dosages of one or more of the therapeutic agent(s) and/or less frequent administration of said agent(s) to a subject with a disease or disorder, *e.g.,* lung cancer *(e.g.,* NSCLC), colon carcinoma or erythroleukemia. The ability to utilize lower the dosage of one or more therapeutic agent and/or to administer said therapeutic agent less frequently reduces the toxicity associated with the administration of said agent to a subject without reducing the efficacy of said therapy in the treatment of a disease or disorder. In addition, a synergistic effect can result in improved efficacy of agents in the prevention, management or treatment of a disease or disorder, *e.g.* lung cancer *(e.g.,* NSCLC), colon carcinoma or erythroleukemia. Finally, a synergistic effect of a combination of therapies may avoid or reduce adverse or unwanted side effects associated with the use of either therapeutic agent alone.

As used herein, the term "in combination" refers to the use of more than one therapeutic agent. The use of the term "in combination" does not restrict the order in which said therapeutic agents are administered to a subject with lung cancer (*e.g.,* NSCLC), colon carcinoma or erythroleukemia. A first therapeutic agent, such as a compound of the invention, can be administered prior to *(e.g.,* 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to *(e.g.,* 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of a second therapeutic agent, such as an anti-cancer agent, to a subject with lung cancer (*e.g.,* NSCLC), colon carcinoma or erythroleukemia.

As used herein, the terms "therapies" and "therapy" can refer to any protocol(s), method(s), and/or agent(s) that can be used in the prevention, treatment, management, or amelioration of lung cancer (*e.g.,* NSCLC), colon carcinoma or erythroleukemia.

A used herein, a "protocol" includes dosing schedules and dosing regimens. The protocols herein are methods of use and include therapeutic protocols.

As used herein, a composition that "substantially" comprises a compound means that the composition contains more than about 80% by weight, more preferably more than about 90% by weight, even more preferably more than about 95% by weight, and most preferably more than about 97% by weight of the compound.

The compounds of the invention are defined herein by their chemical structures and/or chemical names. Where a compound is referred to by both a chemical structure and a chemical name, and the chemical structure and chemical name conflict, the chemical structure is determinative of the compound's identity.

Only those choices and combinations of substituents that result in a stable structure are contemplated. Such choices and combinations will be apparent to those of ordinary skill in the art and may be determined without undue experimentation.

The invention can be understood more fully by reference to the following detailed description and illustrative examples, which are intended to exemplify non-limiting embodiments of the invention.

The taxane is selected from paclitaxel or docetaxel. In particular embodiments, the taxane is paclitaxel intravenously administered in a weekly dose of about 94 µmol/m² (80 mg/m²).

Also disclosed herein, the taxanes employed may include paclitaxel analogs. Paclitaxel is a well-known anti-cancer drug which can act by enhancing and stabilizing microtubule formation. Thus, the term "paclitaxel analog" is defined herein to mean a compound which has the basic paclitaxel skeleton and which stabilizes microtubule formation. Many analogs of paclitaxel are known, including docetaxel, also referred to as "Taxotere^{®}". Paclitaxel and docetaxel have the respective structural formulas: 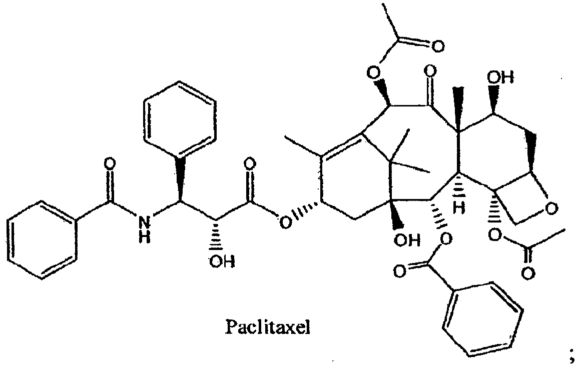 and 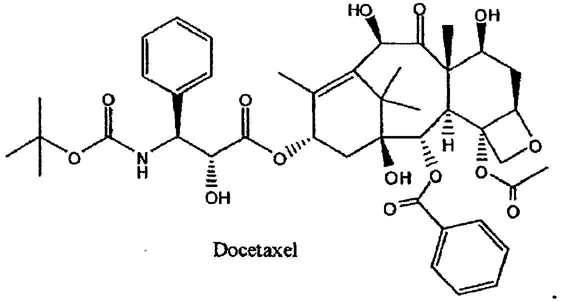 The taxanes employed in the disclosed invention have the basic taxane skeleton as a common structure feature shown below in Structural Formula **A**: 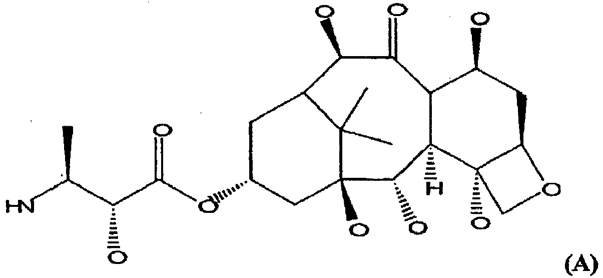 Double bonds have been omitted from the cyclohexane rings in the taxane skeleton represented by Structural Formula **A**. It is to be understood that the basic taxane skeleton can include zero or one double bond in one or both cyclohexane rings, as indicated in the paclitaxel analogs and Structural Formulas **B** and **C** below. A number of atoms have also been omitted from Structural Formula **A** to indicate sites in which structural variation commonly occurs among paclitaxel analogs.

A wide variety of substituents can decorate the taxane skeleton without adversely affecting biological activity. Also, zero, one or both of the cyclohexane rings of a paclitaxel analog can have a double bond at the indicated positions. For example, substitution on the taxane skeleton with simply an oxygen atom indicates that hydroxyl, acyl, alkoxy or other oxygen-bearing substituent is commonly found at the site. It is to be understood that these and other substitutions on the taxane skeleton can be made without losing the ability to enhance and stabilize microtubule formation. Thus, the term "paclitaxel analog" is defined herein to mean a compound which has the basic paclitaxel skeleton and which stabilizes microtubule formation. The term taxane is defined herein to include compounds such as paclitaxel and the paclitaxel analogs described herein, or a pharmaceutically acceptable salt or solvate thereof.

The taxanes disclosed herein may be represented by Structural Fonnula **B** or **C**:

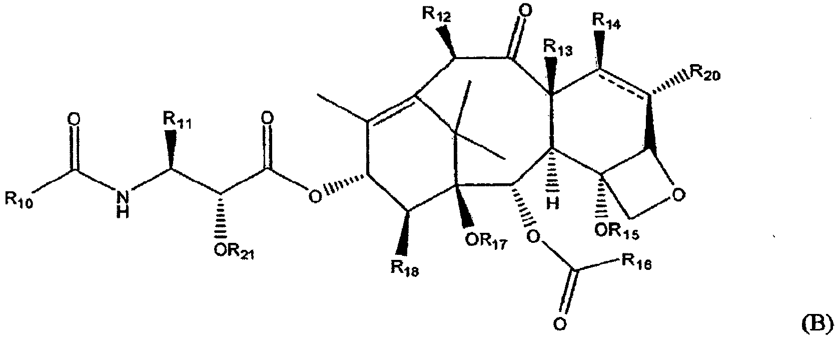

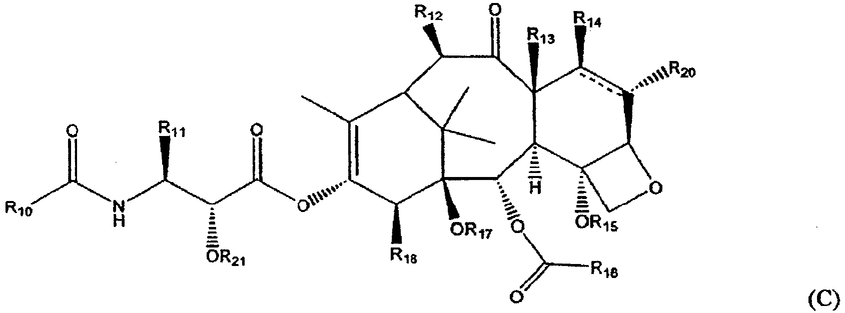

R₁₀ is an optionally substituted lower alkyl group, an optionally substituted phenyl group, -SR₁₉, -NHR₁₉ or -OR₁₉.

R₁₁ is an optionally substituted lower alkyl group, an optionally substituted aryl group.

R₁₂ is -H, -OH, lower alkyl, substituted lower alkyl, lower alkoxy, substituted lower alkoxy, -O-C(O)-(lower alkyl), -O-C(O)-(substituted lower alkyl), -O-CH₂-O-(lower alkyl) -S-CH₂-O-(lower alkyl).

R₁₃ is -H, -CH₃, or, taken together with R₁₄, -CH₂-.

R₁₄ is -H, -OH, lower alkoxy, -O-C(O)-(lower alkyl), substituted lower alkoxy, -O-C(O)-(substituted lower alkyl), -O-CH₂-O-P(O)(OH)₂, -O-CH₂-O-(lower alkyl), -O-CH₂-S-(lower alkyl) or, taken together with R₂₀, a double bond.

R₁₅ -H, lower acyl, lower alkyl, substituted lower alkyl, alkoxymethyl, alkthiomethyl, -C(O)-O(lower alkyl), -C(O)-O(substituted lower alkyl), -C(O)-NH(lower alkyl) or -C(O)-NH(substituted lower alkyl).

R₁₆ is phenyl or substituted phenyl.

R₁₇ is -H, lower acyl, substituted lower acyl, lower alkyl, substituted, lower alkyl, (lower alkoxy)methyl or (lower alkyl)thiomethyl.

R₁₈ -H, -CH₃ or, taken together with R₁₇ and the carbon atoms to which R₁₇ and R₁₈ are bonded, a five or six membered a non-aromatic heterocyclic ring.

R₁₉ is an optionally substituted lower alkyl group, an optionally substituted phenyl group.

R₂₀ is -H or a halogen.

R₂₁ is -H, lower alkyl, substituted lower alkyl, lower acyl or substituted lower acyl.

The variables in Structural Formulas **B** and **C** are defined as follows: R₁₀ is phenyl, *tert*-butoxy, -S-CH₂-CH-(CH₃)₂, -S-CH(CH₃)₃, -S-(CH₂)₃CH₃, -O-CH(CH₃)₃, -NH-CH(CH₃)₃, -CH=C(CH₃)₂ or *para*-chlorophenyl; R₁₁ is phenyl, (CH₃)₂CHCH₂-, -2-furanyl, cyclopropyl or *para*-toluyl; R₁₂ is -H, -OH, CH₃CO- or -(CH₂)₂-*N*-morpholino; R₁₃ is methyl, or, R₁₃ and R₁₄, taken together, are -CH₂-;
R₁₄ is -H, -CH₂SCH₃ or -CH₂-O-P(O)(OH)₂;
R₁₅ is CH₃CO-;
R₁₆ is phenyl; R₁₇ -H, or, R₁₇ and R₁₈, taken together, are -O-CO-O-;
R₁₈ is -H; R₂₀ is -H or -F; and R₂₁ is -H, -C(O)-CHBr-(CH₂)₁₃-CH₃ or -C(O)-(CH₂)₁₄-CH₃; -C(O)-CH₂-CH(OH)-COOH, -C(O)-CH₂-O-C(O)-CH₂CH(NH₂)-CONH₂, -C(O)-CH₂-O-CH₂CH₂OCH₃ or -C(O)-O-C(O)-CH₂CH₃.

Specific reference examples of paclitaxel analogs include the following compounds:

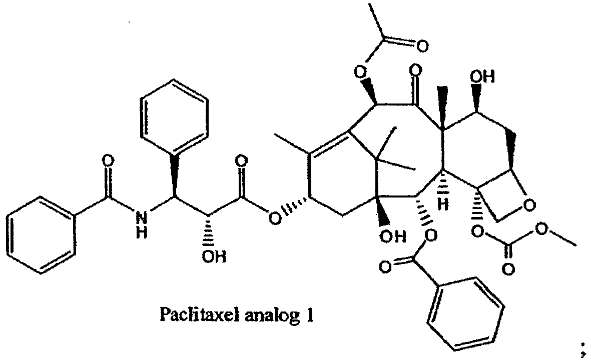

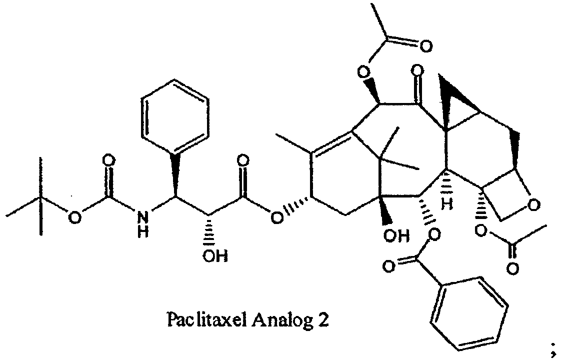

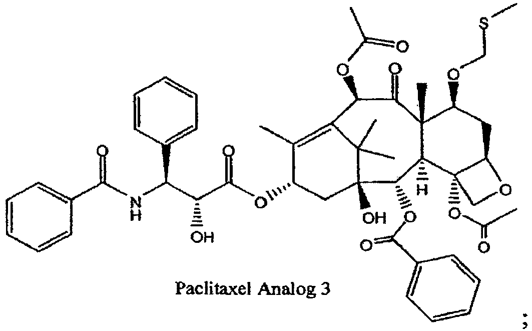

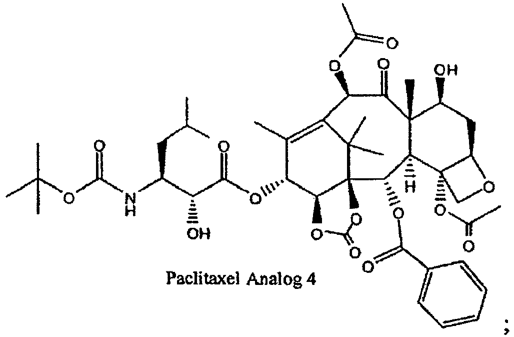

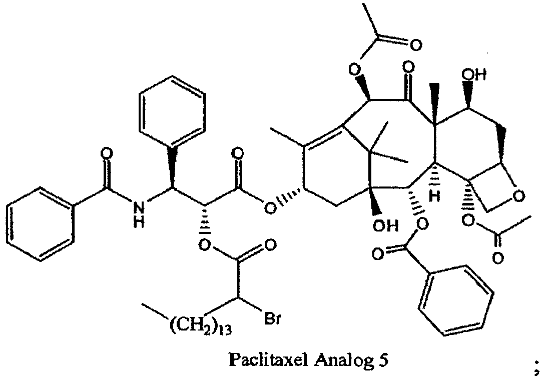

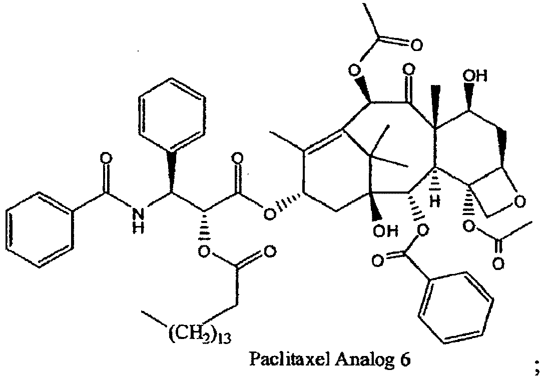

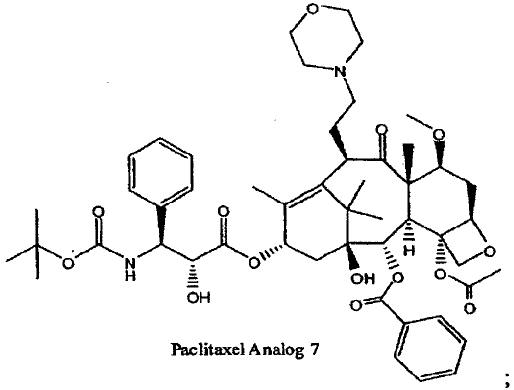

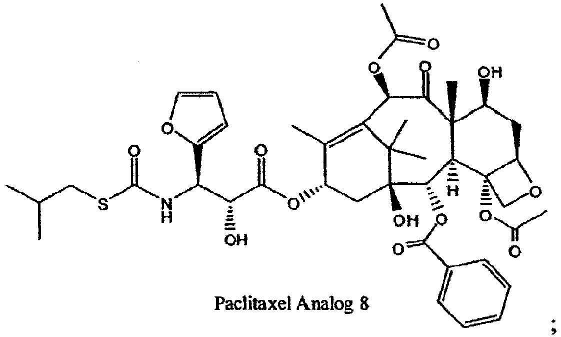

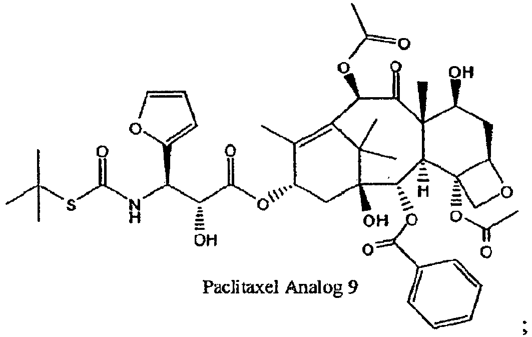

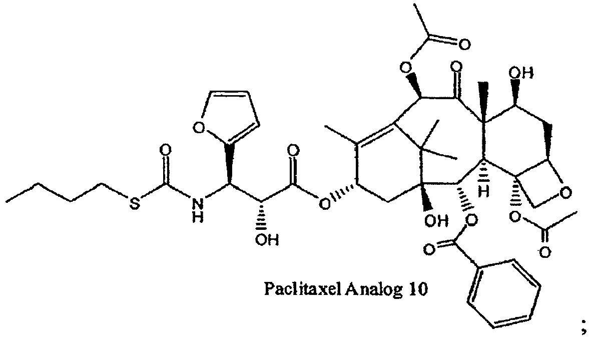

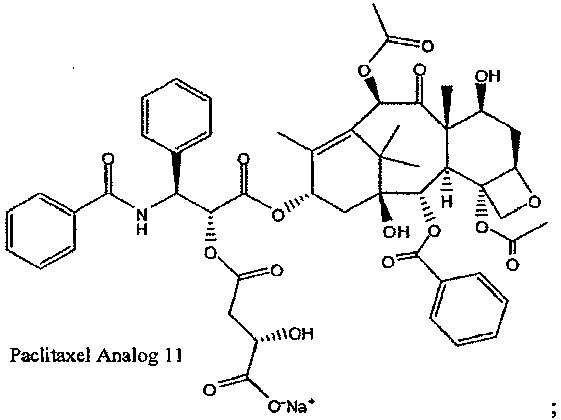

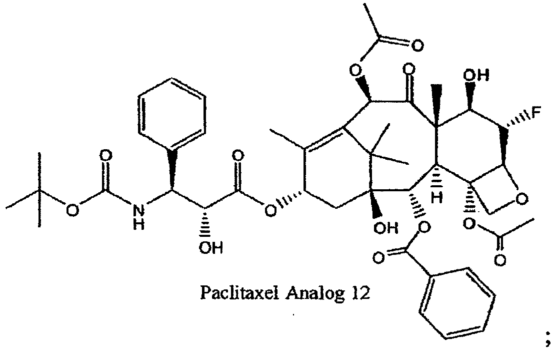

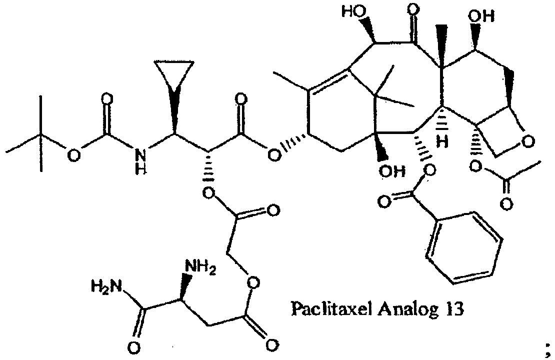

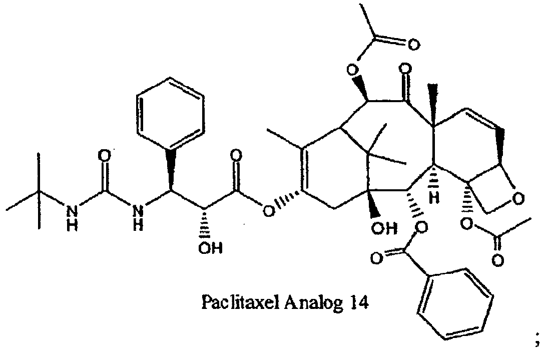

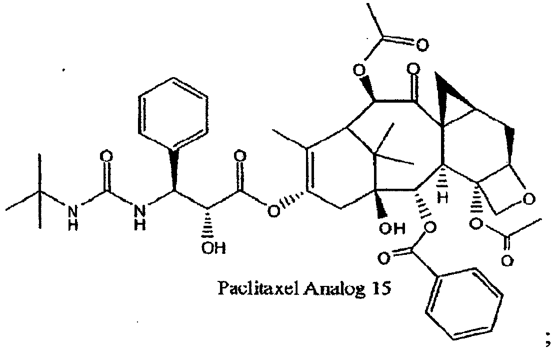

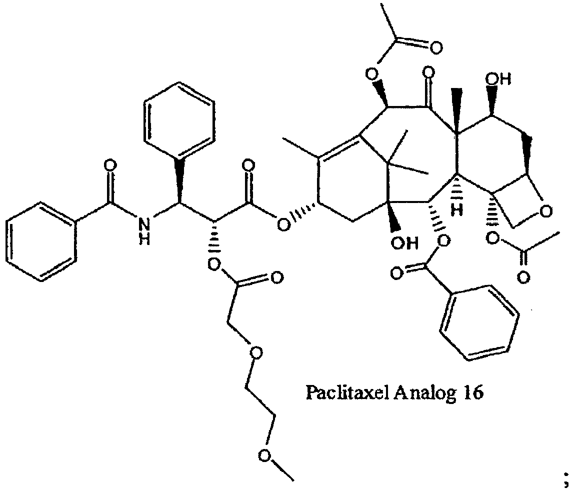

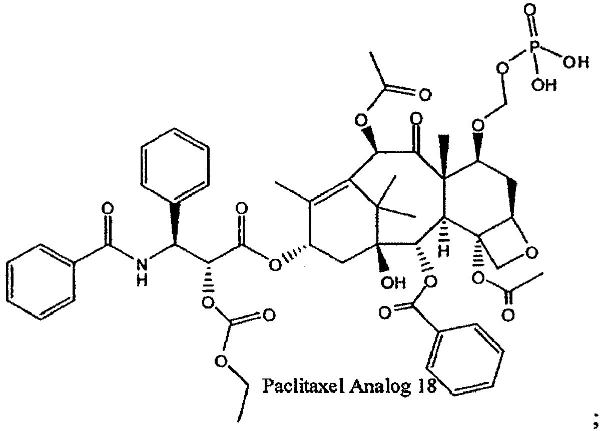

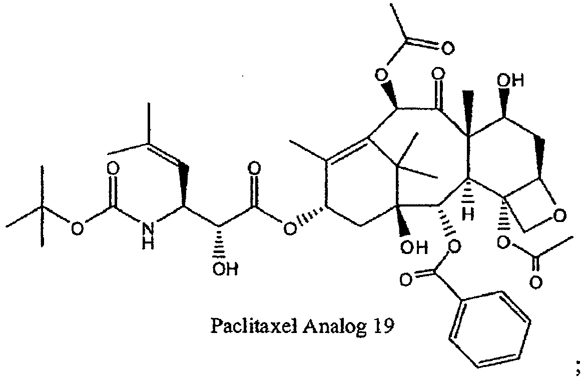

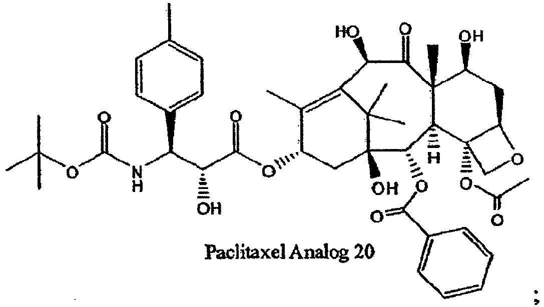

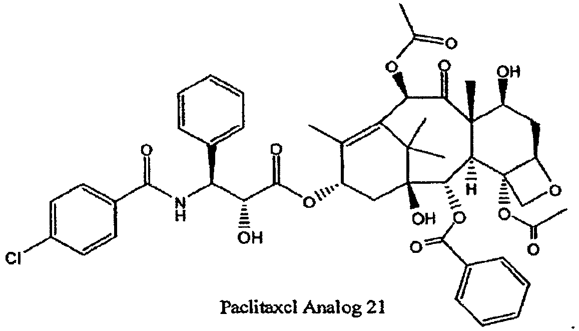

A paclitaxel analog can also be bonded to or be pendent from a pharmaceutically acceptable polymer, such as a polyacrylamide. One example of a polymer of this type is paclitaxel analog 22, below, which has the structure of a polymer comprising a taxol analog group pendent from the polymer backbone. The polymer is a terpolymer of the three monomer units shown. The term "paclitaxel analog", as it is used herein, includes such polymers.

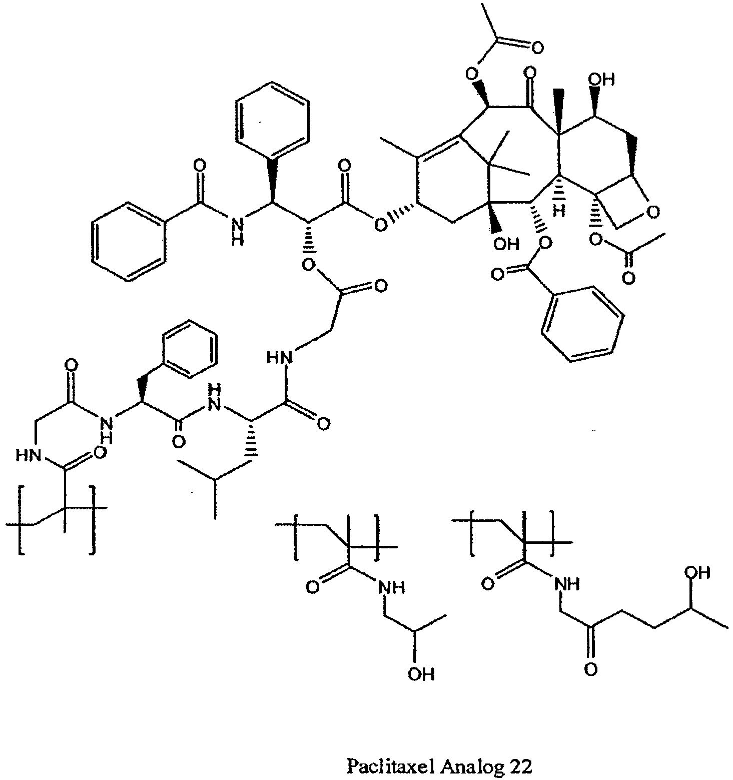

Also disclosed herein, are compounds having any one of Formulae (I)-(III) or (Ia)-(IIIa) and those set forth in Table 1B and 2B and tautomers or pharmaceutically acceptable salts thereof.

Disclosed herein, are compounds represented by Formula (I) or (Ia): 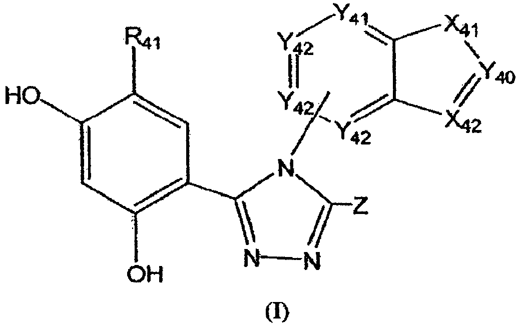 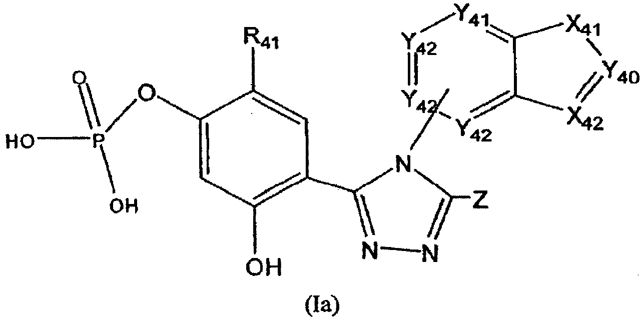 or a tautomer or pharmaceutically acceptable salt thereof, wherein:
X₄₁ is O, S, or NR₄₂;
X₄₂ is CR₄₄ or N;
Y40 is N or CRa3;
Y₄₁ is N or CR₄₅;
Y₄₂, for each occurrence, is independently N, C or CR₄₆;
Z is OH, SH, or NHR₇;
R₄₁ is -H, -OH, -SH, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl, halo, cyano, nitro, guanidino, a haloalkyl, a heteroalkyl, an alkoxy or cycloalkoxy, a haloalkoxy, -NR₁₀R₁₁, -OR₇, -C(O)R₇, -C(O)OR₇, -C(S)R₇, -C(O)SR₇, -C(S)SR₇, -C(S)OR₇, -C(S)NR₁₀R₁₁, -C(NR₈)OR₇, -C(NR₈)R₇₅ -C(NR₈)NR₁₀R₁₁, -C(NR₈)SR₇, -OC(O)R₇, -OC(O)OR₇, -OC(S)OR₇, -OC(NR₈)OR₇, -SC(O)R₇, -SC(O)OR₇, -SC(NR₈)OR₇, -OC(S)R₇, -SC(S)R₇, -SC(S)OR₇, -OC(O)MR₁₀R₁₁, -OC(S)NR₁₀R₁₁, -OC(NR₃)NR₁₀R₁₁, -SC(O)NR₁₀R₁₁, -SC(NR₈)NR₁₀R₁₁, -SC(S)NR₁₀R₁₁, -OC(NR₈)R₇, -SC(NR₈)R₇, -C(O)NR₁₀R₁₁, -NR₈C(O)R₇, -NR₇C(S)R₇, NR₇C(S)OR₇, NR₇C(NR₈)R₇, -NR₇C(O)OR₇, -NR₇C(NR₈)OR₇, -NR₇C(O)NR₁₀R₁₁, -NR₇C(S)NR₁₀R₁₁, -NR₇C(NR₈)NR₁₀R₁₁. -SR₇, -S(O)ₚR₇, -OS(O)ₚR₇, -OS(O)ₚOR₇, -OS(O)ₚNR₁₀R₁₁, -S(O)ₚOR₇, -NR₈S(O)ₚR₇, -NR₇S(O)ₚNR₁₀R₁₁, -NR₇S(O)ₚOR₇, -S(O)ₚNR₁₀R₁₁, -SS(O)ₚR₇, -SS(O)ₚOR₇, -SS(O)ₚNR₁₀R₁₁, -OP(O)(OR₇)₂, or -SP(O)(OR₇)₂;
R₄₂ is -H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl, hydroxyalkyl, alkoxyalkyl, a haloalkyl, a heteroalkyl, -C(O)R₇, -(CH₂)ₘC(O)OR₇, -C(O)OR₇, -OC(O)R₇, -C(O)NR₁₀R₁₁, -S(O)ₚR₇, -S(O)ₚOR₇, or -S(O)ₚNR₁₀R₁₁;
R₄₃ and R₄₄ are, independently, -H, -OH, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl, hydroxyalkyl, alkoxyalkyl, halo, cyano, nitro, guanidino, a haloalkyl, a heteroalkyl, -C(O)R₇, -C(O)OR₇, -OC(O)R₇, -C(O)NR₁₀R₁₁, -NR₈C(O)R₇, -SR₇, -S(O)ₚR₇, -OS(O)ₚR₇, -S(O)ₚOR₇, -NR₈S(O)ₚR₇, -S(O)ₚNR₁₀R₁₁, or R₄₃ and R₄₄ taken together with the carbon atoms to which they are attached form an optionally substituted cycloalkenyl, an optionally substituted aryl, an optionally substituted heterocyclyl, or an optionally substituted heteroaryl;
R₄₅ is -H, -OH, -SH, -NR₇H, -OR₂₆, -SR₂₆, -NHR₂₆, -O(CH₂)mOH, -O(CH₂)ₘSH, -O(CH₂)ₘNR₇H, -S(CH₂)ₘOH, -S(CH₂)ₘSH, -S(CH₂)ₘNR₇H, -OC(O)NR₁₀R₁₁, -SC(O)NR₁₀R₁₁, -NR₇C(O)NR₁₀R₁₁, -OC(O)R₇, -SC(O)R₇, NR₇C(O)R₇, -OC(O)OR₇, -SC(O)OR₇, -NR₇C(O)OR₇, -OCH₂C(O)R₇, -SCH₂C(O)R₇, -NR₇CH₂C(O)R₇, -OCH₂C(O)OR₇, -SCH₂C(O)OR₇, -NR₇CH₂C(O)OR₇, -OCH₂C(O)NR₁₀R₁₁, -SCH₂C(O)NR₁₀R₁₁, -NR₇CH₂C(O)NR₁₀R₁₁, -OS(O)ₚR₇, -SS(O)ₚR₇, -NR₇S(O)ₚR₇, -OS(O)ₚNR₁₀R₁₁, -SS(O)ₚNR₁₀R₁₁, -NR₇S(O)ₚNR₁₀R₁₁, -OS(O)ₚOR₇, -SS(O)ₚOR₇, NR₇S(O)ₚOR₇, -OC(S)R₇, -SC(S)R₇, -NR₇C(S)R₇, -OC(S)OR₇, -SC(S)OR₇, -NR₇C(S)OR₇, -OC(S)NR₁₀R₁₁, -SC(S)NR₁₀R₁₁, -NR₇C(S)NR₁₀R₁₁, -OC(NR₈)R₇, -SC(NR₈)R₇, -NR₇C(NR₈)R₇, -OCNR₈)OR₇, -SC(NR₈)OR₇, -NR₇C(NR₈)OR₇, -OC(NR₃)NR₁₀R₁₁, -SC(NR₈)NR₁₀R₁₁, or -NR₇C(NR₈)NR₁₀R₁₁;
R₄₆, for each occurrence, is independently selected from the group consisting of H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl, halo, cyano, nitro, guanidino, a haloalkyl, a heteroalkyl, NR₁₀R₁₁, -OR₇, -C(O)R₇, -C(O)OR₇, -OC(O)R₇, -C(O)NR₁₀R₁₁, -NR₈C(O)R₇, -SR₇, -S(O)ₚR₇, -OS(O)ₚR₇, -S(O)ₚOR₇, -NR₈S(O)ₚR₇, or -S(O)ₚNR₁₀R₁₁;
R₇ and R₈, for each occurrence, are, independently, -H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl;
R₁₀ and R₁₁, for each occurrence, are independently -H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl; or R₁₀ and R₁₁, taken together with the nitrogen to which they are attached, form an optionally substituted heterocyclyl or an optionally substituted heteroaryl;
R₂₆ is a lower alkyl;
p, for each occurrence, is, independently, 1 or 2; and
m, for each occurrence, is independently, 1, 2, 3, or 4.

In one disclosure, in formula (I) or (Ia), X₄₁ is NR₄₂ and X₄₂ is CR₄₄.

In another disclosure, in formula (I) or (Ia), X₄₁ is NR₄₂ and X₄₂ is N.

In another disclosure, in formula (I) or (Ia), R₄₁ is selected from the group consisting of -H, lower alkyl, lower alkoxy, lower cycloalkyl, and lower cycloalkoxy.

In another disclosure, in formula (I) or (Ia), R₄₁ is selected from the group consisting of -H, methyl, ethyl, propyl, isopropyl, cyclopropyl, methoxy, ethoxy, propoxy, and cyclopropoxy.

In another disclosure, in formula (I) or (Ia), X₄₁ is NR₄₂, and R₄₂ is selected from the group consisting of -H, a lower alkyl, a lower cycloalkyl, -C(O)N(R₂₇)₂, and -C(O)OH, wherein R₂₇ is -H or a lower alkyl.

In another disclosure, in formula (I) or (Ia), X₄₁ is NR₄₂, and R₄₂ is selected from the group consisting of -H, methyl, ethyl, n-propyl, isopropyl, cyclopropyl, n-butyl, sec-butyl, *tert*-butyl, n-pentyl, n-hexyl, -C(O)OH, -(CH2)ₘC(O)OH, -CH₂OCH₃, -CH₂CH₂OCH₃, and -C(O)N(CH₃)₂.

In one disclosure, Y₄₀ is CR₄₃. Y₄₀ may be CR₄₃ and R₄₃ may be H or a lower alkyl.

In another disclosure, in formula (I) or (Ia), R₄₃ and R₄₄ are, independently, selected from the group consisting of -H, methyl, ethyl, propyl, isopropyl or cyclopropyl.

In another disclosure, in formula (I) or (Ia), X₄₂ is CR₄₄; Y is CR₄₃; and R₄₃ and R₄₄ together with the carbon atoms to which they are attached form a cycloalkenyl, an aryl, heterocyclyl, or heteroaryl ring. In one disclosure, R₄₃ and R₄₄ together with the carbon atoms to which they are attached may form a C₅-C₈ cycloalkenyl or a C₅-C₈ aryl.

In another disclosure, in formula (I) or (Ia), R₄₅ is selected from the group consisting of -H, -OH, -SH, -NH₂, a lower alkoxy and a lower alkyl amino.

In another disclosure, in formula (I) or (Ia), R₄₅ is selected from the group consisting of -H, -OH, methoxy and ethoxy.

In another disclosure, in formula (I) or (Ia), X₄₁ is O.

In another disclosure, the compound is selected from the group consisting of:
3-((2,4-dihydroxy-5-ethyl-phenyl)-4-(2-methyl-7-methoxy-benzofuran-4-yl)-5-mercapto-[1,2,4]triazole,
3-((2,4-dihydroxy-5-ethyl-phenyl)-4-(benzofuran-5-yl)-5-mercapto-[1,2,4]triazole,
3-((2,4-dihydroxy-5-ethyl-phenyl)-4-(2-methyl-1,3-benzoxaz-5-yl)-5-mercapto-[1,2,4]triazole, or a tautomers or a pharmaceutically acceptable salt thereof.

In another disclosure, in formula (I) or (Ia), Z is -OH.

In another disclosure, the compound is selected from the group consisting of:
3-((2,4-dihydroxy-5-ethyl-phenyl)-4-(1,3-dimethyl-indol-5-yl)-5-hydroxy-[1,2,4]triazole,
3-((2,4-dihydroxy-5-isopropyl-phenyl)-4-(1,3-dimethyl-indol-5-yl)-5-hydroxy-[1,2,4]triazole,
3 -((2,4-dihydroxy-5-isopropyl-phenyl)-4-(1-isopropyl-indol-4-yl)-5-hydroxy-[1,2,4]triazole, or a tautomer or a pharmaceutically acceptable salt thereof.

In another embodiment, Z is -SH.

In another embodiment, the compound is selected from the group consisting of:
3-((2,4-dihydroxy-5-isopropyl-phenyl)4-(1-methyl-indazol-5-yl)-5-mercapto-[1,2,4]triazole,
3-((2,4-dihydroxy-5-isopropyl-phenyl)-4-(1-methyl-indazol-6-yl)-5-mercapto-[1,2,4]triazole, or a tautomer, or a pharmaceutically acceptable salt thereof.

In one embodiment of the invention, the compound is 3-(2,4-dihydroxy-5-isopropyl-phenyl)-4-(1-methyl-indol-5-yl)-5-hydroxy-[1,2,4]triazole, or a tautomer, or a pharmaceutically acceptable salt thereof.

Also disclosed herein, are compounds represented by formula (II) or (IIa): 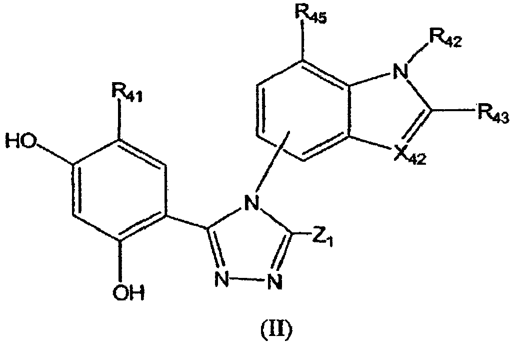 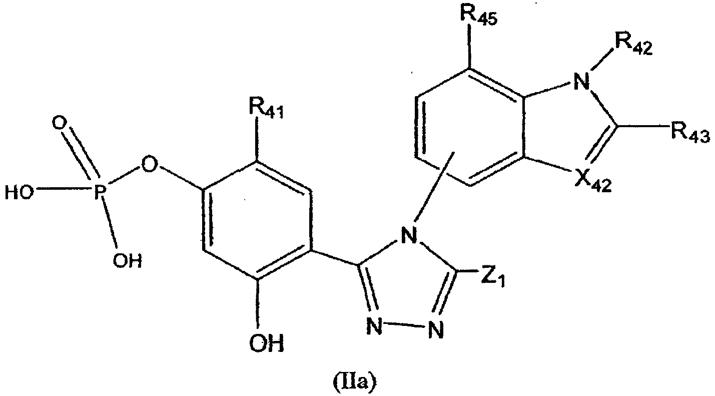 or a tautomer or pharmaceutically acceptable salt thereof, wherein:
Z₁ is -OH or -SH; and
X₄₂, R₄₁, R₄₂, R₄₃, and R₄₅ are defined as above.

In one disclosure, in formula (II) or (IIa), Z₁ is -OH.

In another disclosure, in formula (II) or (IIa), Z₁ is -SH.

In another disclosure, in formula (II) or (IIa), R₄₁ is selected from the group consisting of -H, lower alkyl, lower alkoxy, lower cycloalkyl, and lower cycloalkoxy.

In another disclosure, in formula (II) or (IIa), R₄₁ is selected from the group consisting of -H, methyl, ethyl, propyl, isopropyl, cyclopropyl, methoxy, ethoxy, propoxy, and cyclopropoxy.

In another disclosure, in formula (II) or (IIa), R₄₂ is selected from the group consisting of lower alkyl, lower cycloalkyl, -C(O)N(R₂₇)₂, or -C(O)OH, wherein R₂₇ is-H or a lower alkyl.

In another disclosure, in formula (II) or (IIa), R₄₂ is selected from the group consisting of -H, methyl, ethyl, n-propyl, isopropyl, cyclopropyl, n-butyl, sec-butyl, *tert-*butyl, n-pentyl, n-hexyl, -C(O)OH, -(CH₂)ₘC(O)OH, -CH₂OCH₃, -CH₂CH₂OCH₃, and -C(O)N(CH₃)₂.

In another disclosure, R₄₃ is H or a lower alkyl.

In another disclosure, in formula (II) or (IIa), X₄₂ is CR₄₄, and R₄₃ and R₄₄ are, independently, selected from the group consisting of -H, methyl, ethyl, propyl, isopropyl, and cyclopropyl.

In another disclosure, in formula (II) or (IIa), X₄₂ is CR₄₄, and R₄₃ and R₄₄, taken together with the carbon atoms to which they are attached, form a cycloalkenyl, aryl, heterocyclyl, or heteroaryl ring. R₄₃ and R₄₄, taken together with the carbon atoms to which they are attached, may form a C₅-C₈ cycloalkenyl or a C₅-C₈ aryl.

In another disclosure, in formula (II) or (IIa), R₄₅ is selected from the group consisting of -H, -OH, -SH, -NH₂, a lower alkoxy and a lower alkyl amino.

In another disclosure, in formula (II) or (IIa), R₄₅ is selected from the group consisting of -H, -OH, methoxy, and ethoxy.

In another disclosure, in formula (II) or (IIa), X₄₃ is CR₄₄.

In another disclosure, the compound is selected from the group consisting of:
3-((2,4-dihydroxyphenyl)-4-(1-ethyl-indol-4-yl)-5-mercapto-[1,2,4]triazole,
3-((2,4-dihydroxyphenyl)-4-(1-isopropyl-indol-4-yl)-5-mercapto-[1,2,4]triazole,
3-((2,4-dihydroxyphenyl)-4-(indol-4-yl)-5-mercapto-[1,2,4]triazole,
3-((2,4-dihydroxyphenyl)-4-(1-methoxyethyl-indol-4-yl)-5-mercapto-[1,2,4]triazole,
3-((2,4-dihydroxy-5-ethyl-phenyl)-4-(1-isopropyl-indol-4-yl)-5-mercapto-[1,2,4]triazole,
3-((2,4-dihydroxyphenyl)-4-(1-dimethylcarbamoyl-indol-4-yl)-5-mercapto-[1,2,4]triazole,
3-((2,4-dihydroxy-5-ethyl-phenyl)-4-(1-propyl-indol-4-yl)-5-mercapto-[l,2,4]triazole,
3-((2,4-dihydroxy-5-ethyl-phenyl)-4-(1,2,3-trimethyl-indol-5-yl)-5-mercapto-[1,2,4]triazole,
3-((2,4-dihydroxy-5-ethyl-phenyl)-4-(2,3-dimethyl-indol-5-yl)-5-mercapto-[1,2,4]triazole,
3-((2,4-dihydroxy-5-ethyl-phenyl)-4-(1-acetyl-2,3-dimethyl-indol-5-yl)-5-mercapto-[1,2,4]triazole,
3-((2,4-dihydroxy-5-ethyl-phenyl)4-(1-isopropyl-7-methoxy-indol-4-yl)-5-mercapto-[1,2,4]triazole,
3-((2,4-dihydroxy-5-ethyl-phenyl)-4-(1-propyl-2,3-dimethyl-indol-5-yl)-5-mercapto-[1,2,4]triazole,
3-((2,4-dihydroxy-5-ethyl-phenyl)-4-(N-methyl-tetrahydrocarbozol-7-yl)-5-mercapto-[1,2,4]triazole,
3-((2,4-dihydroxy-5-ethyl-phenyl)-4-(N-methyl-cyclononan[a]indol-5-yl)-5-mercapto-[1,2,4]triazole,
3-((2,4-dihydroxy-5-ethyl-phenyl)-4-(1-n-butyl-indol-4-yl)-5-mercapto-[1,2,4]triazole,
3-((2,4-dihydroxy-5-ethyl-phenyl)-4-(1-n-pentyl-indol-4-yl)-5-mercapto-[1,2,4]triazole,
3-((2,4-dihydroxy-5-ethyl-phenyl)-4-(1-n-hexyl-indol-4-yl)-5-mercapto-[1,2,4]triazole,
3-((2,4-dihydroxy-5-cyclopropyl-phenyl)-4-(1-(1-methylcyclopropyl)-indol-4-yl)-5-mercapto-[1,2,4]triazole,
3-((2,4-dihydroxy-5-cyclopropyl-phenyl)-4-(1-isopropyl-7-methoxy-indol-4-yl)-5-mercapto-[1,2,4]triazole,
3-((2,4-dihydroxy-5-cyclopropyl-phenyl)-4-(1,2,3-trimethyl-indol-5-yl)-5-mercapto-[1,2,4]triazole,
3-((2,4-dihydroxy-5-ethyl-phenyl)-4-(1-isopropyl-7-methoxy-indol-4-yl)-5-mercapto-[1,2,4]triazole disodium salt,
3-((2,4-dihydroxy-5-tert-butyl-phenyl)-4-(1-isopropyl-7-methoxy-indol-4-yl)-5-mercapto-[1,2,4]triazole,
3-((2,4-dihydroxy-5-cyclopropyl-phenyl)-4-(1-propyl-7-methoxy-indol-4-yl)-5-mercapto-[1,2,4]triazole,
3-((2,4-dihydroxy-5-ethyl-phenyl)4-(1-methyl-3-ethyl-indol-5-yl)-5-mercapto-[1,2,4]triazole,
3-((2,4-dihydroxy-5-ethyl-phenyl)4-(1,3-dimethyl-indol-5-yl)-5-mercapto-[1,2,4]triazole,
3-((2,4-dihydroxy-5-isopropyl-phenyl)-4-(1-isopropyl-7-methoxy-indol-4-yl)-5-mercapto-[1,2,4]triazole,
3-((2,4-dihydroxy-5-ethyl-phenyl)-4-(1-methyl-3-isopropyl-indol-5-yl)-5-mercapto-[1,2,4]triazole,
3-((2,4-dihydroxy-5-ethyl-phenyl)-4-(N-ethyl-carbozol-7-yl)-5-mercapto-[1,2,4]triazole,
3-((2,4-dihydroxy-5-ethyl-phenyl)-4-(1-isopropyl-7-hydroxy-indol-4-yl)-5-mercapto-[1,2,4]triazole,
3-((2,4-dihydroxy-5-ethyl-phenyl)-4-(1-isopropyl-7-ethoxy-indol-4-yl)-5-mercapto-[1,2,4]triazole,
3-((2,4-dihydroxy-5-ethyl-phenyl)-4-(1,2-dimethyl-indol-5-yl)-5-mercapto-[1,2,4]triazole,
3-((2,4-dihydroxy-5-ethyl-phenyl)-4-(N-methyl-indol-5-yl)-5-mercapto-[1,2,4]triazole,
3-((2,4-dihydroxy-5-isopropyl-phenyl)-4-(1,3-dimethyl-indol-5-yl)-5-mercapto-[1,2,4]triazole,
3-((2,4-dihydroxy-5-cyclopropyl-phenyl)-4-(1,3-dimethyl-indol-5-yl)-5-mercapto-[1,2,4]triazole,
3-((2,4-dihydroxy-5-cyclopropyl-phenyl)4-(1-methyl-indol-5-yl)-5-mercapto-[1,2,4]triazole,
3-((2,4-dihydroxy-5-isopropyl-phenyl)-4-(1H-indol-5-yl)-5-mercapto-[1,2,4]triazole,
3-((2,4-dihydroxy-5-ethyl-phenyl)-4-(1,2-dimethyl-indol-5-yl)-5-mercapto-[1,2,4]triazole,
3-((2,4-dihydroxy-5-isopropyl-phenyl)-4-(1-ethyl-indol-5-yl)-5-mercapto-[1,2,4]triazole,
3-((2,4-dihydroxy-5-isopropyl-phenyl)-4-(1-propyl-indol-5-yl)-5-mercapto-[1,2,4]triazole, or a tautomer, or a pharmaceutically acceptable salt thereof.

In another disclosure, in formula (II) or (Ha), X₄₂ is N.

In another disclosure, the compound is selected from the group consisting of
3-((2,4-dihydroxy-5-ethyl-phenyl)-4-(1-ethyl-benzimidazol-4-yl)-5-mercapto-[1,2,4]triazole,
3-((2,4-dihydroxy-5-ethyl-phenyl)4-(1-ethyl-benzimidazol-4-yl)-5-mercapto-[1,2,4]triazole HCL salt,
3-((2,4-dihydroxy-5-ethyl-phenyl)-4-(2-methyl-3-ethyl-benzimidazol-5-yl)-5-mercapto-[1,2,4]triazole,
3-((2,4-dihydroxy-5-ethyl-phenyl)-4-(1-ethyl-2-methyl-benzimidazol-5-yl)-5-mercapto-[1,2,4]triazole,
3-((2,4-dihydroxy-5-isopropyl-phenyl)-4-(1-methyl-2-trifluoromethyl-benzimidazol-5-yl)-5-mercapto-[1,2,4]triazole, or a tautomer, or a pharmaceutically acceptable salt thereof.

Also disclosed herein, are compounds having the formula (III) or (IIIa): 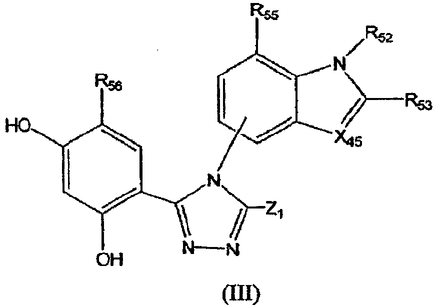 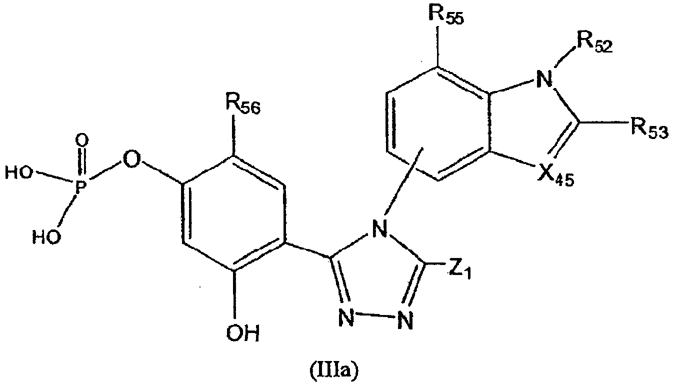 or a tautomer or pharmaceutically acceptable salt thereof, wherein:
X₄₅ is CR₅₄ or N;
Z₁ is -OH or -SH;
R₅₂ is selected from the group consisting of -H, methyl, ethyl, n-propyl, isopropyl, n-butyl, n-pentyl, n-hexyl, -(CH₂)₂OCH₃, -CH₂C(O)OH, and -C(O)N(CH₃)₂;
R₅₃ and R₅₄ are each, independently, -H, methyl, ethyl, or isopropyl; or R₅₃ and R₅₄ taken together with the carbon atoms to which they are attached form a phenyl, cyclohexenyl, or cyclooctenyl ring;
R₅₅ is selected from the group consisting of -H, -OH, -OCH₃, and -OCH₂CH₃; and
R₅₆ is selected from the group consisting of -H, methyl, ethyl, isopropyl, and cyclopropyl.

In one disclosure, in formula (III) or (IIIa), Z₁ is -OH.

In another disclosure, in formula (III) or (IIIa), Z₁ is -SH.

In another disclosure, in formula (III) or (IIIa), R₅₃ is H or a lower alkyl.

In another disclosure, in formula (III) or (IIIa), X₄₅ is CR₅₄. Preferably, R₅₄ is H or a lower alkyl.

In another disclosure, X₄₅ is N.

In another disclosure, the compound is 3-(2,4-dihydroxy-5-isopropyl-phenyl)-4-(N-methyl-indol-5-yl)-5-mereapto-[1,2,4]triazole or a tautomer or pharmaceutically acceptable salt thereof.

### i) Exemplary Compounds of the Invention

Exemplary compounds of the invention are depicted in Table 1A below, including tautomers or pharmaceutically acceptable salts.

**Table 1A**

| | Structure | Tautomeric Structure | Name |
|---|---|---|---|
| 1 | 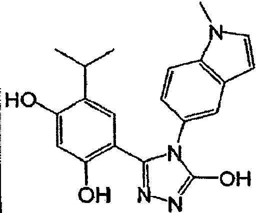 | 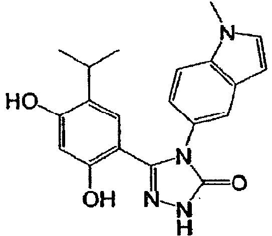 | 3-(2,4-dihydroxy-5-isopropyl-phenyl)-4-(1-methyl-indol-5-yl)-5-hydroxy-[1,2,4] triazole |

Reference compounds are depicted in Table 1B below, including tautomers or pharmaceutically acceptable salts.

**Table 1B**

| | | | |
|---|---|---|---|
| 2 | 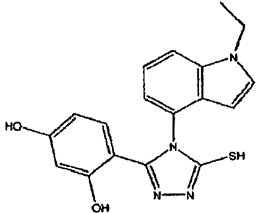 | 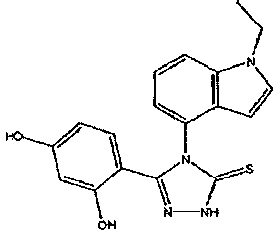 | 3-(2,4-Dihydroxyphenyl)-4-(1-ethyl-indol-4-yl)-5-mercapto-[1,2,4] triazole |
| 3 | 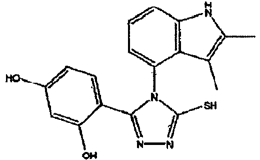 | 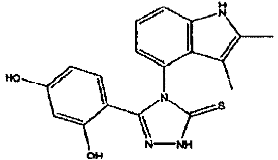 | 3-(2,4-Dihydroxyphenyl)-4-(2,3-dimethyl-1*H*-indol-4-yl)-5-mercapto-[1,2,4] triazole |
| 4 | 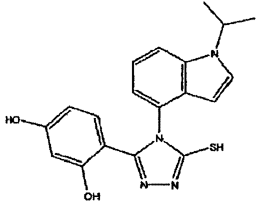 | 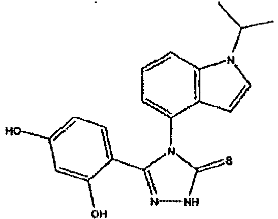 | 3-(2,4-Dihydroxyphenyl)-4-(1-isopropyl-indol-4-yl)-5-mercapto-[1,2,4] triazole |
| 5 | 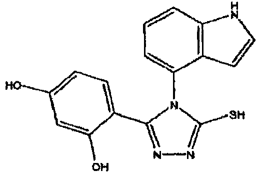 | 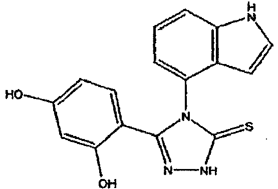 | 3-(2,4-Dihydroxyphenyl)-4-(indol-4-yl)-5-mercapto-[1,2,4] triazole |
| 6 | 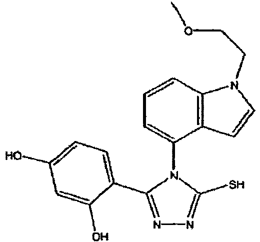 | 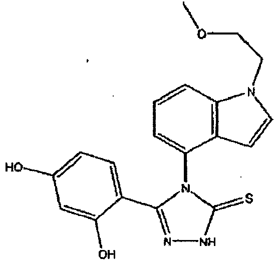 | 3-(2,4-Dihydroxyphenyl)-4-[1-(2-methoxyethoxy)-indol-4-yl]-5-mercapto-[1,2,4] triazole |
| 7 | 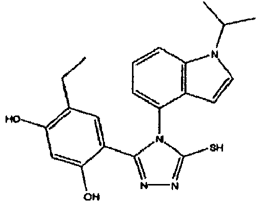 | 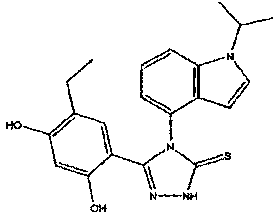 | 3-(2,4-Dihydroxy-5-ethyl-phenyl)-4-(1-isopropyl-indol-4-yl)-5-mercapto-[1,2,4] triazole |
| 8 | 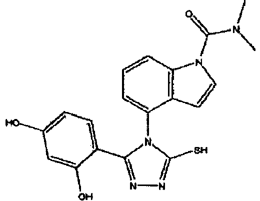 | 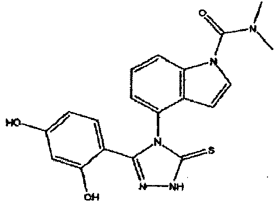 | 3-(2,4-Dihydroxy-5-ethyl-phenyl)-4-[1-(dimethyl-carbamoyl)-indol-4-yl]-5-mercapto-[1,2,4] triazole |
| 9 | 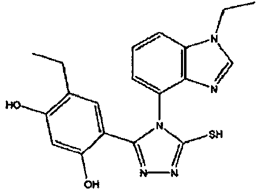 | 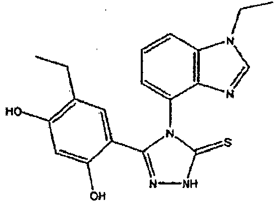 | 3-(2,4-Dihydroxy-5-ethyl-phenyl)-4-(1-ethyl-benzoimidazol-4-yl)-5-mercapto-[1,2,4] triazole |
| 10 | 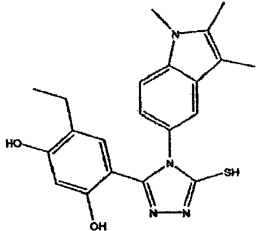 | 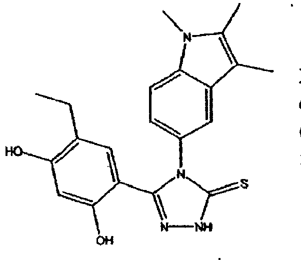 | 3-(2,4-Dihydroxy-5-ethyl-phenyl)-4-1,2,3-trimethyl-indol-5-yl)-5-mercapto-[1,2,4]triazole |
| 11 | 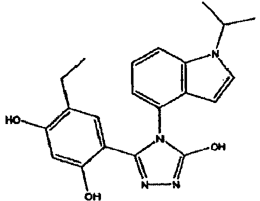 | 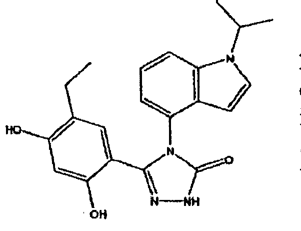 | 3-(2,4-Dihydroxy-5-ethyl-phenyl)-4-(1-isopropyl-indol-3-yl)-5-hydroxy-[1,2,4] triazole |
| 12 | 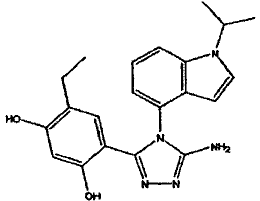 | 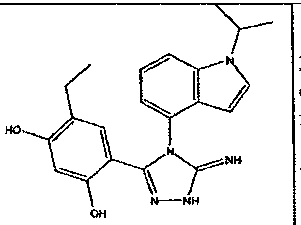 | 3-(2,4-Dihydroxy-5-ethyl-phenyl)-4-(1-isopropyl-indol-4-yl)-5-amino-[1,2,4] triazole |
| 13 | 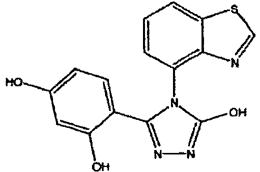 | 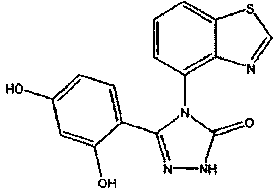 | 3-(2,4-Dihydroxyphenyl)-4-(benzothiazol-4-yl)-5-hydroxy-[1,2,4] triazole |
| 14 | 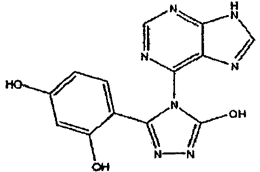 | 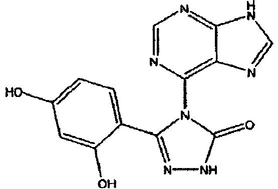 | 3-(2,4-Dihydroxyphenyl)-4-(9H-purin-6-yl)-5-hydroxy-[1,2,4] triazole |
| 15 | 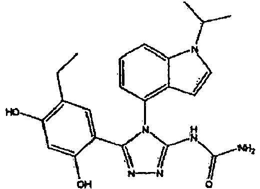 | | 3-(2,4-Dihydroxy-5-ethyl-phenyl)-4-(1-isopropyl-indol-4-yl)-5-ureido-[1,2,4] triazole |
| 16 | 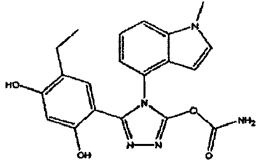 | | 3-(2,4-Dihydroxy-5-ethyl-phenyl)-4-(1-methyl-indol-4-yl)-5-carbamoyloxy-[1,2,4] triazole |
| 17 | 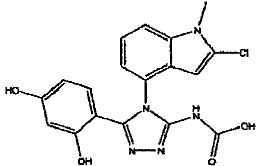 | | 3-(2,4-Dihydroxyphenyl)-4-(1-methyl-2-chloro-indol-4-yl)-5-carbamoyloxy-[1,2,4] triazole |
| 18 | 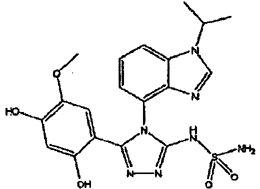 | | 3-(2,4-Dihydroxy-5-methoxy-phenyl)-4-(1-isopropyl-benzoimidazol-4-yl)-5-(sulfamoylamino)-[1,2,4] triazole |
| 19 | 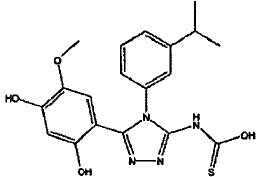 | | 3-(2,4-Dihydroxy-5-methoxy-phenyl)-4-(3-isopropylphenyl)-5-(thiocarboxyamino)-[1,2,4] triazole |
| 20 | 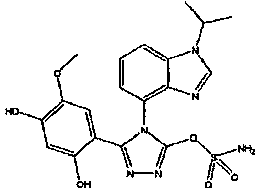 | | 3-(2,4-Dihydroxy-5-methoxy-phenyl)-4-(1-isopropyl-benzoimidazol-4-yl)-5-(sulfamoyloxy)-[1,2,4] triazole |
| 21 | 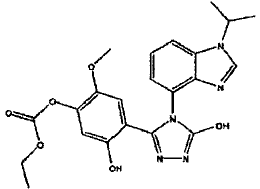 | 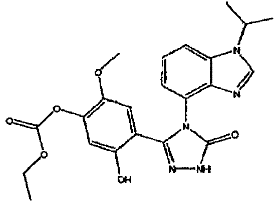 | 3-(2-Hydroxy-4-ethoxycarbonyoxy-5-methoxy-phenyl)-4-(1-isopropyl-benzoimidazol-4-yl)-5-hydroxy-[1,2,4] triazole |
| 22 | 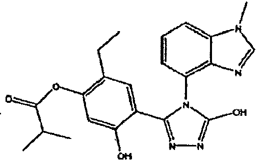 | 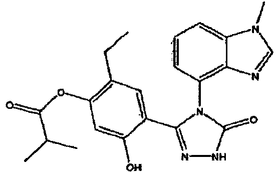 | 3-[2-Hydroxy-4-isobutyryloxy-5-ethylphenyl]-4-(1-methyl-benzo-imidazol-4-yl)-5-hydroxy-[1,2,4] triazole |
| 23 | 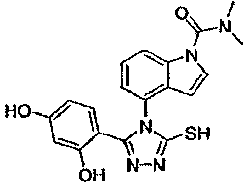 | 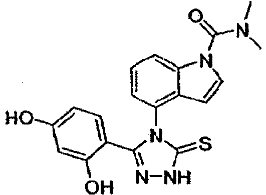 | 3-(2,4-Dihydroxyphenyl)-4-(1-dimethylcarbamoyl-indol-4-yl)-5-mercapto-[1,2,4] triazole |
| 24 | 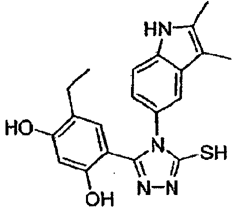 | 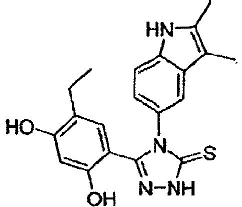 | 3-(2,4-Dihydroxy-5-ethyl-phenyl)-4-(2,3-dimethyl-indol-5-yl)-5-mercapto-[1,2,4] triazole |
| 25 | 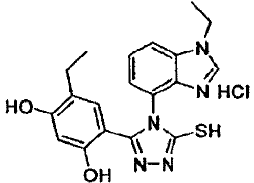 | 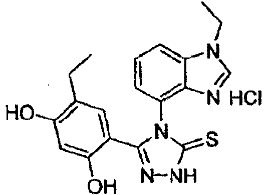 | 3-(2,4-Dihydroxy-5-ethyl-phenyl)-4-(1-ethyl-1H-benzoimidazol-4-yl)-5-mercapto-[1,2,4] triazole, HCl salt |
| 26 | 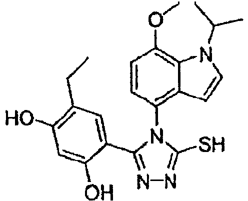 | 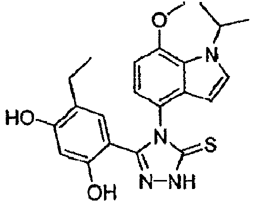 | 3-(2,4-Dihydroxy-5-ethyl-phenyl)-4-(1-isopropyl-7-methoxy-indol-4-yl)-5-mercapto-[1,2,4] triazole |
| 27 | 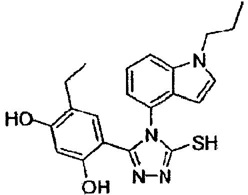 | 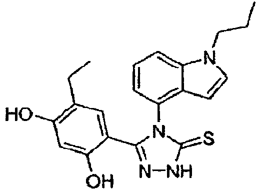 | 3-(2,4-dihydroxy-5-ethyl-phenyl)-4-(1-propyl-indol-4-yl)-5-mercapto-[1,2,4] triazole |
| 28 | 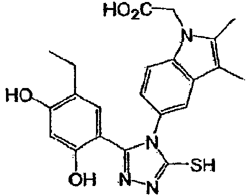 | 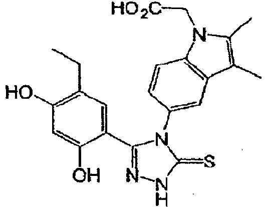 | 3-(2,4-dihydroxy-5-ethyl-phenyl)-4-(1-acetyl-2,3-dimethyl-indol-5-yl)-5-mercapto-[1,2,4] triazole |
| 29 | 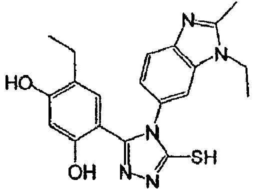 | 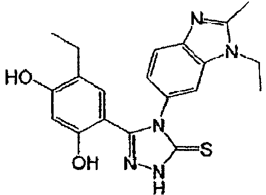 | 3-(2,4-dihydroxy-5-ethyl-phenyl)-4-(2-methyl-3-ethyl-benzimidazol-5-yl)-5-mercapto-[1,2,4] triazole |
| 30 | 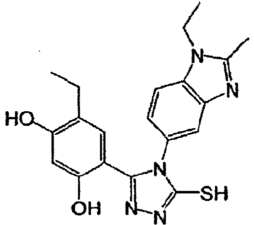 | 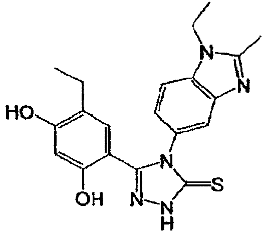 | 3-(2,4-dihydroxy-5-ethyl-phenyl)-4-(1-ethyl-2-methyl-benzimidazol-5-yl)-5-mercapto-[1,2,4] triazole |
| 31 | 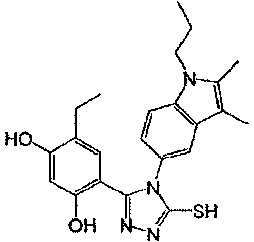 | 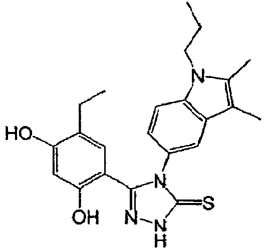 | 3-(2,4-dihydroxy-5-ethyl-phenyl)-4-(1-propyl-2,3-dimethyl-indol-5-yl)-5-mercapto-[l,2,4] triazole |
| 32 | 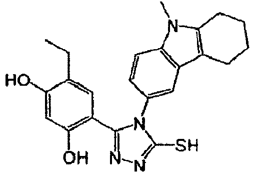 | 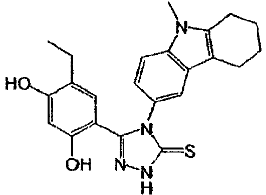 | 3-(2,4-dihydroxy-5-ethyl-phenyl)-4-(N-methyl-tetrahydrocarbozol-7-yl)-5-mercapto-[1,2,4] triazole |
| 33 | 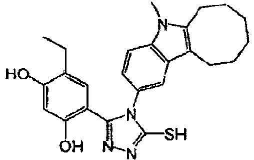 | 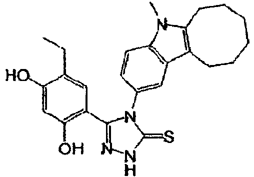 | 3-(2,4-dihydroxy-5-ethyl-phenyl)-4-(N-methyl-cyclononan[a]indol-5-yl)-5-mercapto-[1,2,4] triazole |
| 34 | 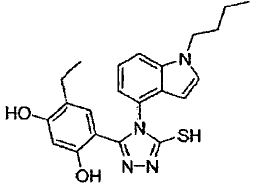 | 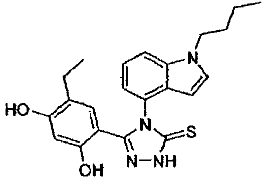 | 3-(2,4-dihydroxy-5-ethyl-phenyl)-4-(1-n-butyl-indol-4-yl)-5-mercapto-[1,2,4] triazole |
| 35 | 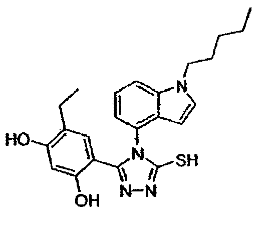 | 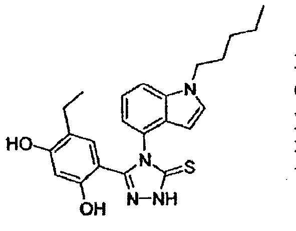 | 3-(2,4-dihydroxy-5-ethyl-phenyl)-4-(1-n-pentyl-indol-4-yl)-5-mercapto-[1,2,4] triazole |
| 36 | 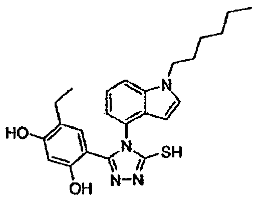 | 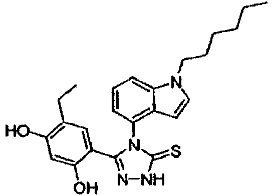 | 3-(2,4-dihydroxy-5-ethyl-phenyl)-4-(1-n-hexyl-indol-4-yl)-5-mercapto-[1,2,4] triazole |
| 37 | 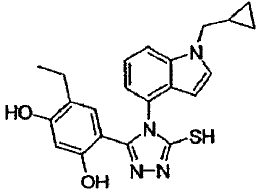 | 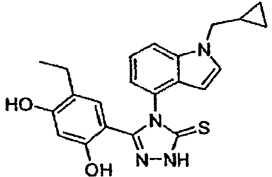 | 3-(2,4-dihydroxy-5-cyclopropyl-phenyl)-4-(1-(1-methylcyclopropyl)-indol-4-yl)-5-mercapto-[1,2,4] triazole |
| 38 | 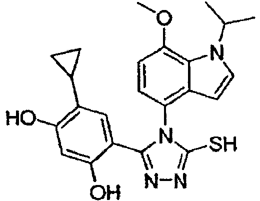 | 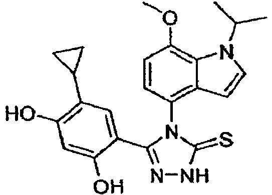 | 3-(2,4-dihydroxy-5-cyclopropyl-phenyl)-4-(1-isopropyl-7-methoxy-indol-4-yl)-5-mercapto-[1,2,4] triazole |
| 39 | 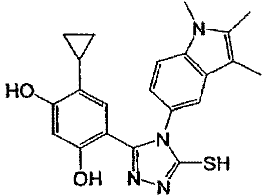 | 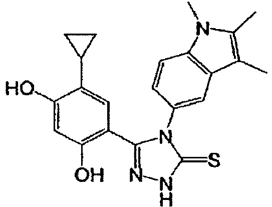 | 3-(2,4-dihydroxy-5-cyclopropyl-phenyl)-4-(1,2,3-trimethyl-indol-5-yl)-5-mercapto-[1,2,4] triazole |
| 40 | 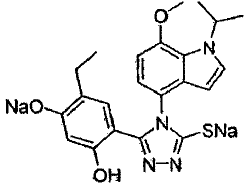 | | 3-(2,4-dihydroxy-5-ethyl-phenyl)-4-(1-isopropyl-7-methoxy-indol-4-yl)-5-mercapto-[1,2,4] triazole disodium salt |
| 41 | 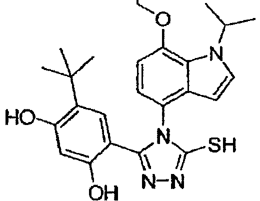 | 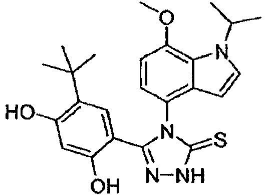 | 3-(2,4-dihydroxy-5-*tert*-butyl-phenyl)-4-(1-isopropyl-7-methoxy-indol-4-yl)-5-mercapto-[1,2,4] triazole |
| 42 | 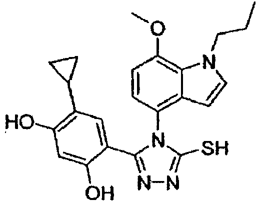 | 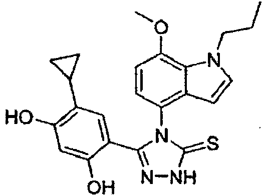 | 3-(2,4-dihydroxy-5-cyclopropyl-phenyl)-4-(1-propyl-7-methoxy-indol-4-yI)-5-mercapto-[1,2,4] triazole |
| 43 | 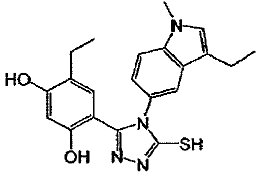 | 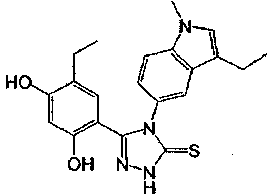 | 3-(2,4-dihydroxy-5-ethyl-phenyl)-4-(1-methyl-3-ethyl-indol-5-yl)-5-mercapto-[1,2,4]triazole |
| 44 | 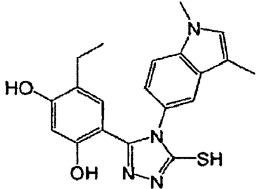 | 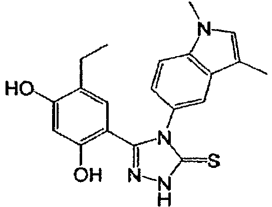 | 3-(2,4-dihydroxy-5-ethyl-phenyl)-4-(1,3-dimethyl-indol-5-yl)-5-mercapto-[1,2,4] triazole |
| 45 | 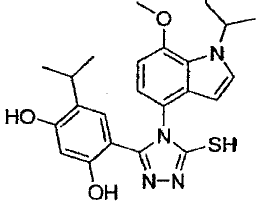 | 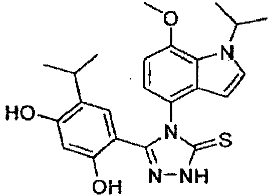 | 3-(2,4-dihydroxy-5-isopropyl-phenyl)-4-(1-isopropyl-7-methoxy-indol-4-yl)-5-mercapto-[1,2,4] triazole |
| 46 | 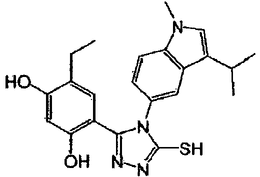 | 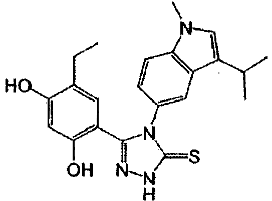 | 3-(2,4-dihydroxy-5-ethyl-phenyl)-A-(1-methyl-3-isopropyl-indol-5-yl)-5-mercapto-[1,2,4] triazole, |
| 47 | 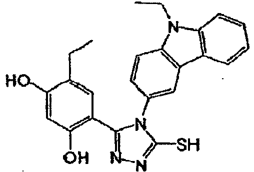 | 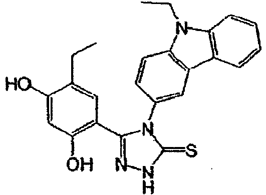 | 3-(2,4-dihydroxy-5-ethyl-phenyl)-4-(N-ethyl-carbozol-7-yl)-5-mercapto-[1,2,4] triazole |
| 48 | 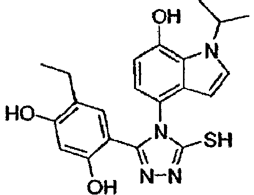 | 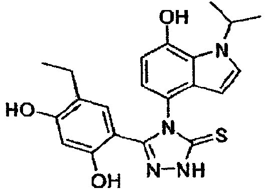 | 3-(2,4-dihydroxy-5-ethyl-phenyl)-4-(1-isopropyl-7-hydroxy-indol-4-yl)-5-mercapto-[1,2,4] triazole |
| 49 | 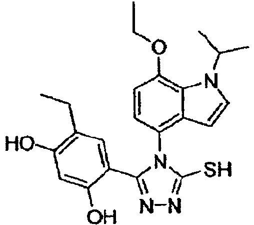 | 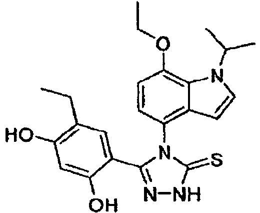 | 3-(2,4-dihydroxy-5-ethyl-phenyl)-4-(1-isopropyl-7-ethoxy-indol-4-yl)-5-mercapto-[1,2,4] triazole |
| 50 | 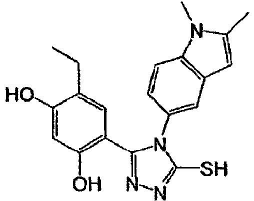 | 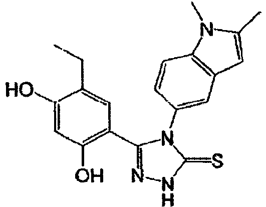 | 3-(2,4-dihydroxy-5-ethyl-phenyl)-4-(1,2-dimethyl-indol-5-yl)-5-mercapto-[1,2,4] triazole |
| 51 | 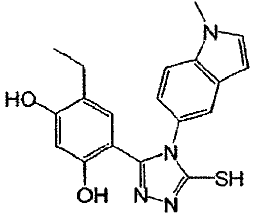 | 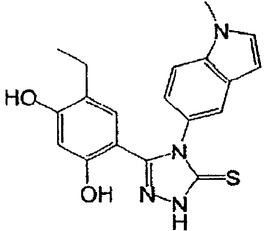 | 3-(2,4-dihydroxy-5-ethyl-phenyl)-4-(N-methyl-indol-5-yl)-5-mercapto-[1,2,4] triazole |
| 52 | 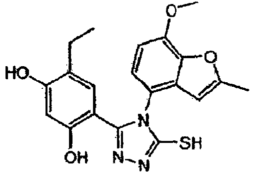 | 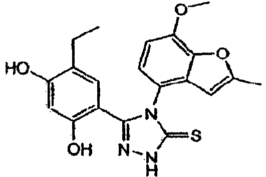 | 3-(2,4-dihydroxy-5-ethyl-phenyl)-4-(2-methyl-7-methoxy-benzofuran-4-yl)-5-mercapto-[1,2,4] triazole |
| 53 | 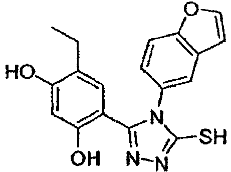 | 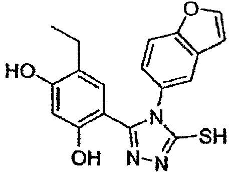 | 3-(2,4-dihydroxy-5-ethyl-phenyl)-4-(benzofuran-5-yl)-5-mercapto-[1,2,4] triazole |
| 54 | 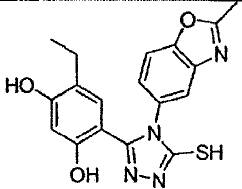 | 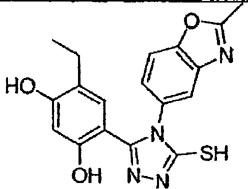 | 3-(2,4-dihydroxy-5-ethyl-phenyl)-4-(2-methyl-1,3-benzoxaz-5-yl)-5-mercapto-[1,2,4]triazole |
| 55 | 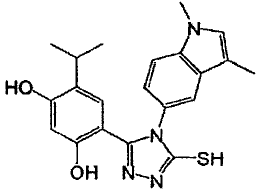 | 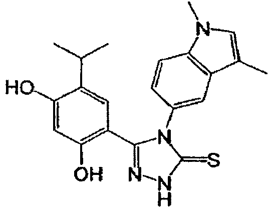 | 3-(2,4-dihydroxy-5-isopropyl-phenyl)-4-(1,3-dimethyl-indol-5-yl)-5-mercapto-[1,2,4] triazole |
| 56 | 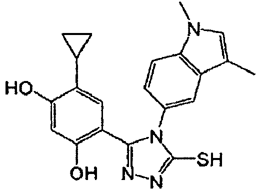 | 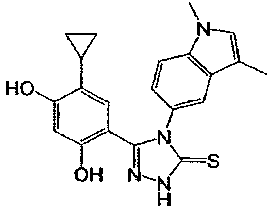 | 3-(2,4-dihydroxy-5-cyclopropyl-phenyl)-4-(1,3-dimethyl-indol-5-yl)-5-mercapto-[1,2,4] triazole |
| 57 | 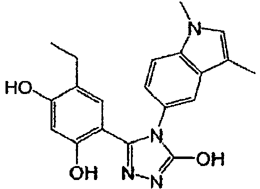 | 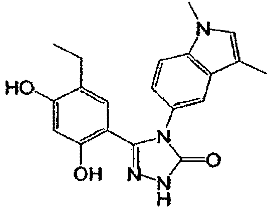 | 3-(2,4-dihydroxy-5-ethyl-phenyl)-4-(1,3-dimethyl-indol-5-yl)-5-hydroxy-[1,2,4] triazole |
| 58 | 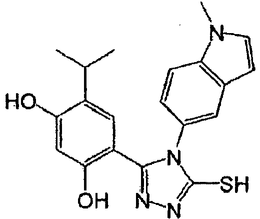 | 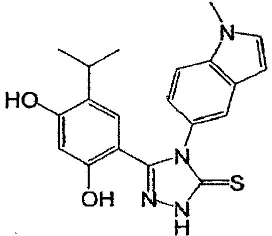 | 3-(2,4-dihydroxy-5-isopropyl-phenyl)-4-(N-methyl-indol-5-yl)-5-mercapto-[1,2,4] triazole |
| 59 | 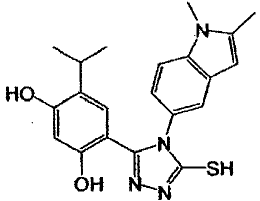 | 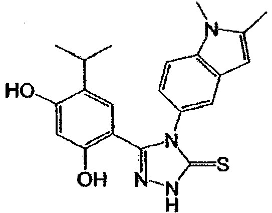 | 3-(2,4-dihydroxy-5-isopropyl-phenyl)-4-(1,2-dimethyl-indol-5-yl)-5-mercapto-[1,2,4] triazole |
| 60 | 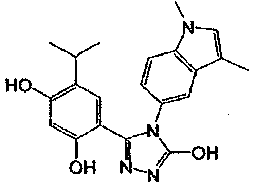 | 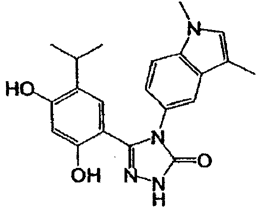 | 3-(2,4-dihydroxy-5-isopropyl-phenyl)-4-(1,3-dimethyl-indol-5-yl)-5-hydroxy-[1,2,4] triazole |
| 61 | 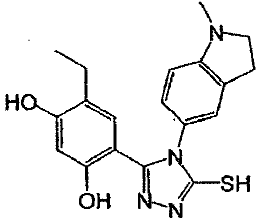 | 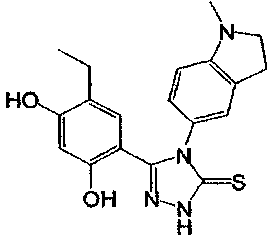 | 3-(2,4-dihydroxy-5-cyclopropyl-phenyl)-4-(1-methyl-indol-5-yl)-5-mercapto-[1,2,4] triazole |
| 62 | 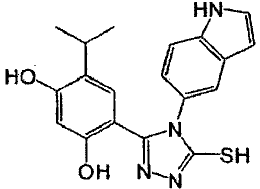 | 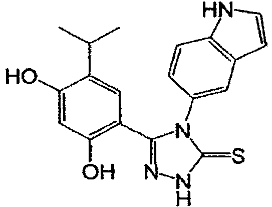 | 3-(2,4-dihydroxy-5-isopropyl-phenyl)-4-(1H-indol-5-yl)-5-mercapto-[1,2,4] triazole |
| 63 | 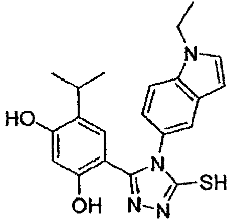 | 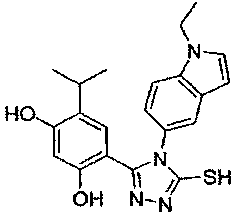 | 3-(2,4-dihydroxy-5-isopropyl-phenyl)-4-(1-ethyl-indol-5-yl)-5-mercapto-[1,2,4] triazole |
| 64 | 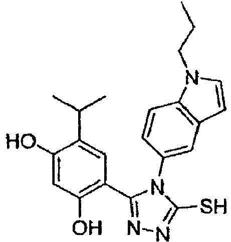 | 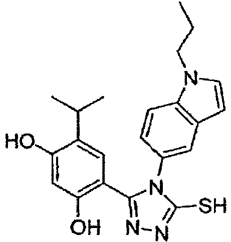 | 3-(2,4-dihydroxy-5-isopropyl-phenyl)-4-(1-propyl-indol-5-yl)-5-mercapto-[1,2,4] triazole |
| 65 | 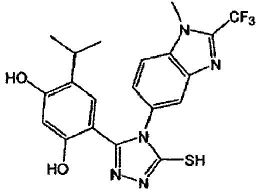 | 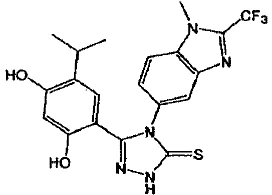 | 3-(2,4-dihydroxy-5-isopropyl-phenyl)-4-(1-methyl-2-trifluoromethyl-benzimidazol-5-yl)-5-mercapto-[1,2,4] triazole |
| 66 | 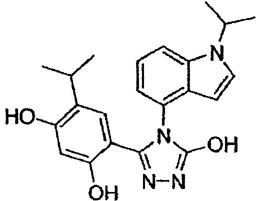 | 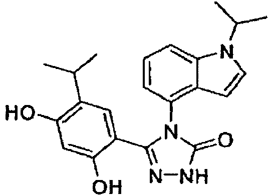 | 3-(2,4-dihydroxy-5-isopropyl-phenyl)-4-(1-isopropyl-indol-4-yl)-5-hydroxy-[1,2,4] triazole |
| 67 | 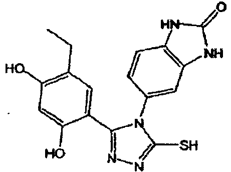 | 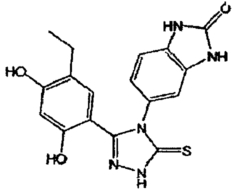 | 3-(2,4-dihydroxy-5-ethyl-phenyl)-4-(2-oxo-1,3-dihydro-benzoimidazol-5-yl)-5-mercapto-[1,2,4] triazole |

**Table 2A: Compounds according to Formula (Ia)**

| No. | Structure | Tautomeric structure | Name |
|---|---|---|---|
| 1a | 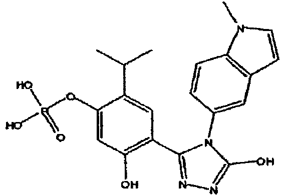 | 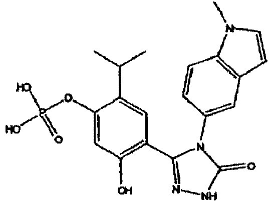 | 5-hydroxy-4-(5-hydroxy-4-(1-methyl-1H-indol-5-yl)-4H-1,2,4-triazol-3-yl)-2-isopropylphenyl dihydrogen phosphate |

**Table 2B: Reference compounds according to Formaula (Ia)**

| | | | |
|---|---|---|---|
| 2a | 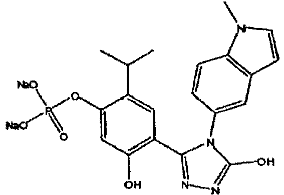 | 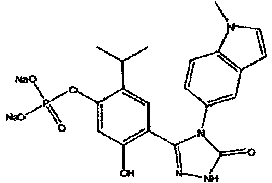 | sodium 5-hydroxy-4-(5-hydroxy-4-(1-methyl-1H-indol-5-yl)-4H-1,2,4-triazol-3-yl)-2-isopropylphenyl phosphate |
| 3a | 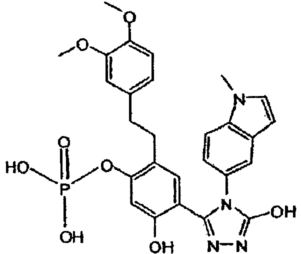 | 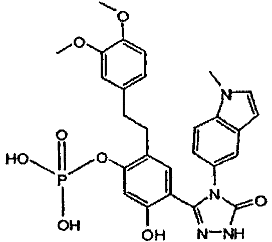 | 2-(3,4-dimethoxyphenet hyl)-5-hydroxy-4-(5-hydroxy-4-(1-methyl-1H-indol-5-yl)-4H-1,2,4-triazol-3-yl)phenyl dihydrogen phosphate |
| 4a | 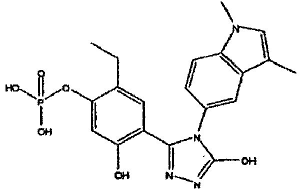 | 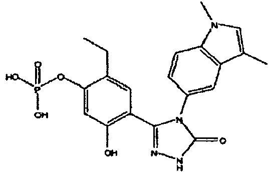 | 4-(4-(1,3-dimethyl-1H-indol-5-yl)-5-hydroxy-4H-1,2,4-triazol-3-yl)-2-ethyl-5-hydroxyphenyl dihydrogen phosphate |

Compounds used in the disclosed methods can be prepared according to methods disclosed in U.S. Application No. 2006-0167070 and WO2009/023211.

Compounds of the invention, and those disclosed herein, typically can form a tautomeric structure as shown below and as exemplified by the tautomeric structures shown in Tables 1A, 1B, 2A and 2B:

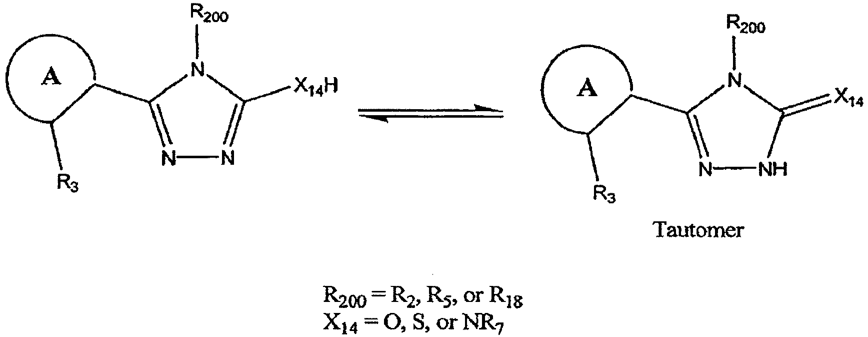

The invention also provides compounds for use in methods of treating, managing, or ameliorating lung cancer (*e.g.,* NSCLC), colon carcinoma or erythroleukemia, or one or more symptoms thereof, said methods comprising administering to a subject in need thereof one or more compounds of the invention and one or more other therapies (*e.g.,* one or more therapeutic agents that are currently being used, have been used, are known to be useful or in development for use in the treatment or amelioration of lung cancer (*e.g.,* NSCLC), colon carcinoma or erythroleukemia or one or more symptoms associated with lung cancer (*e.g.,* NSCLC), colon carcinoma or erythroleukemia.

The therapeutic agents of the combination therapies of the invention can be administered sequentially or concurrently. In a specific embodiment, the combination therapies of the invention comprise one or more compounds and at least one other therapy which has the same mechanism of action as said compounds. In another specific embodiment, the combination therapies of the invention comprise one or more compounds of the invention and at least one other therapy which has a different mechanism of action than said compounds. In certain embodiments, the combination therapies of the present invention improve the therapeutic effect of one or more compounds of the invention by functioning together with the compounds to have an additive or synergistic effect. In certain embodiments, the combination therapies of the present invention reduce the side effects associated with the therapies. In certain embodiments, the combination therapies of the present invention reduce the effective dosage of one or more of the therapies.

The therapeutic agents of the combination therapies can be administered to a subject, preferably a human subject, in the same pharmaceutical composition. In alternative embodiments, the therapeutic agents of the combination therapies can be administered concurrently to a subject in separate pharmaceutical compositions. The therapeutic agents may be administered to a subject by the same or different routes of administration. In a specific embodiment, a pharmaceutical composition comprising one or more compounds of the invention is administered to a subject, preferably a human, to prevent, treat, manage, or ameliorate a proliferative disorder, such as cancer, or one or more symptom thereof. In accordance with the invention, pharmaceutical compositions of the invention may also comprise one or more other agents being used, have been used, or are known to be useful in the treatment or amelioration of lung cancer (*e.g.,* NSCLC), colon carcinoma or erythroleukemia or a symptom thereof).

The invention provides compounds for use in methods of managing, treating or ameliorating lung cancer (*e.g.,* NSCLC), colon carcinoma or erythroleukemia or one or more symptoms thereof in a subject refractory (either completely or partially) to existing agent therapies for lung cancer (*e.g.* NSCLC), colon carcinoma or erythroleukemia, said methods comprising administering to said subject a dose of an effective amount of one or more compounds of the invention and a dose of an effective amount of one or more therapies. The invention also provides compounds for use in methods of treating, managing, or ameliorating lung cancer (*e.g.,* NSCLC), colon carcinoma or erythroleukemia or a symptom thereof by administering one or more compounds of the invention in combination with any other therapy(ies) to patients who have proven refractory to other therapies but are no longer on these therapies.

The compounds of the invention and/or other therapies can be administered to a subject by any route known to one of skill in the art. Examples of routes of administration include, but are not limited to, parenteral, *e.g.*, intravenous, intradermal, subcutaneous, oral (*e.g.,* inhalation), intranasal, transdermal (topical), transmucosal, and rectal administration.

The present invention provides compositions for the treatment, and amelioration of lung cancer (*e.g.,* NSCLC), colon carcinoma or erythroleukemia. In a specific embodiment, a composition comprises one or more compounds of the invention, or a pharmaceutically acceptable salt, thereof. In another embodiment, a composition of the invention comprises one or more therapeutic agents other than a compound of the invention, or a pharmaceutically acceptable salt. In another embodiment, a composition of the invention comprises one or more compounds of the invention, or a pharmaceutically acceptable salt thereof, and one or more other therapeutic agents. In another embodiment, the composition comprises a compound of the invention, or a pharmaceutically acceptable salt, thereof, and a pharmaceutically acceptable carrier, diluent or excipient.

In a preferred embodiment, a composition of the invention is a pharmaceutical composition or a single unit dosage form. Pharmaceutical compositions and dosage forms of the invention comprise one or more active ingredients in relative amounts and formulated in such a way that a given pharmaceutical composition or dosage form can be used to treat lung cancer (*e.g.,* NSCLC), colon carcinoma or erythroleukemia. Preferred pharmaceutical compositions and dosage forms comprise a compound in Table 1A or 2A, or a pharmaceutically acceptable thereof, optionally in combination with one or more additional active agents.

A pharmaceutical composition of the invention is formulated to be compatible with its intended route of administration. Examples of routes of administration include, but are not limited to, parenteral, *e.g.,* intravenous, intradermal, subcutaneous, oral (*e.g.,* inhalation), intranasal, transdermal (topical), transmucosal, and rectal administration. In a specific embodiment, the composition is formulated in accordance with routine procedures as a pharmaceutical composition adapted for intravenous, subcutaneous, intramuscular, oral, intranasal or topical administration to human beings. In a preferred embodiment, a pharmaceutical composition is formulated in accordance with routine procedures for subcutaneous administration to human beings.

Single unit dosage forms of the invention are suitable for oral, mucosal (*e.g.,* nasal, sublingual, vaginal, buccal, or rectal), parenteral (*e.g.,* subcutaneous, intravenous, bolus injection, intramuscular, or intraarterial), or transdermal administration to a patient. Examples of dosage forms include, but are not limited to: tablets; caplets; capsules, such as soft elastic gelatin capsules; cachets; troches; lozenges; dispersions; suppositories; ointments; cataplasms (poultices); pastes; powders; dressings; creams; plasters; solutions; patches; aerosols (*e.g.,* nasal sprays or inhalers); gels; liquid dosage forms suitable for oral or mucosal administration to a patient, including suspensions (*e.g.,* aqueous or non-aqueous liquid suspensions, oil-in-water emulsions, or a water-in-oil liquid emulsions), solutions, and elixirs; liquid dosage forms suitable for parenteral administration to a patient; and sterile solids (*e.g.,* crystalline or amorphous solids) that can be reconstituted to provide liquid dosage forms suitable for parenteral administration to a patient.

The composition, shape, and type of dosage forms of the invention will typically vary depending on their use. For example, a dosage form suitable for mucosal administration may contain a smaller amount of active ingredient(s) than an oral dosage form used to treat the same indication. This aspect of the invention will be readily apparent to those skilled in the art. *See, e.g.,* Remington's Pharmaceutical Sciences (1990) 18th ed., Mack Publishing, Easton PA.

Typical pharmaceutical compositions and dosage forms comprise one or more excipients. Suitable excipients are well known to those skilled in the art of pharmacy, and non-limiting examples of suitable excipients are provided herein. Whether a particular excipient is suitable for incorporation into a pharmaceutical composition or dosage form depends on a variety of factors well known in the art including, but not limited to, the way in which the dosage form will be administered to a patient. For example, oral dosage forms such as tablets may contain excipients not suited for use in parenteral dosage forms.

The suitability of a particular excipient may also depend on the specific active ingredients in the dosage form. For example, the decomposition of some active ingredients can be accelerated by some excipients such as lactose, or when exposed to water. Active ingredients that comprise primary or secondary amines (*e.g.,* N-desmethylvenlafaxine and N,N-didesmethylvenlafaxine) are particularly susceptible to such accelerated decomposition. Consequently, this invention encompasses pharmaceutical compositions and dosage forms that contain little, if any, lactose. As used herein, the term "lactose-free" means that the amount of lactose present, if any, is insufficient to substantially increase the degradation rate of an active ingredient. Lactose-free compositions of the invention can comprise excipients that are well known in the art and are listed, for example, in the U.S. Pharmocopia (USP) SP (XXI)/NF (XVI). In general, lactose-free compositions comprise active ingredients, a binder/filler, and a lubricant in pharmaceutically compatible and pharmaceutically acceptable amounts. Preferred lactose-free dosage forms comprise active ingredients, microcrystalline cellulose, pre-gelatinized starch, and magnesium stearate.

This invention further encompasses anhydrous pharmaceutical compositions and dosage forms comprising active ingredients, since water can facilitate the degradation of some compounds. For example, the addition of water *(e.g.,* 5%) is widely accepted in the pharmaceutical arts as a means of simulating long-term storage in order to determine characteristics such as shelf-life or the stability of formulations over time. *See, e.g.,* Jens T. Carstensen (1995) Drug Stability: Principles & Practice, 2d. Ed., Marcel Dekker, NY, NY, 379-80. In effect, water and heat accelerate the decomposition of some compounds. Thus, the effect of water on a formulation can be of great significance since moisture and/or humidity are commonly encountered during manufacture, handling, packaging, storage, shipment, and use of formulations.

Anhydrous pharmaceutical compositions and dosage forms of the invention can be prepared using anhydrous or low moisture containing ingredients and low moisture or low humidity conditions. Pharmaceutical compositions and dosage forms that comprise lactose and at least one active ingredient that comprises a primary or secondary amine are preferably anhydrous if substantial contact with moisture and/or humidity during manufacturing, packaging, and/or storage is expected.

An anhydrous pharmaceutical composition should be prepared and stored such that its anhydrous nature is maintained. Accordingly, anhydrous compositions are preferably packaged using materials known to prevent exposure to water such that they can be included in suitable formulary kits. Examples of suitable packaging include, but are not limited to, hermetically sealed foils, plastics, unit dose containers (*e*.*g*., vials), blister packs, and strip packs.

The invention further encompasses pharmaceutical compositions and dosage forms that comprise one or more compounds that reduce the rate by which an active ingredient will decompose. Such compounds, which are referred to herein as "stabilizer" include, but are not limited to, antioxidants such as ascorbic acid, pH buffers, or salt buffers.

Pharmaceutical compositions of the invention that are suitable for oral administration can be presented as discrete dosage forms, such as, but are not limited to, tablets (*e.g.,* chewable tablets), caplets, capsules, and liquids (*e.g*, flavored syrups). Such dosage forms contain predetermined amounts of active ingredients, and may be prepared by methods of pharmacy well known to those skilled in the art. *See generally,* Remington's Pharmaceutical Sciences (1990) 18th ed., Mack Publishing, Easton PA.

Typical oral dosage forms of the invention are prepared by combining the active ingredient(s) in an admixture with at least one excipient according to conventional pharmaceutical compounding techniques. Excipients can take a wide variety of forms depending on the form of preparation desired for administration. For example, excipients suitable for use in oral liquid or aerosol dosage forms include, but are not limited to, water, glycols, oils, alcohols, flavoring agents, preservatives, and coloring agents. Examples of excipients suitable for use in solid oral dosage forms (*e.g.,* powders, tablets, capsules, and caplets) include, but are not limited to, starches, sugars, microcrystalline cellulose, diluents, granulating agents, lubricants, binders, and disintegrating agents.

Because of their ease of administration, tablets and capsules represent the most advantageous oral dosage unit forms, in which case solid excipients are employed. If desired, tablets can be coated by standard aqueous or nonaqueous techniques. Such dosage forms can be prepared by any of the methods of pharmacy. In general, pharmaceutical compositions and dosage forms are prepared by uniformly and intimately admixing the active ingredients with liquid carriers, finely divided solid carriers, or both, and then shaping the product into the desired presentation if necessary.

For example, a tablet can be prepared by compression or molding. Compressed tablets can be prepared by compressing in a suitable machine the active ingredients in a free-flowing form such as powder or granules, optionally mixed with an excipient. Molded tablets can be made by molding in a suitable machine a mixture of the powdered compound moistened with an inert liquid diluent.

Examples of excipients that can be used in oral dosage forms of the invention include, but are not limited to, binders, fillers, disintegrants, and lubricants. Binders suitable for use in pharmaceutical compositions and dosage forms include, but are not limited to, corn starch, potato starch, or other starches, gelatin, natural and synthetic gums such as acacia, sodium alginate, alginic acid, other alginates, powdered tragacanth, guar gum, cellulose and its derivatives *(e.g.,* ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, *(e.g.,* Nos. 2208, 2906, 2910), microcrystalline cellulose, and mixtures thereof.

Suitable forms of microcrystalline cellulose include, but are not limited to, the materials sold as AVICEL-PH-101, AVICEL-PH-103 AVICEL RC-581, AVICEL-PH-105 (available from FMC Corporation, American Viscose Division, Avicel Sales, Marcus Hook, PA), and mixtures thereof. One specific binder is a mixture of microcrystalline cellulose and sodium carboxymethyl cellulose sold as AVICEL RC-581. Suitable anhydrous or low moisture excipients or additives include AVICEL-PH-103J and Starch 1500 LM.

Examples of fillers suitable for use in the pharmaceutical compositions and dosage forms disclosed herein include, but are not limited to, talc, calcium carbonate *(e.g.,* granules or powder), microcrystalline cellulose, powdered cellulose, dextrates, kaolin, mannitol, silicic acid, sorbitol, starch, pre-gelatinized starch, and mixtures thereof. The binder or filler in pharmaceutical compositions of the invention is typically present in from about 50 to about 99 weight percent of the pharmaceutical composition or dosage form.

Disintegrants are used in the compositions of the invention to provide tablets that disintegrate when exposed to an aqueous environment. Tablets that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients should be used to form solid oral dosage forms of the invention. The amount of disintegrant used varies based upon the type of formulation, and is readily discernible to those of ordinary skill in the art. Typical pharmaceutical compositions comprise from about 0.5 to about 15 weight percent of disintegrant, preferably from about 1 to about 5 weight percent of disintegrant.

Disintegrants that can be used in pharmaceutical compositions and dosage forms of the invention include, but are not limited to, agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, other starches, clays, other algins, other celluloses, gums, and mixtures thereof.

Lubricants that can be used in pharmaceutical compositions and dosage forms of the invention include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil *(e.g.,* peanut oil, cottonseed oil, sunflower oil, sesame oil, olive oil, corn oil, and soybean oil), zinc stearate, ethyl oleate, ethyl laureate, agar, and mixtures thereof. Additional lubricants include, for example, a syloid silica gel (AEROSIL 200, manufactured by W.R. Grace Co. of Baltimore, MD), a coagulated aerosol of synthetic silica (marketed by Degussa Co. of Plano, TX), CAB-O-SIL (a pyrogenic silicon dioxide product sold by Cabot Co. of Boston, MA), and mixtures thereof. If used at all, lubricants are typically used in an amount of less than about 1 weight percent of the pharmaceutical compositions or dosage forms into which they are incorporated.

Active ingredients of the invention can be administered by controlled release means or by delivery devices that are well known to those of ordinary skill in the art. Examples include, but are not limited to, those described in U.S. Patent Nos.: 3,845,770; 3,916,899; 3,536,809; 3,598,123; and 4,008,719, 5,674,533, 5,059,595, 5,591,767, 5,120,548, 5,073,543, 5,639,476, 5,354,556, and 5,733,566. Such dosage forms can be used to provide slow or controlled-release of one or more active ingredients using, for example, hydropropylmethyl cellulose, other polymer matrices, gels, permeable membranes, osmotic systems, multilayer coatings, microparticles, liposomes, microspheres, or a combination thereof to provide the desired release profile in varying proportions. Suitable controlled-release formulations known to those of ordinary skill in the art, including those described herein, can be readily selected for use with the active ingredients of the invention. The invention thus encompasses single unit dosage forms suitable for oral administration such as, but not limited to, tablets, capsules, gelcaps, and caplets that are adapted for controlled-release.

All controlled-release pharmaceutical products have a common goal of improving drug therapy over that achieved by their non-controlled counterparts. Ideally, the use of an optimally designed controlled-release preparation in medical treatment is characterized by a minimum of drug substance being employed to cure or control the condition in a minimum amount of time. Advantages of controlled-release formulations include extended activity of the drug, reduced dosage frequency, and increased patient compliance.

Most controlled-release formulations are designed to initially release an amount of drug (active ingredient) that promptly produces the desired therapeutic effect, and gradually and continually release of other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled-release of an active ingredient can be stimulated by various conditions including, but not limited to, pH, temperature, enzymes, water, or other physiological conditions or compounds.

A particular extended release formulation of this invention comprises a therapeutically or prophylactically effective amount of a compound in Table 1A or 2A, or a pharmaceutically acceptable salt, in spheroids which further comprise microcrystalline cellulose and, optionally, hydroxypropylmethyl-cellulose coated with a mixture of ethyl cellulose and hydroxypropylmethylcellulose. Such extended release formulations can be prepared according to U.S. Patent No. 6,274,171.

A specific controlled-release formulation of this invention comprises from about 6% to about 40% a compound in Table 1A or 2A, or a pharmaceutically acceptable salt, solvate, hydrate, clathrate, by weight, about 50% to about 94% microcrystalline cellulose, NF, by weight, and optionally from about 0.25% to about 1% by weight of hydroxypropyl-methylcellulose, USP, wherein the spheroids are coated with a film coating composition comprised of ethyl cellulose and hydroxypropylmethylcellulose.

Parenteral dosage forms can be administered to patients by various routes including, but not limited to, subcutaneous, intravenous (including bolus injection), intramuscular, and intraarterial. Because their administration typically bypasses patients' natural defenses against contaminants, parenteral dosage forms are preferably sterile or capable of being sterilized prior to administration to a patient Examples of parenteral dosage forms include, but are not limited to, solutions ready for injection, dry products ready to be dissolved or suspended in a pharmaceutically acceptable vehicle for injection, suspensions ready for injection, and emulsions.

Suitable vehicles that can be used to provide parenteral dosage forms of the invention are well known to those skilled in the art. Examples include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

Compounds that increase the solubility of one or more of the active ingredients disclosed herein can also be incorporated into the parenteral dosage forms of the invention.

Transdermal, topical, and mucosal dosage forms of the invention include, but are not limited to, ophthalmic solutions, sprays, aerosols, creams, lotions, ointments, gels, solutions, emulsions, suspensions, or other forms known to one of skill in the art. *See, e.g.,* Remington's Pharmaceutical Sciences (1980 & 1990) 16th and 18th eds., Mack Publishing, Easton PA and Introduction to Pharmaceutical Dosage Forms (1985) 4th ed., Lea & Febiger, Philadelphia. Dosage forms suitable for treating mucosal tissues within the oral cavity can be formulated as mouthwashes or as oral gels. Further, transdermal dosage forms include "reservoir type" or "matrix type" patches, which can be applied to the skin and worn for a specific period of time to permit the penetration of a desired amount of active ingredients.

Suitable excipients (*e.g.,* carriers and diluents) and other materials that can be used to provide transdermal, topical, and mucosal dosage forms encompassed by this invention are well known to those skilled in the pharmaceutical arts, and depend on the particular tissue to which a given pharmaceutical composition or dosage form will be applied. With that fact in mind, typical excipients include, but are not limited to, water, acetone, ethanol, ethylene glycol, propylene glycol, butane-1,3-diol, isopropyl myristate, isopropyl palmitate, mineral oil, and mixtures thereof to form lotions, tinctures, creams, emulsions, gels or ointments, which are non-toxic and pharmaceutically acceptable. Moisturizers or humectants can also be added to pharmaceutical compositions and dosage forms if desired. Examples of such additional ingredients are well known in the art. *See, e.g.,* Remington's Pharmaceutical Sciences (1980 & 1990) 16th and 18th eds., Mack Publishing, Easton PA.

Depending on the specific tissue to be treated, additional components may be used prior to, in conjunction with, or subsequent to treatment with active ingredients of the invention. For example, penetration enhancers can be used to assist in delivering the active ingredients to the tissue. Suitable penetration enhancers include, but are not limited to: acetone; various alcohols such as ethanol, oleyl, and tetrahydrofuryl; alkyl sulfoxides such as dimethyl sulfoxide; dimethyl acetamide; dimethyl formamide; polyethylene glycol; pyrrolidones such as polyvinylpyrrolidone; Kollidon grades (Povidone, Polyvidone); urea; and various water-soluble or insoluble sugar esters such as Tween 80 (polysorbate 80) and Span 60 (sorbitan monostearate).

The pH of a pharmaceutical composition or dosage form, or of the tissue to which the pharmaceutical composition or dosage form is applied, may also be adjusted to improve delivery of one or more active ingredients. Similarly, the polarity of a solvent carrier, its ionic strength, or tonicity can be adjusted to improve delivery. Compounds such as stearates can also be added to pharmaceutical compositions or dosage forms to advantageously alter the hydrophilicity or lipophilicity of one or more active ingredients so as to improve delivery. In this regard, stearates can serve as a lipid vehicle for the formulation, as an emulsifying agent or surfactant, and as a delivery-enhancing or penetration-enhancing agent. Different salts, hydrates or solvates of the active ingredients can be used to further adjust the properties of the resulting composition.

The amount of the compound or composition of the invention which will be effective in the prevention, treatment, management, or amelioration of a proliferative disorders, such as cancer, or one or more symptoms thereof, will vary with the nature and severity of the disease or condition, and the route by which the active ingredient is administered. The frequency and dosage will also vary according to factors specific for each patient depending on the specific therapy (*e.g.,* therapeutic or prophylactic agents) administered, the severity of the disorder, disease, or condition, the route of administration, as well as age, body, weight, response, and the past medical history of the patient. Effective doses may be extrapolated from dose-response curves derived from *in vitro* or animal model test systems. Suitable regiments can be selected by one skilled in the art by considering such factors and by following, for example, dosages reported in the literature and recommended in the Physician's Desk Reference (57th ed., 2003).

Exemplary doses of a small molecule include milligram or microgram amounts of the small molecule per kilogram of subject or sample weight (*e.g.,* about 1 microgram per kilogram to about 500 milligrams per kilogram, about 100 micrograms per kilogram to about 5 milligrams per kilogram, or about 1 microgram per kilogram to about 50 micrograms per kilogram).

In general, the recommended daily dose range of a compound of the invention for the conditions described herein lie within the range of from about 0.01 mg to about 1000 mg per day, given as a single once-a-day dose preferably as divided doses throughout a day. In one embodiment, the daily dose is administered twice daily in equally divided doses. Specifically, a daily dose range should be from about 5 mg to about 500 mg per day, more specifically, between about 10 mg and about 200 mg per day. In managing the patient, the therapy should be initiated at a lower dose, perhaps about 1 mg to about 25 mg, and increased if necessary up to about 200 mg to about 1000 mg per day as either a single dose or divided doses, depending on the patient's global response. It may be necessary to use dosages of the active ingredient outside the ranges disclosed herein in some cases, as will be apparent to those of ordinary skill in the art. Furthermore, it is noted that the clinician or treating physician will know how and when to interrupt, adjust, or terminate therapy in conjunction with individual patient response.

Different therapeutically effective amounts may be applicable for different proliferative disorders, as will be readily known by those of ordinary skill in the art. Similarly, amounts sufficient to prevent, manage, treat or ameliorate such proliferative disorders, but insufficient to cause, or sufficient to reduce, adverse effects associated with the compounds of the invention are also encompassed by the above described dosage amounts and dose frequency schedules. Further, when a patient is administered multiple dosages of a compound of the invention, not all of the dosages need be the same. For example, the dosage administered to the patient may be increased to improve the prophylactic or therapeutic effect of the compound or it may be decreased to reduce one or more side effects that a particular patient is experiencing.

In a specific embodiment, the dosage of the composition of the invention or a compound of the invention administered to prevent, treat, manage, or ameliorate a proliferative disorders, such as cancer, or one or more symptoms thereof in a patient is 150 µg/kg, preferably 250 µgkg, 500 µg/kg, 1 mg/kg, 5 mg/kg, 10 mg/kg, 25 mg/kg, 50 mg/kg, 75 mg/kg, 100 mg/kg, 125 mg/kg, 150 mg/kg, or 200 mg/kg or more of a patient's body weight. In another embodiment, the dosage of the composition of the invention or a compound of the invention administered to prevent, treat, manage, or ameliorate a proliferative disorders, such as cancer, or one or more symptoms thereof in a patient is a unit dose of 0.1 mg to 20 mg, 0.1 mg to 15 mg, 0.1 mg to 12 mg, 0.1 mg to 10 mg, 0.1 mg to 8 mg, 0.1 mg to 7 mg, 0.1 mg to 5 mg, 0.1 to 2.5 mg, 0.25 mg to 20 mg, 0.25 to 15 mg, 0.25 to 12 mg, 0.25 to 10 mg, 0.25 to 8 mg, 0.25 mg to 7m g, 0.25 mg to 5 mg, 0.5 mg to 2.5 mg, 1 mg to 20 mg, 1 mg to 15 mg, 1 mg to 12 Mg, 1 mg to 10 mg, 1 mg to 8 mg, 1 mg to 7 mg, 1 mg to 5 mg, or 1 mg to 2.5 mg. The unit dose can be administered 1, 2, 3, 4 or more times daily, or once every 2, 3, 4, 5, 6 ot 7 days, or once weekly, once every two weeks, once every three weeks or once monthly.

In one embodiment, the taxane (*e.g.,* paclitaxel or docetaxel) is administered once every 3 weeks schedule and the HSP inhibitor (*e.g.,* Compound 1) is administered on week 1 and 2 (with a rest week after that) before beginning again. Alternatively, a once every three week regimen at a starting dose of 60 mg/m2 escalating to 75mg/m2 for paclitaxel or docetaxel is used. In another alternative, a 3 week on/1 week off regimen starting at 30 mg/m2 escalating to 35 mg/m2 with paclitaxel or docetaxel is used. The amount of the HSP 90 inhibitor is adjusted according to tolerability and efficacy, as described above.

In another altnerative, Paclitaxel is given either once weekly (typical dose 90 mg/m2, range 70-100). Alternatively it is given once every three weeks. Doses range from 175 to 225 mg/m2 when given once every three weeks. The dose of the HSP 90 inhibitor is commonly a full single agent dose (*e.g.,* 200 mg/m2, or less, depending on tolerability, as described above.

In another altnerative, docetaxel is given once very three weeks (dose level 75 mg/m2, range 60-100 mg/m2). It can be also given weekly, range 30-40 mg/m2. The dose of the HSP 90 inhibitor is commonly a full single agent dose *(e.g.,* 200 mg/m2, or less, depending on tolerability, as described above.

Alternatively, the treatment cycle comprises weekly treatments for 2 weeks followed by a 1-week rest period. Treatment cycles will be repeated every 3 weeks. The HSP90 inhibitor is administered (150 mg/m² or 200 mg/m²) on Days 1 and 8 of each cycle and docetaxel (60 mg/m² or 75) mg/m² is administered on Day 1 of each cycle. The treatment is repeated every three weeks.

In another alternative, subjects are administered 200 mg/m² of the HSP90 inhibitor followed by docetaxel 25 mg/m², 30 mg/m or 35 mg/m² for three consecutive weeks followed by a 1-week dose-free interval. Treatment is then repeated.

The dosages of prophylactic or therapeutic agents other than compounds of the invention, which have been or are currently being used to prevent, treat, manage, or proliferative disorders, such as cancer, or one or more symptoms thereof can be used in the combination therapies of the invention. Preferably, dosages lower than those which have been or are currently being used to prevent, treat, manage, or ameliorate a proliferative disorders, or one or more symptoms thereof, are used in the combination therapies of the invention. The recommended dosages of agents currently used for the prevention, treatment, management, or amelioration of a proliferative disorders, such as cancer, or one or more symptoms thereof, can obtained from any reference in the art including, but not limited to, Hardman et al., eds., 1996, Goodman & Gilman's The Pharmacological Basis Of Basis Of Therapeutics 9th Ed, Mc-Graw-Hill, New York; Physician's Desk Reference (PDR) 57th Ed., 2003, Medical Economics Co., Inc., Montvale, NJ.

In certain embodiments, when the compounds of the invention are administered in combination with another therapy, the therapies (*e.g.,* prophylactic or therapeutic agents) are administered less than 5 minutes apart, less than 30 minutes apart, 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours part. In one embodiment, two or more therapies (e.g., prophylactic or therapeutic agents) are administered within the same patent visit.

In certain embodiments, one or more compounds of the invention and one or more other the therapies (*e.g.,* therapeutic agents) are cyclically administered. Cycling therapy involves the administration of a first therapy (*e.g.,* a first prophylactic or therapeutic agents) for a period of time, followed by the administration of a second therapy (*e.g.,* a second prophylactic or therapeutic agents) for a period of time, followed by the administration of a third therapy (*e.g.,* a third prophylactic or therapeutic agents) for a period of time and so forth, and repeating this sequential administration, *i.e.,* the cycle in order to reduce the development of resistance to one of the agents, to avoid or reduce the side effects of one of the agents, and/or to improve the efficacy of the treatment.

In certain embodiments, administration of the same compound of the invention may be repeated and the administrations may be separated by at least 1 day, 2 days, 3 days, 5 days, 10 days, 15 days, 30 days, 45 days, 2 months, 75 days, 3 months, or 6 months. In other embodiments, administration of the same prophylactic or therapeutic agent may be repeated and the administration may be separated by at least at least 1 day, 2 days, 3 days, 5 days, 10 days, 15 days, 30 days, 45 days, 2 months, 75 days, 3 months, or 6 months.

In a specific embodiment, the invention provides compounds for use in a method of preventing, treating, managing, or ameliorating a proliferative disorders, such as cancer, or one or more symptoms thereof, said methods comprising administering to a subject in need thereof a dose of at least 150 µg/kg, preferably at least 250 µg/kg, at least 500 µg/kg, at least 1 mg/kg, at least 5 mg/kg, at least 10 mg/kg, at least 25 mg/kg, at least 50 mg/kg, at least 75 mg/kg, at least 100 mg/kg, at least 125 mg/kg, at least 150 mg/kg, or at least 200 mg/kg or more of one or more compounds of the invention once every day, preferably, once every 2 days, once every 3 days, once every 4 days, once every 5 days, once every 6 days, once every 7 days, once every 8 days, once every 10 days, once every two weeks, once every three weeks, or once a month. Alternatively, the dose can be divided into portions (typically equal portions) administered two, three, four or more times a day.

The invention is illustrated by the following examples which are not intended to be limiting in any way.

### Example 1: The Combination of Compound 1 and Paclitaxel Displays Enhanced Anti-tumor Activity against Human Tumor Cells in a SCID Mouse Xenograft Model With NCI-H1975 NSCLC Cells

The human non-small cell lung cancer (NSCLC) cell line, NCI-H1975 (ATCC #CRL-5908) was obtained from the American Type Culture Collection (ATCC; Manassas, Virginia, USA). The cell line was cultured in growth media prepared from 50% Dulbecco's Modified Eagle Medium (high glucose), 50% RPMI Media 1640 (4.5 g/L glucose), 10% fetal bovine serum (FBS), 10 mM HEPES, 1% 100X Penicillin-Streptomycin, 1% 100X sodium pyruvate and 1% 100X MEM non-essential amino acids. FBS was obtained from ATCC and all other reagents were obtained from Invitrogen Corp. (Carlsbad, California, USA). Cells that had been cryopreserved in liquid nitrogen were rapidly thawed at 37°C and transferred to a tissue culture flask containing growth media and then incubated at 37°C in a 5% CO₂ incubator. To expand the NCI-H1975 cell line, cultures were split 1:5 every 3 days when 175 cm² flasks became 85% confluent. Cultures were passaged by washing with 10 mL of room temperature phosphate buffered saline (PBS) and then disassociating cells by adding 5 mL 1X trypsin-EDTA and incubating at 37°C until the cells detached from the surface of the flask. To inactivate the trypsin, 5 mL of growth media was added and then the contents of the flask were centrifuged to pellet the cells. The supernatant was aspirated and the cell pellet was resuspended in 10 mL of growth media and the cell number determined using a hemocytometer. Cells were seeded into 175 cm² flasks containing 50 mL of growth media and incubated at 37°C in a 5% CO₂ incubator. When the flasks reached 85% confluence, the above passaging process was repeated until sufficient cells had been obtained for implantation into mice.

Six to seven week old, female CB17/Icr-*Prkdc^{scid}*/Crl (SCID) mice were obtained from Charles River Laboratories (Wilmington, Massachusetts, USA). Animals were housed 4-5/cage in micro-isolators, with a 12hr/12hr light/dark cycle, acclimated for at least 1 week prior to use and fed normal laboratory chow *ad libitum.* Animals were between seven to eight weeks of age at implantation. To implant NCI-H1975 tumor cells into SCID mice, cells were collected as described above, washed in PBS and resusupended at a concentration of 5 x 10(7) cells/mL in 50% non-supplemented medium and 50% Matrigel Basement Membrane Matrix (#354234; BD Biosciences; Bedford, Massachusetts, USA). Using a 27 gauge needle and 1 cc syringe, 5 x 10(6) NCI-H1975 cells in 0.1 mL of a cell suspension were injected subcutaneously into the flanks of SCID mice.

Tumors were then permitted to develop *in vivo* until the majority reached 95-195 mm³ in tumor volume, which required ~ 1 1/2 weeks following implantation for the NCI-H1975 model. Animals with oblong, very small or large tumors were discarded and only animals carrying tumors that displayed consistent growth rates were selected for studies. Tumor volumes (V) were calculated by caliper measurement of the width (W), length (L) and thickness (T) of tumors using the following formula: V = 0.5236 x (L x W x T). Animals were randomized into treatment groups so that the average tumor volumes of each group were similar at the start of dosing. %T/C values, as a measure of efficacy, were determined as follows:
(i) If ΔT > 0: %T/C = (ΔT/ΔC) x 100
(ii) If ΔT < 0: %T/C = (ΔT/T₀) x 100
(iii) ΔT = Change in average tumor volume between start of dosing and the end of study.
(iv) ΔC = Change in average tumor volume between start of dosing and the end of study.
(v) To = Average tumor volume at start of dosing.

To formulate Compound 1, paclitaxel or a combination of both in DRD, stock solutions of the test article were prepared by dissolving the appropriate amounts of the compound in dimethyl sulfoxide (DMSO) by sonication in an ultrasonic water bath. Stock solutions were prepared weekly, stored at -20°C and diluted fresh each day for dosing. A solution of 20% Cremophore RH40 (polyoxyl 40 hydrogenated castor oil; BASF Corp., Aktiengesellschaft, Ludwigshafen, Germany) in 5% dextrose in water (Abbott Laboratories, North Chicago, Illinois, USA) was also prepared by first heating 100% Cremophore RH40 at 50-60°C until liquefied and clear, diluting 1:5 with 100% D5W, reheating again until clear and then mixing well. This solution can be stored at room temperature for up to 3 months prior to use. To prepare DRD formulations for daily dosing, DMSO stock solutions were diluted 1:10 with 20% Cremophore RH40. The final DRD formulation for dosing contained 10% DMSO, 18% Cremophore RH40, 3.6% dextrose, 68.4% water and the appropriate amount of test article. Animals were intravenously (i.v.) injected with this formulation at 10 mL per kg body weight 1 day each week

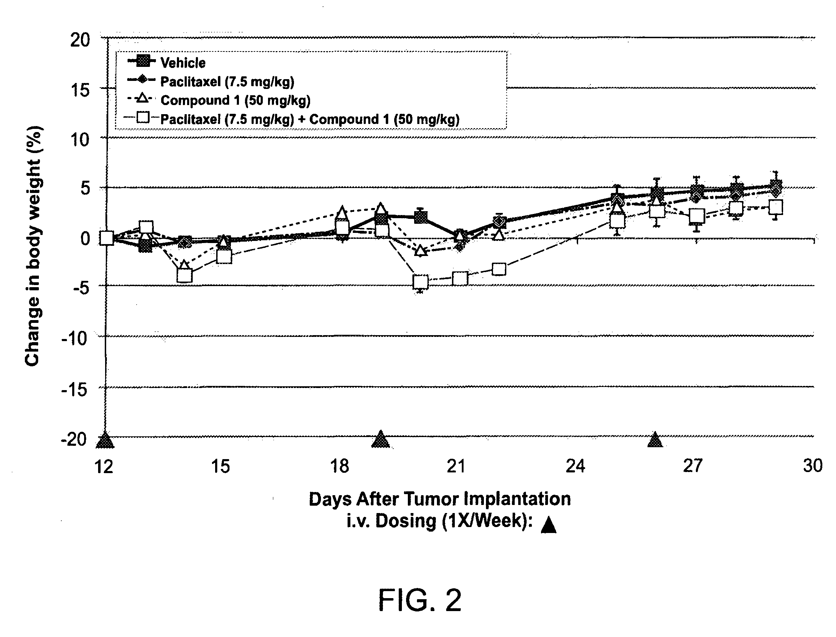

Treatment with a dose of 50 mg/kg body weight of Compound 1 moderately inhibited NCI-H1975 tumor growth in SCID mice, with a %T/C value of 55. Similarly, treatment with a dose of 7.5 mg/kg body weight of paclitaxel moderately inhibited NCI-H1975 tumor growth in SCID mice, with a %T/C value of 38. In contrast, concurrent treatment with a combination of 50 mg/kg body weight of Compound 1 plus 7.5 mg/kg body weight paclitaxel dramatically inhibited NCI-H1975 tumor growth in SCID mice, with a %T/C value of 7. The efficacy observed for the combination treatment group was significantly greater than that observed for either single-agent group alone (P < 0.05; one-way ANOVA). The results are shown in Figure 1. This effect was not associated with excessive toxicity, as the Compound 1 plus paclitaxel combination treatment group had an average bodyweight change on day 29 (last day measured) relative to the start of the study of +3.1% (+/- 1.2 SEM), as compared to +5.1% (+/-1.4 SEM) for the vehicle-treated group. The results are shown in Figure 2.

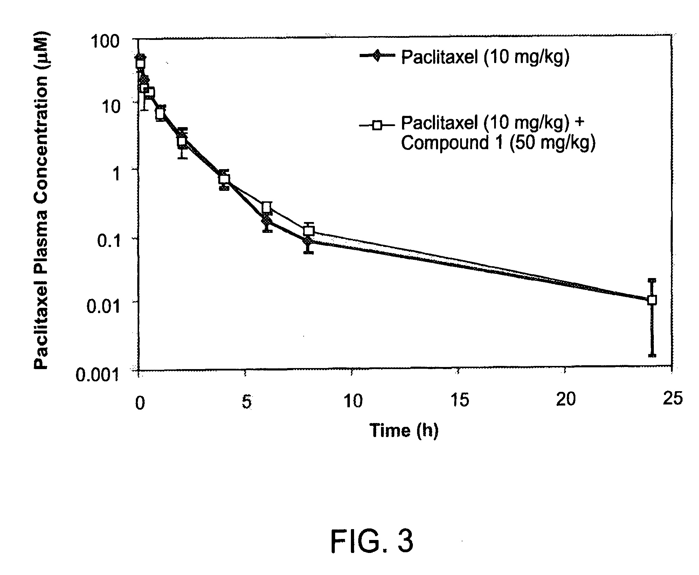

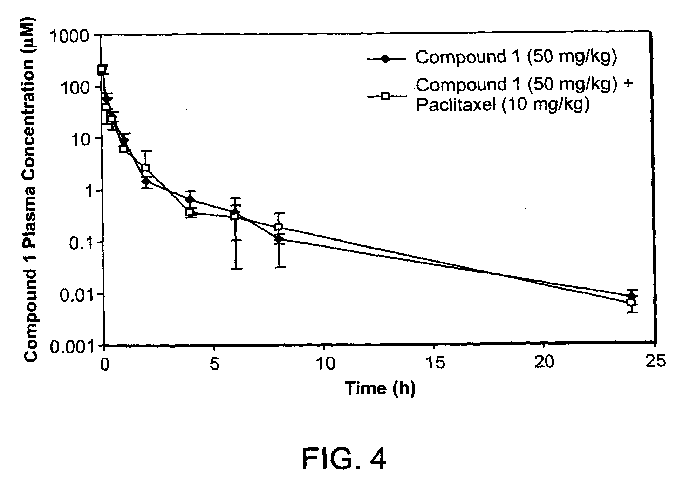

To examine the potential for drug-drug interactions between Compound 1 and paclitaxel, an *in vivo* pharmacokinetic study was conduced. Seven to eight week old, female Crl:CD-1-*nu*BR (*nude*) mice were obtained from Charles River Laboratories (Wilmington, Massachusetts, USA). Animals were housed 4-5/cage in micro-isolators, with a 12hr/12hr light/dark cycle, acclimated for at least 1 week prior to use and fed normal laboratory chow *ad libitum.* Animals (3/time point) were i.v. dosed a single time with 50 mg/kg body weight Compound 1 alone, 10 mg/kg body weight paclitaxel alone, or a combination of 50 mg/kg body weight Compound 1 plus 10 mg/kg body weight paclitaxel. Blood samples were withdrawn at multiple time points (0.083, 0.25, 0.5, 1, 2, 4, 6, 8, 24 hr), plasma prepared, and the concentrations of Compound 1 and paclitaxel were determined by HPLC. As shown in Table 3, Compound 1 had no significant effect on the plasma half-life (t½), peak plasma concentration (Cmax) or total plasma exposure (AUCinf) of paclitaxel. The results are shown in Figure 3. Similarly, paclitaxel had no significant affect on the plasma half-life (t½), peak plasma concentration (Cmax) or total plasma exposure (AUCinf) of Compound 1. The results are shown in Figure 4.

**Table 3**

| Treatment | HPLC analyte | Dose (mg/kg) | t½ (hr) | Cmax (µM) | AUCinf (µMxhr) |
|---|---|---|---|---|---|
| Compound 1 | Compound 1 | 50 | 3.5 | 216 | 78.0 |
| Compound 1 + Paclitaxel | Compound 1 | 50 | 3.2 | 215 | 75.8 |
| Paclitaxel | Paclitaxel | 10 | 4.6 | 48.0 | 32.2 |
| Paclitaxel + Compound 1 | Paclitaxel | 10 | 3.9 | 41.1 | 29.5 |

### Example 2: In Vitro Combination Analysis of Compound 1 and Paclitaxel

### A. Cells and Cell Culture

Human non-small cell lung carcinoma cells (H1975) from American Type Culture Collection were grown in Dulbecco's modified Eagle's medium with 4 mM L-glutamine, antibiotics (100 IU/ml penicillin and 100 µg/ml streptomycin) and 10% fetal bovine serum from Sigma Aldrich. Cells were subcultivated at a 1:3 to 1:6 ratio two to three times per week. Growth curves were performed on the cells in black wall, clear bottom 96 well plates to ensure logarithmic growth throughout the four day assays described below. To do so, cells were seeded at several different densities on day zero, and total net growth was calculated by comparing the total growth at day four versus day zero as determined by alamarBlue. From the results, 2000 cells/well were determined to be optimal for a four day study.

### B. Combination Studies with Paclitaxel and a Compound 1

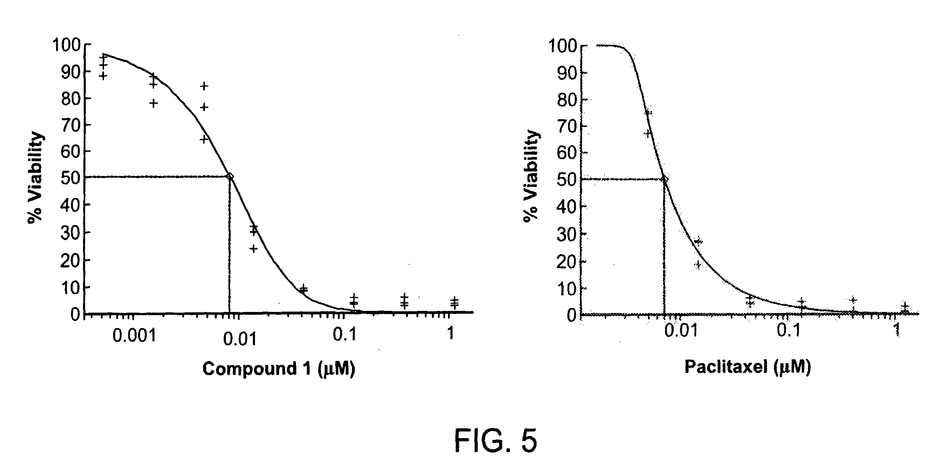

The half maximal inhibitory concentration (IC₅₀) for paclitaxel and Compound 1 was determined using three-fold serial dilutions of compound starting with a top concentration of 1 µM. After 72 h exposure to either drug, viability was determined by alamarBlue, data from which was used to calculate the ICso values using XLFit software (ID Business Solutions). The single agent IC₅₀ value for Compound 1 in H1975 was calculated at 15 nM; for paclitaxel the IC₅₀ was 7 nM (Figure 5).

Combinations between paclitaxel and Compound 1 were then performed concurrently and analyzed by median effect analysis. Drugs were either combined at their equipotent ratio, based on the IC₅₀ molar concentrations for each agent, or in non-equal ratios. Cells were incubated for 3 days with the drug combinations. The surviving fraction of cells relative to control was determined using the alamarBlue cell viability assay. Combination Index (CI) was determined using the median effect analysis software CalcuSyn 2.0 (CalcuSyn, Inc.) for combination concentrations where 20-70% of the cells were affected (*i.e.,* killed). A Combination Index greater than 1, equal to 1 or less than 1 indicates antagonism, additivity and synergism, respectively.

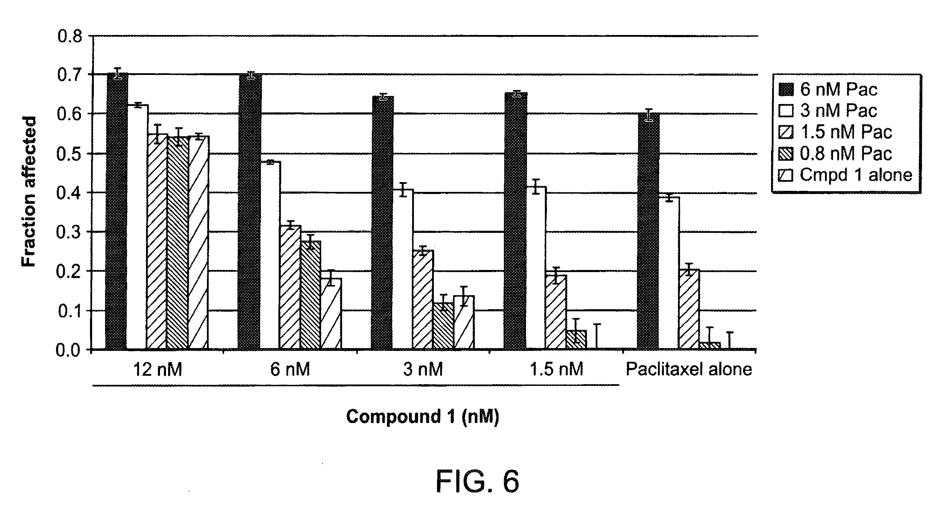

Shown in Figure 6 and Table 4, the combination of Compound 1 and paclitaxel was found to be synergistic when the concentrations of both compounds were less than the IC₅₀ but greater than the IC₂₀.

**Table 4. Combination Index values for the concurrent treatment of paclitaxel and Compound 1**

| **Compound 1 (nM)** | **Paclitaxel (nM)** | **Cl** | |
|---|---|---|---|
| 3 | 0.8 | 1.085 | Slight antagonism |
| 3 | 1.5 | 0.772 | Moderate synergism |
| 3 | 3 | 0.713 | Moderate synergism |
| 3 | 6 | 0.576 | Synergism |
| 6 | 0.8 | 0.745 | Moderate synergism |
| 6 | 1.5 | 0.819 | Moderate synergism |
| 6 | 3 | 0.701 | Moderate synergism |
| 6 | 6 | 0.541 | Synergism |

Chemotherapeutic agents such as paclitaxel and other taxanes have their cytotoxic effect during mitosis and require the cell to progress through this portion of the cell cycle for their effect. From unpublished work with Compound 1, as well as previously published studies on other Hsp90 inhibitors (Hexner, E. 0. et al., Blood 111 (12), 5663 (2008)), these data suggest that inhibition of Hsp90 function leads to cell cycle arrest. Accordingly, testing was done to see whether treating cells sequentially (paclitaxel first, followed by Compound 1 subsequently) would influence the efficacy of the combination when compared to concurrent administration.

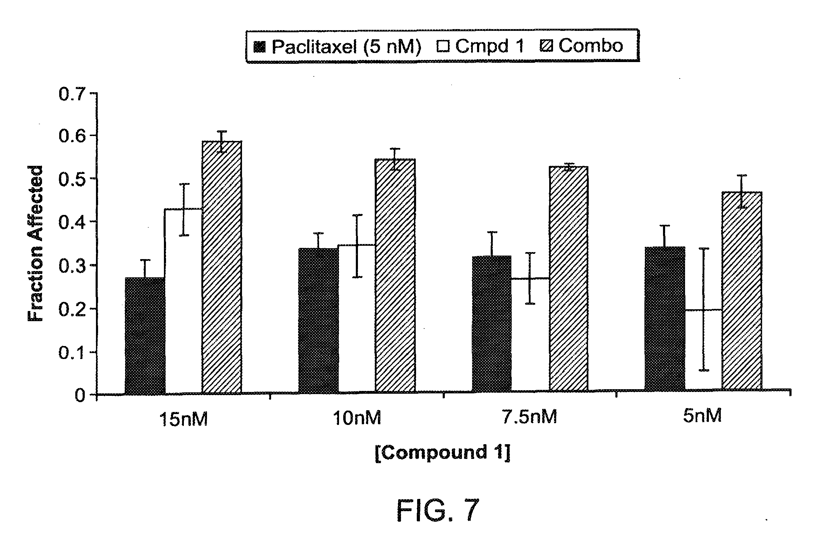

H1975 cells were treated with the 5 nM paclitaxel for 24 h at 37°C, washed the cells to remove the drug, and treated with varying amounts of Compound 1 for 24 h. Cells were washed again with media, incubated an additional 24 h and then subjected to viability analysis by alamarBlue. Shown in Figure 7 and Table 5, treatment with paclitaxel prior to Compound 1 again led to synergistic benefit.

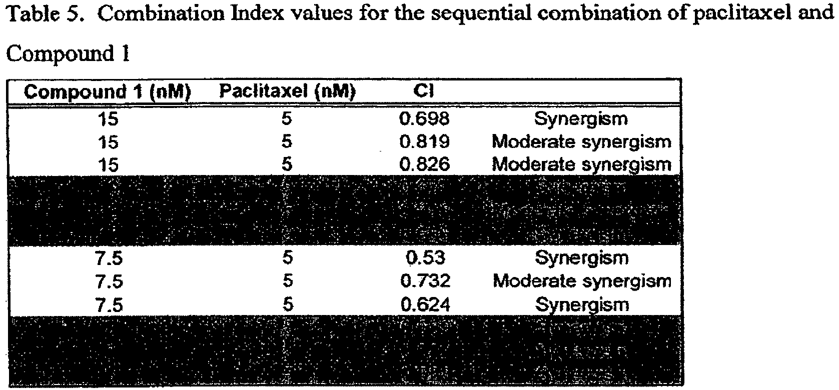

### Example 3 In Vitro Combination Analysis of Compound 1 and Taxanes

### A. Materials and Methods

### Cell Lines

Human NCI-H1975 non-small cell lung carcinoma cells (American Type Culture Collection) were grown in Dulbecco's modified Eagle's medium with 4 mM L-glutamine, antibiotics (100 IU/ml penicillin and 100 µg/ml streptomycin) and 10% fetal bovine serum (Sigma Aldrich). Human HEL92.1.7 erythroleukemia cells (ATCC) were grown in RPMI with 2 mM L-glutamine, antibiotics (100 IU/ml penicillin and 100 µg/ml streptomycin) and 10% fetal bovine serum. Human HT29 colon cancer cells (ATCC) were grown in McCoy's 5a modified medium with 10% fetal bovine serum and antibiotics (100 IU/ml penicillin and 100 µg/ml streptomycin). All cells were maintained at 37°C, 5% CO₂ atmosphere and passaged at a 1:3 to 1:6 ratio two to three times per week.

### Cell Viability Assays

Cell viability was measured using the alamarBlue assay. In brief, cells were plated in 96-well plates in triplicate at 2000 cells per well (H1975) or 5000 cells per well (HEL92.1.7) and incubated at 37°C, 5% CO₂ atmosphere for 24 hr prior to the addition of drug or vehicle (0.3% DMSO) to the culture medium. After 72 hr, 10 µl/well alamarBlue was added to the wells and incubated for an additional 3 hr at 37°C, 5% CO₂ atmosphere. Fluorescence (560_{EX}/590_{EM} nM) was measured with a SpectraMax microplate reader (Molecular Devices) and the resulting data were used to calculate cell viability, normalized to vehicle control.

### B. Combination Studies with Paclitaxel and a Compound 1

The half maximal inhibitory concentration (ICso) for the taxanes (docetaxel or paclitaxel) or Compound 1 was determined using a three-fold serial dilution series of compound starting with a top concentration of 1 µM. After 72 hr exposure to drug, cell viability was measured. Data were used to calculate the IC₅₀ values using XLFit software (ID Business Solutions) and are reported in Table 6.

**Table 6. Single agent ICso values (nM) for Compound 1 and taxanes (N/D = not determined).**

| | **NCI-H1975** | **HEL-92.1.7** | **HT29** |
|---|---|---|---|
| **Compound 1** | 13 | 32 | 36 |
| **Docetaxel** | 1 | 14 | 0.6 |
| **Paclitaxel** | 4 | 25 | N/D |

Combinations between the taxanes and Compound 1 were then performed concurrently based on the IC₅₀ for each agent. Specifically, serial 1.2 fold dilutions, starting from the IC₅₀ of paclitaxel or docetaxel, were mixed with similar fold dilutions from the IC₅₀ of Compound 1. The combined drugs, as well as each drug alone, were incubated with the cells for 3 days and the surviving fraction of cells relative to control was determined using the alamarBlue assay. Combination Index (CI) was determined using the median effect analysis software CalcuSyn 2.0 (CalcuSyn, Inc.). A Combination Index greater than 1, equal to 1 or less than 1 indicates antagonism, additivity and synergism, respectively (Table 7).

**Table 7. Description of Combination Index values.**

| Range of CI | Description |
|---|---|
| <0.1 | Very strong synergism |
| 0.1-0.3 | Strong synergism |
| 0.3-0,7 | Synergism |
| 0.7-0.85 | Moderate synergism |
| 0.85-0.9 | Slight synergism |
| 0.9-1.0 | Nearly additive |
| 1.1-12 | Slight antagonism |
| 1.2-1.45 | Moderate antagonism |
| 1.45-3.3 | Antagonsim |
| 3.3-10 | Strong antagonism, |
| >10 | Very strong antagonism |

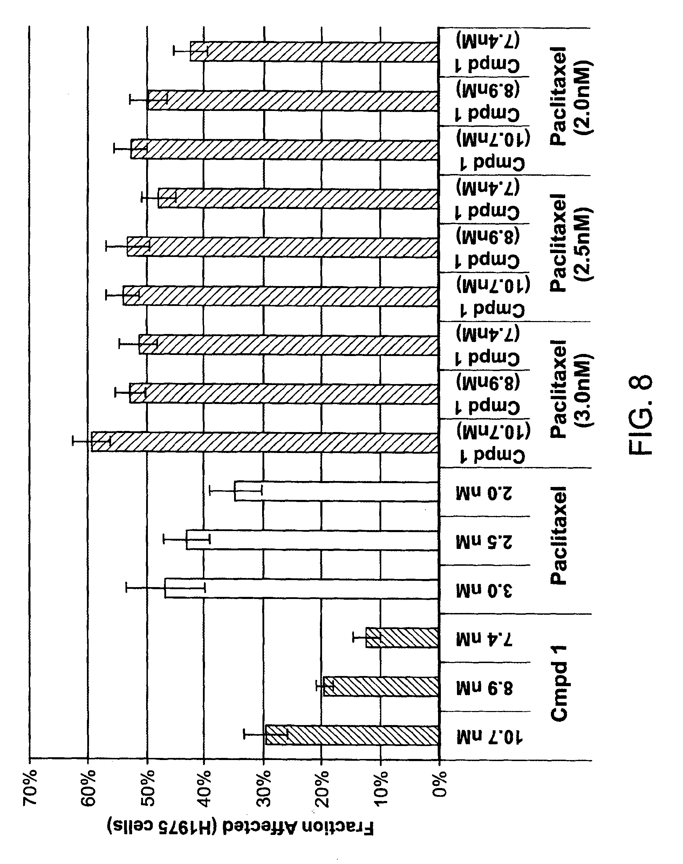

Figure 8 presents a typical dataset from a combination experiment. In this case, the results show the percent of cells affected by Compound 1, paclitaxel or the combination of the two in H1975 cells. The data is then subject to linear curve fitting by CalcuSyn to generate the combination index.

Shown in Figure 9a and 9b, the combination of Compound 1 with either docetaxel or paclitaxel in H1975 NSCLC cells produced more cell death than that expected from the additive effects of their respective doses (combination index values < 1). Hence, these combinations are synergistic. Strong to very strong synergism was found when either of the taxanes was combined concurrently with Compound 1 in HEL92.1.7 erythroleukemia (Figure 10) cells. Docetaxel was also found to be synergistic when administered with Compound 1 in HT29 colon carcinoma cells (Figure 11). Taken together, the data demonstrate that Compound 1 and the taxanes function synergistically to kill cancer cells.

### Example 4 The Combination of Compound 1 and Docetaxel Displays Enhanced Anti-tumor Activity against Human Tumor Cells in a SCID Mouse Xenograft Model With HCC827 NSCLC Cells

SCID mice were implanted with HCC827 (EGFR^{DelE726-A750}) human non-small cell lung cancer (NSCLC) cells exactly as described in Example 1. Tumors were permitted to develop in vivo until the majority reached 95-195 mm³ and then treated one time per week with vehicle alone, 75mg/kg at 10 ml per kg body weight of Compound 1, 4 mg/kg docetaxel (formulated similar to paclitaxel), or the combination of the two concurrently. As shown in Figure 12, 75 mg/kg Compound 1 plus 4 mg/kg docetaxel displayed enhanced efficacy compared to either single agent alone, with %T/C values of 0 versus 46 and 26 for docetaxel and Compound 1 alone, respectively. This effect was not associated with excessive toxicity, and no drug-drug interactions were observed between Compound 1 and docetaxel in pharmacokinetic studies (data not shown).

## Claims

1. A compound selected from:
(a) 3-(2,4-dihydroxy-5-isopropyl-phenyl)-4-(1-methyl-indol-5-yl)-5-hydroxy-[1,2,4]triazole, or a tautomer, or a pharmaceutically acceptable salt thereof; or
(b) 5-hydroxy-4-(5-hydroxy-4-(1-methyl-1H-indol-5-yl)-4H-1,2,4-triazol-3-yl)-2-isopropylphenyl dihydrogen phosphate, or a tautomer, or a pharmaceutically acceptable salt thereof,
for use in the treatment of lung cancer, colon carcinoma, or erythroleukemia in a patient who is also administered paclitaxel or docetaxel.

2. Compound for use according to Claim 1, wherein the compound is 3-(2,4-dihydroxy-5-isopropyl-phenyl)-4-(1-methyl-indol-5-yl)-5-hydroxy-[1,2,4]triazole, or a tautomer, or a pharmaceutically acceptable salt thereof.

3. Compound for use according to Claim 1, wherein the compound is 5-hydroxy-4-(5-hydroxy-4-(1-methyl-1H-indol-5-yl)-4H-1,2,4-triazol-3-yl)-2-isopropylphenyl dihydrogen phosphate, or a tautomer, or a Pharmaceutical acceptable salt thereof.

4. Compound for use according to any one of Claims 1 to 3, wherein the cancer is lung cancer.

5. Compound for use according to Claim 4, wherein the lung cancer is non-small cell lung cancer.

6. Compound for use according to any one of Claims 1 to 3, wherein the cancer is colon cancer.

7. Compound for use according to any one of Claims 1 to 3, wherein the cancer is erythroleukemia.

8. Compound for use according to any one of Claims 1 to 7, wherein the patient is also administered docetaxel.

9. Compound for use according to any one of Claims 1 to 7, wherein the patient is also administered paclitaxel.

10. Compound for use according to any one of Claims 1 to 9, wherein the paclitaxel or docetaxel and the 1,2,4-triazole compound are administered sequentially.

11. Compound for use according to any one of Claims 1 to 10, wherein the 1,2,4-triazole compound is administered in a dosage of from about 0.01 mg to about 1000 mg per day.

12. Use of a compound selected from:
(a) 3-(2,4-dihydroxy-5-isopropyl-phenyl)-4-(1-methyl-indol-5-yl)-5-hydroxy-[1,2,4]triazole, or a tautomer, or a pharmaceutically acceptable salt thereof; or
(b) 5-hydroxy-4-(5-hydroxy-4-(1-methyl-1H-indol-5-yl)-4H-1,2,4-triazol-3-yl)-2-isopropylphenyl dihydrogen phosphate, or a tautomer, or a pharmaceutically acceptable salt thereof,
for the manufacture of a medicament for the treatment of lung cancer, colon carcinoma, or erythroleukemia in a patient who is also administered paclitaxel or docetaxel.

13. Use according to Claim 12, wherein the compound is 3-(2,4-dihydroxy-5-isopropylphenyl)-4-(1-methyl-indol-5-yl)-5-hydroxy-[1,2,4]triazole, or a tautomer, or a pharmaceutically acceptable salt thereof.

14. Use according to Claim 12 or Claim 13, wherein the cancer is non-small cell lung cancer.

15. Use according to any one of Claims 12 to 14, wherein the patient is also administered docetaxel.

## Patentansprüche

1. Verbindung, die ausgewählt ist aus:
(a) 3-(2,4-Dihydroxy-5-isopropyl-phenyl)-4-(1-methyl-indol-5-yl)-5-hydroxy-[1,2,4]triazol oder ein Tautomer oder ein pharmazeutisch annehmbares Salz davon; oder
(b) 5-Hydroxy-4-(5-hydroxy-4-(1-methyl-1H-indol-5-yl)-4H-1,2,4-triazol-3-yl)-2-isopropylphenyl-dihydrogen-phosphat oder ein Tautomer oder ein pharmazeutisch annehmbares Salz davon
zur Verwendung bei der Behandlung von Lungenkrebs, Darmkrebs oder Erythroleukämie in einem Patienten, dem auch Paclitaxel oder Docetaxel verabreicht wird.

2. Verbindung zur Verwendung nach Anspruch 1, wobei die Verbindung 3-(2,4-Dihydroxy-5-isopropyl-phenyl)-4-(1-methyl-indol-5-yl)-5-hydroxy-[1,2,4]triazol oder ein Tautomer oder ein pharmazeutisch annehmbares Salz davon ist.

3. Verbindung zur Verwendung nach Anspruch 1, wobei die Verbindung 5-Hydroxy-4-(5-hydroxy-4-(1-methyl-1H-indol-5-yl)-4H-1,2,4-triazol-3-yl)-2-isopropylphenyl-dihydrogen-phosphat oder ein Tautomer oder ein pharmazeutisch annehmbares Salz davon ist.

4. Verbindung zur Verwendung nach einem der Ansprüche 1 bis 3, wobei der Krebs Lungenkrebs ist.

5. Verbindung zur Verwendung nach Anspruch 4, wobei der Lungenkrebs nichtkleinzelliger Lungenkrebs ist.

6. Verbindung zur Verwendung nach einem der Ansprüche 1 bis 3, wobei der Krebs Darmkrebs ist.

7. Verbindung zur Verwendung nach einem der Ansprüche 1 bis 3, wobei der Krebs Erythroleukämie ist.

8. Verbindung zur Verwendung nach einem der Ansprüche 1 bis 7, wobei dem Patienten auch Docetaxel verabreicht wird.

9. Verbindung zur Verwendung nach einem der Ansprüche 1 bis 7, wobei dem Patienten auch Paclitaxel verabreicht wird.

10. Verbindung zur Verwendung nach einem der Ansprüche 1 bis 9, wobei das Paclitaxel oder Docetaxel und die 1,2,4-Triazol-Verbindung nacheinander verabreicht werden.

11. Verbindung zur Verwendung nach einem der Ansprüche 1 bis 10, wobei die 1,2,4-Triazol-Verbindung in einer Dosierung von etwa 0,01 mg bis etwa 1000 mg pro Tag verabreicht wird.

12. Verwendung einer Verbindung, die ausgewählt ist aus:
(a) 3-(2,4-Dihydroxy-5-isopropyl-phenyl)-4-(1-methyl-indol-5-yl)-5-hydroxy-[1,2,4]triazol oder ein Tautomer oder ein pharmazeutisch annehmbares Salz davon; oder
(b) 5-Hydroxy-4-(5-hydroxy-4-(1-methyl-1H-indol-5-yl)-4H-1,2,4-triazol-3-yl)-2-isopropylphenyl-dihydrogen-phosphat oder ein Tautomer oder ein pharmazeutisch annehmbares Salz davon
zur Herstellung eines Medikaments zur Behandlung von Lungenkrebs, Darmkrebs oder Erythroleukämie in einem Patienten, dem auch Paclitaxel oder Docetaxel verabreicht wird.

13. Verwendung nach Anspruch 12, wobei die Verbindung 3-(2,4-Dihydroxy-5-isopropyl-phenyl)-4-(1-methyl-indol-5-yl)-5-hydroxy-[1,2,4]triazol oder ein Tautomer oder ein pharmazeutisch annehmbares Salz davon ist.

14. Verwendung nach Anspruch 12 oder Anspruch 13, wobei der Krebs nichtkleinzelliger Lungenkrebs ist.

15. Verwendung nach einem der Ansprüche 12 bis 14, wobei dem Patienten auch Docetaxel verabreicht wird.

## Revendications

1. Composé choisi parmi :
(a) le 3-(2,4-dihydroxy-5-isopropyl-phényl)-4-(1-méthyl-indol-5-yl)-5-hydroxy-[1,2,4]triazole ou un tautomère, ou un sel pharmaceutiquement acceptable de ceux-ci ; ou
(b) le dihydrogénophosphate de 5-hydroxy-4-(5-hydroxy-4-(1-méthyl-1H-indol-5-yl)-4H-1,2,4-triazol-3-yl)-2-isopropylphényle ou un tautomère, ou un sel pharmaceutiquement acceptable de ceux-ci,
pour son utilisation dans le traitement du cancer du poumon, du cancer du côlon ou de la leucémie érythroïde chez un patient qui reçoit également du paclitaxel ou du docétaxel.

2. Composé pour son utilisation selon la revendication 1, dans lequel le composé est le 3-(2,4-dihydroxy-5-isopropyl-phényl)-4-(1-méthyl-indol-5-yl)-5-hydroxy-[1,2,4]triazole ou un tautomère, ou un sel pharmaceutiquement acceptable de ceux-ci.

3. Composé pour son utilisation selon la revendication 1, dans lequel le composé est le dihydrogénophosphate de 5-hydroxy-4-(5-hydroxy-4-(1-méthyl-1H-indol-5-yl)-4H-1,2,4-triazol-3-yl)-2-isopropylphényle ou un tautomère, ou un sel pharmaceutiquement acceptable de ceux-ci.

4. Composé pour son utilisation selon l'une quelconque des revendications 1 à 3, dans lequel le cancer est le cancer du poumon.

5. Composé pour son utilisation selon la revendication 4, dans lequel le cancer du poumon est le cancer du poumon non à petites cellules.

6. Composé pour son utilisation selon l'une quelconque des revendications 1 à 3, dans lequel le cancer est le cancer du côlon.

7. Composé pour son utilisation selon l'une quelconque des revendications 1 à 3, dans lequel le cancer est la leucémie érythroïde.

8. Composé pour son utilisation selon l'une quelconque des revendications 1 à 7, dans lequel le patient reçoit également du docétaxel.

9. Composé pour son utilisation selon l'une quelconque des revendications 1 à 7, dans lequel le patient reçoit également du paclitaxel.

10. Composé pour son utilisation selon l'une quelconque des revendications 1 à 9, dans lequel le paclitaxel ou le docétaxel et le composé 1,2,4-triazole sont administrés de manière séquentielle.

11. Composé pour son utilisation selon l'une quelconque des revendications 1 à 10, dans lequel le composé 1,2,4-triazole est administré dans une dose d'environ 0,01 mg à environ 1000 mg par jour.

12. Utilisation d'un composé choisi parmi :
(a) le 3-(2,4-dihydroxy-5-isopropyl-phényl)-4-(1-méthyl-indol-5-yl)-5-hydroxy-[1,2,4]triazole ou un tautomère, ou un sel pharmaceutiquement acceptable de ceux-ci ; ou
(b) le dihydrogénophosphate de 5-hydroxy-4-(5-hydroxy-4-(1-méthyl-1H-indol-5-yl)-4H-1,2,4-triazol-3-yl)-2-isopropylphényle ou un tautomère, ou un sel pharmaceutiquement acceptable de ceux-ci,
pour la fabrication d'un médicament destiné au traitement du cancer du poumon, du cancer du côlon ou de la leucémie érythroïde chez un patient qui reçoit également du paclitaxel ou du docétaxel.

13. Utilisation selon la revendication 12, dans laquelle le composé est le 3-(2,4-dihydroxy-5-isopropyl-phényl)-4-(l-méthyl-indol-5-yl)-5-hydroxy-[1,2,4]triazole ou un tautomère, ou un sel pharmaceutiquement acceptable de ceux-ci.

14. Utilisation selon la revendication 12 ou 13, dans laquelle le cancer est le cancer du poumon non à petites cellules.

15. Utilisation selon 1"une quelconque des revendications 12 à 14, dans laquelle le patient reçoit également du docétaxel.
